# EUROPEAN PATENT SPECIFICATION

(11) **EP 0 813 867 B1**
(45) Date of publication and mention of the grant of the patent: **01.06.2005**
(21) Application number: 97105350.9
(22) Date of filing: 18.11.1991
(51) Int. Cl.: A61K 31/17, A61K 31/47, A61K 31/44, A61K 31/535, A61K 31/505, A61K 31/34, A61K 31/415, A61K 31/495, A61K 31/215, A61K 31/18, A61K 31/325, A61K 31/40, A61K 31/195

(54) **Retroviral protease inhibitors**
Retrovirusprotease Inhibitoren
Inhibiteurs de protéases rétrovirales

(30) Priority: 19.11.1990 US 615210
(43) Date of publication of application: 29.12.1997
(62) Divisional of application: 92901068.4
(73) Proprietor: MONSANTO COMPANY, St. Louis, Missouri 63167 (US); G.D. Searle & Co., Skokie, Illinois 60077 (US)
(72) Inventor: Clare, Michael, Skokje, Illinois 60077 (US); Freskos, John Nicholas, Clayton, Missouri 63105 (US); Heintz, Robert Martin, Ballwin, Missouri 63021 (US); Decrescenzo, Gary Anthony, St. Peters, Missouri 63376 (US); Getman, Daniel Paul, Chesterfield, Mo. 63107 (US); Lin, Ko-Chung, St. Louis, Missouri 63146 (US); Mueller, Richard August, Glencoe, Illinois 60022 (US); Talley, John Jeffrey, Chesterfield, Missouri 63017 (US); Sun, Eric Tak On, San Diego, CA 92212 (US); Reed, Kathryn Lea, Richmond Heights, Missouri 63117 (US); Vasquez, Michael Lawrence, Gurnee, Illinois 60031 (US)
(74) Representative: Colens, Alain

(56) References cited:
- EP-A- 0 159 156
- EP-A- 0 365 992
- WO-A-89/10920
- FR-A- 2 620 451
- GB-A- 2 200 115
- EMBO J., vol. 8, no. 8, 1988, pages 2179-2188, XP002052826 SALI ET AL.: "High-resolution X-ray diffraction study of the complex between endothiapepsin and an oligopeptide inhibitor: the analysis of the inhibitor binding and description of the rigid body shift in the enzyme"
- SCIENCE, vol. 248, no. 4953, 20 April 1990, pages 358-361, XP002024830 ROBERTS N A ET AL: "RATIONAL DESIGN OF PEPTIDE-BASED HIV PROTEINASE INHIBITORS"

## Description

### BACKGROUND OF THE INVENTION

### 1. Field of the Invention

The present invention relates to retroviral protease inhibitors and, more particularly, relates to novel compounds and a composition and method for inhibiting retroviral proteases. This invention, in particular, relates to urea-containing hydroxyethylamine protease inhibitor compounds, a composition and method for inhibiting retroviral proteases such as human immunodeficiency virus (HIV) protease and for treating a retroviral infection, e.g., an HIV infection. The subject invention also relates to processes for making such compounds as well as to intermediates useful in such processes.

### 2. Related Art

During the replication cycle of retroviruses, gag and gag-pol gene products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. It has also been shown through site-directed mutagenesis of an aspartic acid residue in the HIV protease that processing of the gag precursor protein is prevented. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit replication of structural proteins and, more importantly, the retroviral protease itself. In this manner, retroviral replication proteases can be effectively inhibited.

Several classes of mimetic compounds have been proposed, particularly for inhibition of proteases, such as for inhibition of HIV protease. Such mimetics include hydroxyethylamine isosteres and reduced amide isosteres. See, for example, EP O 346 847; EP O 342,541; Roberts et al, "Rational Design of Peptide-Based Proteinase Inhibitors, "Science, 248, 358 (1990); and Erickson et al, "Design Activity, and 2.8Å Crystal Structure of a C₂ Symmetric Inhibitor Complexed to HIV-1 Protease," Science, 249, 527 (1990).

Several classes of mimetic compounds are known to be useful as inhibitors of the proteolytic enzyme renin. See, for example, U.S. No. 4,599,198; U.K. 2,184,730; G.B. 2,209,752; EP O 264 795; G.B. 2,200,115 and U.S. SIR H725. Of these, G.B. 2,200,115, GB 2,209,752, EP O 264,795, U.S. SIR H725 and U.S. 4,599,198 disclose urea-containing hydroxyethylamine renin inhibitors. However, it is known that, although renin and HIV proteases are both classified as aspartyl proteases, compounds which are effective renin inhibitors generally cannot be predicted to be effective HIV protease inhibitors.

### BRIEF DESCRIPTION OF THE INVENTION

The present invention is directed to virus inhibiting compounds and compositions. More particularly, the present invention is directed to retroviral protease inhibiting compounds and compositions, to a method of inhibiting retroviral proteases, to processes for preparing the compounds and to intermediates useful in such processes. The subject compounds are characterized as urea-containing hydroxyethylamine inhibitor compounds.

### DETAILED DESCRIPTION OF THE INVENTION

In accordance with the present invention, there is provided the use of a compound represented by the formula: 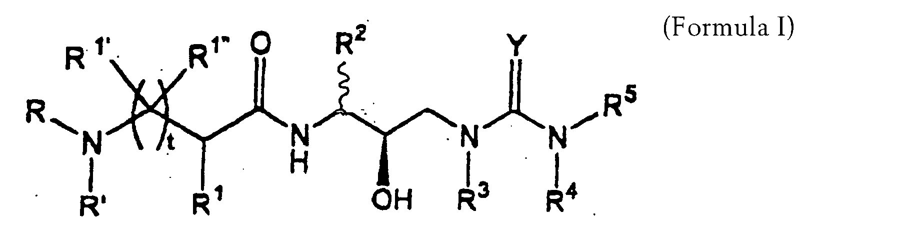 for preparing a medicament for inhibiting a retroviral protease, for treating a retroviral infection and for treating AIDS wherein:
R represents hydrogen, alkoxycarbonyl, aralkoxycarbonyl, alkylcarbonyl, cycloalkylcarbonyl, cycloalkylalkoxycarbonyl, cycloalkylalkanoyl, alkanoyl, aralkanoyl, aroyl, aryloxycarbonyl, aryloxyalkanoyl, heterocyclocarbonyl, heterocyclyloxycarbonyl, heterocyclylalkanoyl, heterocyclylalkoxycarbonyl, heteroaralkoxycarbonyl, heteroaryloxycarbonyl, heteroaroyl, alkyl,aryl, aralkyl, aryloxyalkyl, heteroaryloxyalkyl, hydroxyalkyl, alkylaminocarbonyl, arylaminocarbonyl, aralkylaminoalkylcarbonyl, and aminoalkanoyl radicals; alkylaminoalkylcarbonyl and mono- and disubstituted aminoalkanoyl radicals wherein the substituents are selected from alkyl, aryl, aralkyl, cycloalkyl, cycloalkylalkyl, heteroaryl, heteroaralkyl, heteroalkyl, heterocycloalkylalkyl radicals;
R' represents hydrogen and radicals as defined for R³, or R and R' together with the nitrogen to which they are attached form a heterocycloalkyl or heteroaryl radical;
R¹ represents -CH₂SO₂NH₂, hydrogen, -CO₂CH₃, -CONH₂, alkyl and cycloalkyl radicals, and amino acid side chains selected from the group of asparagine, S-methyl cysteine and the sulfoxide (SO) and sulfone (SO₂) derivatives thereof, histidine, norleucine, glutamine, glycine, allo-isoleucine, alanine, threonine, isoleucine, leucine, tert-leucine, phenylalanine, ornithine, allothreonine, serine, aspartic acid, beta-cyano alanine and valine side chains;
R¹' and R¹" independently represent hydrogen and radicals as defined for R¹ ;
R² represents alkyl, aryl, cycloalkyl, cycloalkylalkyl, and aralkyl radicals, which radicals are optionally substituted with a group selected f rom halogen radicals and -NO₂, -OR⁹ and -SR⁹ wherein R⁹ represents hydrogen and alkyl radicals;
R³ represents alkyl, alkenyl, hydroxyalkyl, cycloalkyl, cycloalkylalkyl, heterocycloalkyl, heterocycloalkylalkyl, aryl, aralkyl, heteroaryl and heteroaralkyl radicals;
R⁴ and R⁵ independently represent hydrogen and radicals as defined by R³, or together with the nitrogen atom to which they are bonded represent a heterocycloalkyl or heteroaryl radical,
t represents, 0 or 1.; and
Y represents O or S.
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro,
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom.
- heteroaryl is an aromatic monocyclic, bicyclic, or tricyclic heterocycle which contains the hetero atoms and is optionally substituted as defined above with respect to the definition of heterocycloalkyl.
- heteroaralkyl is an alkyl radical having 1 to 10 carbon atoms in which one hydrogen is replaced by a heteroaryl.
Preferably Y represent O.

Preferably, R³ represents radicals as defined above which contain no α-branching, e.g., as in an isopropyl radical or a t-butyl radical. The preferred radicals are those which contain a -CH₂- moiety between the nitrogen of the urea and the remaining portion of the radical. Such preferred groups include, but are not limited to, benzyl, isobutyl, n-butyl, isoamyl and cyclohexylmethyl.

As utilized herein, the term "alkyl", alone or in combination, means a straight-chain or branched-chain alkyl radical containing from 1 to 10, preferably from 1 to 8, carbon atoms. Examples of such radicals include methyl, ethyl, n-propyl, isopropyl, n-butyl, isobutyl, sec-butyl, tert-butyl, pentyl, isoamyl, hexyl and octyl. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy and tert-butoxy. The term "cycloalkyl" means an alkyl radical which contains from about 3 to 8 carbon atoms and is cyclic. The term "cycloalkylalkyl" means an alkyl radical as defined above which is substituted by a cycloalkyl radical containing from about 3 to 8, preferably from about 3 to 6, carbon atoms. Examples of such cycloalkyl radicals include cyclopropyl, cyclobutyl, cyclopentyl and cyclohexyl. The term "aryl", alone or in combination, means a phenyl or naphthyl radical which optionally carries one or more substituents selected from alkyl, alkoxy, halogen, hydroxy, amino and nitro, such as phenyl, p-tolyl, 4-methoxyphenyl, 4-(tert-butoxy)phenyl, 4-fluorophenyl, 4-chlorophenyl, 4-hydroxyphenyl, 1-naphthyl and 2-naphthyl. The term "aralkyl", alone or in combination, means an alkyl radical as defined above in which one hydrogen atom is replaced by an aryl radical as defined above, such as benzyl and 2-phenylethyl. The term "aralkoxy carbonyl", alone or in combination, means a radical of the formula -C(O)-O-aralkyl in which the term "aralkyl" has the significance given above. An example of an aralkoxycarbonyl radical is benzyloxycarbonyl. The term "aryloxy" means a radical of the formula aryl-o- in which the term aryl has the significance given above. The term "alkanoyl", alone or in combination, means an acyl radical derived from an alkanecarboxylic acid, examples of which include acetyl, propionyl, butyryl, valeryl and 4-methylvaleryl. The term "cycloalkylcarbonyl" means an acyl group derived from a monocyclic or bridged cycloalkanecarboxylic acid such as cyclopropanecarbonyl, cyclohexanecarbonyl and adamantanecarbonyl or from a benz-fused monocyclic cycloalkanecarboxylic acid which is optionally substituted by, for example, alkanoylamino, such as 1,2,3,4-tetrahydro-2-naphthoyl,2-acetamido-1,2,3,4-tetrahydro-2-naphthoyl. The term "aralkanoyl" means an acyl radical derived from an aryl-substituted alkanecarboxylic acid such as phenylacetyl, 3-phenylpropionyl (hydrocinnamoyl), 4-phenylbutyryl, (2-naphthyl)acetyl, 4-chlorohydrocinnamoyl, 4-aminohydroinnamoyl and 4-methoxyhydrocinnamoyl.

The term "aroyl" means an acyl radical derived from an aromatic carboxylic acid. Examples of such radicals include aromatic carboxylic acids, an optionally substituted benzoic or naphthoic acid such as benzoyl, 4-chlorobenzoyl, 4-carboxybenzoyl, 4-(benzyloxycarbonyl)benzoyl, 1-naphthoyl, 2-naphthoyl, 6-carboxy-2 naphthoyl, 6-(benzyloxycarbonyl)-2-naphthoyl, 3-benzyloxy-2-naphthoyl, 3-hydroxy-2-naphthoyl and 3-(benzyloxyformamido)-2-naphthoyl. The heterocyclyl or heterocycloalkyl portion of a heterocyclylcarbonyl, heterocyclyloxycarbonyl, heterocyclylalkoxycarbonyl, or heterocyclyalkyl group or the like is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy and oxo, and/or on a secondary nitrogen atom (i.e., -NH-) by alkyl, aralkoxycarbonyl, alkanoyl, phenyl or phenylalkyl or on a tertiary nitrogen atom (i.e. = N-) by oxido and which is attached via a carbon atom. The heteroaryl portion of a heteroaroyl, heteroaryloxycarbonyl, or a heteroaralkoxy carbonyl group or the like is an aromatic monocyclic, bicyclic, or tricyclic heterocycle which contains the hetero atoms and is optionally substituted as defined above with respect to the definition of heterocyclyl. Examples of such heterocyclyl and heteroaryl groups are pyrrolidinyl, piperidinyl, piperazinyl, morpholinyl, thiamorpholinyl, pyrrolyl, imidazolyl (e.g., imidazol 4-yl, 1-benzyloxycarbonylimidazol-4-yl, etc.), pyrazolyl, pyridyl, pyrazinyl, pyrimidinyl, furyl, thienyl, triazolyl, oxazolyl, thiazolyl, indolyl (e.g., 2-indolyl, etc.), quinolinyl, (e.g., 2-quinolinyl, 3-quinolinyl, 1-oxido-2-quinolinyl, etc.), isoquinolinyl (e.g., 1-isoquinolinyl, 3-isoquinolinyl, etc.), tetrahydroquinolinyl (e.g., 1,2,3,4-tetrahydro-2-quinolyl, etc.), 1,2,3,4-tetrahydroisoquinolinyl (e.g., 1,2,3,4-tetrahydro-1-oxo-isoquinolinyl, etc.), quinoxalinyl, β-carbolinyl, 2-benzofurancarbonyl, 1-,2-,4- or 5-benzimidazolyl, and the like. The term "cycloalkylalkoxycarbonyl" means an acyl group derived from a cycloalkylalkoxycarboxylic acid of the formula cycloalkylalkyl-O-COOH wherein cycloalkylalkyl has the significance given above. The term "aryloxyalkanoyl" means an acyl radical of the formula aryl-O-alkanoyl wherein aryl and alkanoyl have the significance given above. The term "heterocyclyloxycarbonyl" means an acyl group derived from heterocyclyl-O-COOH wherein heterocyclyl is as defined above. The term "heterocyclylalkanoyl" is an acyl radical derived from a heterocyclyl-substituted alkane carboxylic acid wherein heterocyclyl has the significance given above. The term "heterocyclylalkoxycarbonyl" means an acyl radical derived from a heterocyclyl-substituted alkane-O-COOH wherein heterocyclyl has the significance given above. The term "heteroaryloxycarbonyl" means an acyl radical derived from a carboxylic acid represented by heteroaryl-O-COOH wherein heteroaryl has the significance given above. The term "aminoalkanoyl" means an acyl group derived from an amino-substituted alkanecarboxylic acid wherein the amino group can be a primary, secondary or tertiary amino group containing substituents selected from hydrogen, and alkyl, aryl, aralkyl, cycloalkyl, cycloalkylalkyl radicals and the like. The term "halogen" means fluorine, chlorine, bromine or iodine. The term "leaving group" generally refers to groups readily displaceable by a nucleophile, such as an amine, a thiol or an alcohol nucleophile. Such leaving groups are well known and include carboxylates, N-hydroxysuccinimide, N-hydroxybenzotriazole, halides, triflates, tosylates -OR and -SR and the like. Preferred leaving groups are indicated herein where appropriate.

Procedures for preparing the compounds of Formula I are set forth below. It should be noted that the general procedure is shown as it relates to preparation of compounds having the specified stereochemistry, namely wherein the stereochemistry about the hydroxyl group is designated as (R).

### Preparation of Compounds of Formula I

The compounds of the present invention represented by Formula I above can be prepared utilizing the following general procedure. An N-protected chloroketone derivative of an amino acid having the formula: 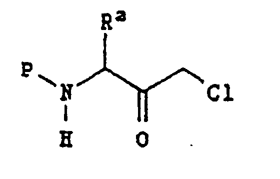 wherein P represents an amino protecting group, and R² is as defined above, is reduced to the corresponding alcohol utilizing an appropriate reducing agent. Suitable amino protecting groups are well known in the art and include carbobenzoxy, butyryl, t-butoxycarbonyl, acetyl, benzoyl and the like. A preferred amino protecting group is.carbobenzoxy. A preferred N-protected chloroketone is N-benzyloxycarbonyl-L-phenylalanine chloromethyl ketone. A preferred reducing agent is sodium borohydride. The reduction reaction is conducted at a temperature of from -10°C to about 25°C, preferably at about 0°C, in a suitable solvent system such as, for example, tetrahydrofuran, and the like. The N-protected chloroketones are commercially available from Bachem, Inc., Torrance, California. Alternatively, the chloroketones can be prepared by the procedure set forth in S. J. Fittkau, J. Prakt. Chem., 315, 1037 (1973), and subsequently N-protected utilizing procedures which are well known in the art.

The resulting alcohol is then reacted, preferably at room temperature, with a suitable base in a suitable solvent system to produce an N-protected amino epoxide of the formula: 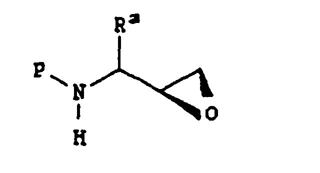 wherein P and R² are as defined above. Suitable solvent systems for preparing the amino epoxide include ethanol, methanol, isopropanol, tetrahydrofuran, dioxane, and the like including mixtures thereof. Suitable bases for producing the epoxide from the reduced chloroketone include potassium hydroxide, sodium hydroxide, potassium t-butoxide, DBU and the like. A preferred base is potassium hydroxide.

The amino epoxide is then reacted, in a suitable solvent system, with an equal amount, or preferably an excess of, a desired amine of the formula:

R³NH₂

wherein R³ is hydrogen or is as defined above. The reaction can be conducted over a wide range of temperatures, e.g., from about 10°C to about 100°C, but is preferably, but not necessarily, conducted at a temperature at which the solvent begins to reflux. Suitable solvent systems include those wherein the solvent is an alcohol, such as methanol, ethanol, isopropanol, and the like, ethers such as tetrahydrofuran, dioxane and the like, and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine, naphthylene methyl amine and the like. The resulting product is a 3-(N-protected amino)-3-(R²)-1-(NHR³)-propan-2-ol derivative (hereinafter referred to as an amino alcohol) can be represented by the formula: 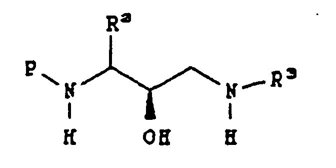 wherein P, R² and R³ are as described above.

For producing compounds of Formula I wherein R⁵ is hydrogen, the resulting amino alcohol described above is then reacted, in a suitable solvent system, with an isocyanate of the formula R⁴NCO wherein R⁴ is as defined above. Where Y in Formula I above is sulfur, the resulting amino alcohol is reacted with an isothiocyanate of the formula R⁴NCS under similar conditions. Suitable solvent systems include tetrahydrofuran, methylene chloride, and the like and mixtures thereof. The resulting product is a urea derivative of the amino alcohol or the corresponding sulfur analog thereof and can be represented by the formula: 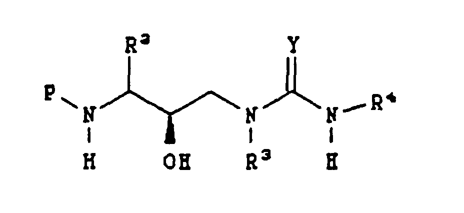 wherein P, Y, R², R³ and R⁴ are as defined above. The isocyanates of the formula R⁴NCO can be prepared by the reaction of an amine (R³NH₂) with phosgene, triphosgene, carbodiimidazole, or carbonate ((RO)₂CO) under conditions well-known in the art. The isothiocyanates of the formula R₄NCS can be prepared by similar procedures, e.g., reaction of the amine with thiophosgene, which are also well known in the art. In addition, the isocyanates and isothiocyanates are commercially available from Aldrich Chemical Company.

For preparing compounds of Formula I wherein R⁵ is other than hydrogen, the resulting amino alcohol described above is then reacted, in a suitable solvent system, with a compound represented by the formula: 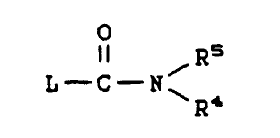 wherein R⁴ and R⁵ are as described above and L represents a leaving group such as a halide, e.g., chloride, imidazole radical, the radical p-NO₂-(C₆H₄)-O-, and the like. A preferred compound represented by this formula is a carbamoyl chloride. The corresponding sulfur analogs can be utilized where Y of Formula I is S.

The urea derivative of the amino alcohol and the corresponding sulfur analog can be represented by the formula:

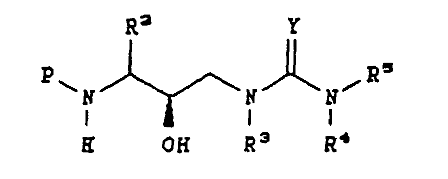

Following preparation of the urea derivative, or corresponding analogs wherein Y is S, the amino protecting group P is removed under conditions which will not affect the remaining portion of the molecule. These methods are well known in the art and include acid hydrolysis, hydrogenolysis and the like. A preferred method involves removal of the protecting group, e.g., removal of a carbobenzoxy group, by hydrogenolysis utilizing palladium on carbon in a suitable solvent system such as an alcohol, acetic acid, and the like or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative. Following neutralization of the salt, the amine is then reacted with an amino acid or corresponding derivative thereof represented by the formula (PN[CR^{1'} R^{1"}]^{t} CH(R¹)COOH) wherein t, R¹, R^{1'} and R^{1"} are as defined above, to produce the antiviral compounds of the present invention having the formula: 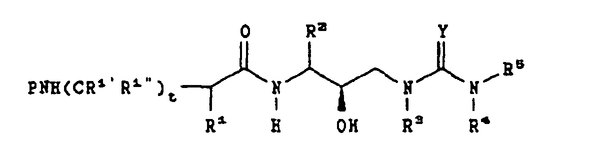 wherein t, P, R¹, R^{1'}, R^{1"}, R², R³, R⁴, R⁵ and Y are as defined above. Preferred protecting groups in this instance are a benzyloxycarbonyl group or a t-butoxycarbonyl group. Where the amine is reacted with a derivative of an amino acid, e.g., when t=1 and R^{1'} and R^{1"} are both H, so that the amino acid is a β-amino acid, such β-amino acids can be prepared according to the procedure set forth in a copending application, U. S. Serial No. 07/345,808. Where t is 1, one of R^{1'} and R^{1"} is H and R¹ is hydrogen so that the amino acid is a homo-β-amino acid, such homo-β-amino acids can be prepared by the same procedure. Where t is 0 and R¹ is alkyl, cycloalkyl, -CH₂SO₂NH₂ or an amino acid side chain, such materials are well known and many are commercially available from Sigma-Aldrich.

The N-protecting group can be subsequently removed, if desired, utilizing the procedures described above, and then reacted with a carboxylate represented by the formula: 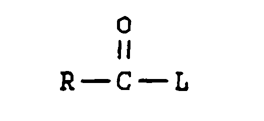 wherein R is as defined above and L is an appropriate leaving group such as a halide. Preferably, where R¹ is a side chain of a naturally occurring α-amino acid, R is a 2-quinoline carbonyl group derived from N-hydroxysuccinimide-2-quinoline carboxylate, i.e., L is hydroxy succinimide. A solution of the free amine (or amine acetate salt) and about 1.0 equivalent of the carboxylate are mixed in an appropriate solvent system and optionally treated with up to five equivalents of a base such as, for example, N-methylmorpholine, at about room temperature. Appropriate solvent systems include tetrahydrofuran, methylene chloride or N,N-dimethylformamide, and the like, including mixtures thereof.

It is contemplated that where R³ of the amino alcohol intermediate is hydrogen, the inhibitor compounds can be prepared through reductive amination of the final product of the reaction between the amino alcohol and the amine or at any other stage of the synthesis for preparing the inhibitor compounds.

Contemplated equivalents of the general formulas set forth above for the antiviral compounds and derivatives as well as the intermediates are compounds otherwise corresponding thereto and having the same general properties wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

All reagents were used as received without purification. All proton and carbon NMR spectra were obtained on either a Varian VXR-300 or VXR-400 nuclear magnetic resonance spectrometer.

### Example 1

### Preparation of [1S-[1R*(R*), 2S*]]- N¹[3-[[[(1,1-dimethylethyl)amino]carbonyl](2-methylpropyl)amino]-2-hydroxy-1-(phenylmethyl)propyl]-2-[(2-quinolinylcarbonyl)amino]-butanediamide

### Part A:

To a solution of 75.0g (0.226 mol) of N-benzyloxycarbonyl-L-phenylalanine chloromethyl ketone in a mixture of 807 mL of methanol and 807 mL of tetrahydrofuran at -2°C, was added 13.17g (0.348 mol, 1.54 equiv.) of solid sodium borohydride over one hundred minutes. The solvents were removed under reduced pressure at 40°C and the residue dissolved in ethyl acetate (approx. 1L). The solution was washed sequentially with 1M potassium hydrogen sulfate, saturated sodium bicarbonate and then saturated sodium chloride solutions. After drying over anhydrous magnesium sulfate and filtering, the solution was removed under reduced pressure. To the resulting oil was added hexane (approx. 1L) and the mixture warmed to 60°C with swirling. After cooling to room temperature, the solids were collected and washed with 2L of hexane. The resulting solid was recrystallized from hot ethyl acetate and hexane to afford 32.3g (43% yield) of N-benzyloxycarbonyl-3(S)-amino-1-chloro-4-phenyl-2(S)-butanol, mp 150-151°C and M+Li⁺ = 340.

### Part B:

To a solution of 6.52g (0.116 mol, 1.2 equiv.) of potassium hydroxide in 968 mL of absolute ethanol at room temperature, was added 32.3g (0.097 mol) of N-CBZ-3(S)-amino-1-chloro-4-phenyl-2(S)-butanol. After stirring for fifteen minutes, the solvent was removed under reduced pressure and the solids dissolved in methylene chloride. After washing with water, drying over magnesium sulfate, filtering and stripping, one obtains 27.9g of a white solid. Recrystallization from hot ethyl acetate and hexane afforded 22.3g (77% yield) of N-benzyloxycarbonyl-3(S)-amino-1,2(S)-epoxy-4-phenylbutane, mp 102-103°C and MH⁺ 298.

### Part C:

A solution of N-benzyloxycarbonyl 3(S)-amino-1,2-(S)-epoxy-4-phenylbutane (1.00g, 3.36 mmol) and isobutylamine (4.90g, 67.2 mmol, 20 equiv.) in 10 mL of isopropyl alcohol was heated to reflux for 1.5 hours. The solution was cooled to room temperature, concentrated *in vacuo* and then poured into 100 mL of stirring hexane whereupon the product crystallized from solution. The product was isolated by filtration and air dried to give 1.18g, 95% of N=[[3(S)-phenylmethylcarbamoyl)amino-2(R)-hydroxy-4-phenylbutyl]N-[(2-methylpropyl)]amine mp 108.0-109.5°C, MH⁺ m/z = 371.

### Part D:

A solution of [2(R), 3(S)]-N-[[3-(phenylmethylcarbamoyl)amino]-2-hydroxy-4-phenylbutyl]N-[(2-methylpropyl)]amine in 10 ml of tetrahydrofuran was treated with tert-butylisocyanate (267 mg, 2.70 mmol) at room temperature for 5 minutes. The solvent was removed *in vacuo* and replaced with ethyl acetate. The ethyl acetate solution was washed with 5% citric acid, water, and brine, dried over anhydrous MgSO₄, filtered and concentrated in vacuo to give 1.19g, 97% of [2(R), 3(S)]-N-[[3-(phenylmethylcarbamoyl)amino]-2-hydroxy-4-phenyl]-1-[(2-methylpropyl)]amino-2-(1,1-dimethyl)amino]carbonyl]butane, MH⁺ m/z - 470.

### Part E:

A solution of (1.00g, 2.21 mmol) [2(R), 3(S)]-N-[[3-(phenylmethylcarbamoyl)amino]-2-hydroxy-4-phenyl]-1-[(2-methylpropyl)]amino-1-(1,1-dimethylethyl)amino]carbonyl]butane in 20 mL of methanol was hydrogenated over 10% palladium-on-carbon for 4 hours to give [2(R), 3(S)]-N-[[3-amino]-2-hydroxy-4-phenyl]-1-[(2-methylpropyl)amino-1-(1,1-dimethylethyl)amino]carbonyl]butane 720 mg, 97%.

### Part F:

A solution of N-Cbz-L-asparagine (602mg, 2.26 mmol) and N-hydroxybenzotriazole (493 mg, 3.22 mmol) in 2mL of dimethylformamide was cooled to 0°C and treated with EDC (473 mg, 2.47 mmol). The solution was allowed to stir at 0°C for 20 minutes and then treated with [2(R), 3(S)]-N-[[3-amino]-2-hydroxy-4-phenyl]-1-[(2-methylpropyl)]amino-1-(1,1-dimethylethyl)amino]carbonyl]butane (720 mg, 2.15 mmol) in 1mL of dimethylformamide. The solution was allowed to warm to room temperature and held at this temperature for 7 hours. The reaction mixture was then poured into 100 mL of 60% saturated aqueous sodium bicarbonate whereupon a white precipitate formed that was isolated by filtration. The filter cake was washed with water, 5% aqueous citric acid, water and then dried *in vacuo* to give 1.04g, 83% of [1S-[1R*(R*), 2S*]]- N¹[3-[[[(1,1-dimethylethyl)amino]carbonyl](2-methylpropyl)amino], mp. 164.0-166.5°C, MH⁺ m/z = 584.

### Part G.

A solution of [1S-[1R*(R*), 2S*]]- N¹[3-[[[(1,1-dimethylethyl)amino]carbonyl](2-methylpropyl)amino]-2-hydroxy-1-(phenylmethyl)propyl]-2-[(phenylmethylcarbamoyl)amino]-butanediamide (1.00g, 1.72 mmol) in 10 mL of methanol was hydrogenated over 10% palladium-on-carbon for 4 hours to give [1S-[1R*(R*), 2S*]]- N¹[3-[[[(1,1-dimethylethyl)amino]carbonyl](2-methylpropyl)amino]-2-hydroxy-1-(phenylmethyl)propyl]-2-amino]-butanediamide, 784mg, 99%.

### Part H:

A mixture of [1S-[1R*(R*), 2S*]]- N¹[3-[[[(1,1-dimethylethyl)amino]carbonyl](2-methylpropyl)amino]-2-hydroxy-1-(phenylmethyl)propyl]-2-amino]-butanediamide, (784 mg, 1.70 mmol), 2-quinoline carboxylic acid N-hydroxysuccinimide ester (459 mg, 1.70 mmol), N-methylmorpholine (343 mg, 3.40 mmol) in 5 mL of dichloromethane was stirred at room temperature for 15 minutes. The solvent was removed *in vacuo* and replaced with ethyl acetate and the solution washed with 5% aqueous citric acid, saturated aqueous sodium bicarbonate, brine, dried over anhydrous MgSO₄, filtered and concentrated *in vacuo*. The crude product was recrystallized from acetone/hexane to give 790 mg, 77% of [1S-[1R*(R*), 2S*]]- N¹[3-[[[(1,1-dimethylethyl)amino]carbonyl](2-methylpropyl)amino]-2-hydroxy-1-(phenylmethyl)propyl]-2-[(2-quinolinylcarbonyl)amino]-butanediamide, mp 107.0-109.8°C, MH⁺ = 605.

### Example 2

The procedure described in Example 1, part C-H, was used to prepare [1S-[1R*(R*), 2S*]]- N¹[3-[[[(1,1-dimethylethyl)amino]carbonyl](3-methylbutyl)amino]-2-hydroxy-1-(phenylmethyl)propyl]-2-[(2-quinolinylcarbonyl)amino]-butanediamide.
a) From the reaction of 1.06g (3.56mmol) of N-benzyloxycarbonyl 3(S)-amino-1,2-(S)-epoxy-4-phenylbutane and 6.25g (71.7mmol) of isoamylamine, one obtains 1.27g (92%) of [2(R), 3(S)]-N-[[3-(phenylmethylcarbamoyl)amino]-2-hydroxy-4-phenylbutyl]N-[(3-methylbutyl)]amine, mp 130-132°C and MH* 385. This amine (400mg, 1.04mmol) was then reacted with tert-butylisocyanate (110mg, 1.11mmol) to afford 500mg (100%) of [2(R), 3(S)]-N-[[3-(phenylmethylcarbamoyl)amino]-2-hydroxy-4-phenyl]-1-[(3-methylbutyl)]amino-1-(1,1-dimethylethy)amino]carbonyl]butane; as an oil, MH⁺ 484.
b) The CBZ protected compound (530mg, 1.10mmol) was then deprotected by hydrogenation over 10% palladium-on-carbon and the resulting free amine coupled with N-CBZ-L-asparagine (377mg, 1.42mmol) in the presence of N-hydroxybenzotriazole (290mg, 2.15mmol) and EDC (300mg, 1.56mmol) to yield 430mg (53%) of [1S-[1R*(R*), 2S*]]- N¹[3-[[[(1,1-dimethylethyl)amino]carbonyl](3-methylbutyl)amino]-2-hydroxy-1-(phenylmethyl)propyl]-2-[(phenylmethylcarbamoyl)amino]-butanediamide, mp 148-151°C (dec) and MH⁺ 598. This compound (370mg, 0.619mmol) was then deprotected by hydrogenation over 10% palladium-on-carbon and the resulting free amine coupled with 2-quinolinecarboxylic acid N-hydroxy-succinimide ester (193mg, 0.714mmol), in the presence of N-methylmorpholine, to afford 310mg (70%) of pure [1S-[1R*(R*), 2S*]]- N¹[3-[[[(1,1-dimethylethyl)amino]carbonyl](3-methylbutyl)amino]-2-hydroxy-1-(phenylmethyl)propyl]-2-[(2-quinolinylcarbonyl)amino]-butanediamide; mp 93.5-95.5°C and MH⁺ 619.

### Example 3

The procedure described in Example 1, part C-H, was used to prepare [1S-[1R*(R*), 2S*]]- N¹[3-[[[(1,1-dimethylethyl)amino]carbonyl]2-napthylmethyl)amino]-2-hydroxy-1-(phenylmethyl)propyl]-2-[(2-quinolinylcarbonyl)amio]-butanediamide.
a) From the reaction of 1.80g (6.05mmol) of N-benzyloxycarbonyl 3(S)-amino-1,2-(S)-epoxy-4-phenylbutane and 1.15g (7.31mmol) of 2-(aminomethyl)naphthalene, one obtains 2.11g (77%) of [2(R), 3(S)]-N-[[3-(phenylmethylcarbamoyl)amino]-2-hydroxy-4-phenylbutyl]N-[(2-napthylmethyl)]amine, MH⁺ 455. This amine (366.8mg, 0.807mmol) was then reacted with tert-butylisocyanate (66.4mg, 0.67mmol) to afford 350.0mg (94%) of [2(R), 3(S)]-N-[[3-(phenylmethylcarbamoyl)amino]-2-hydroxy-4-phenyl]-1-[(2-napthylmethyl)]amino-1-(1,1-dimethylethyl)amino]carbonyl]butane; as an oil, MH⁺ 554.
b) The CBZ protected compound (330mg, 0.596mmol) was then deprotected by hydrogenation over 10% palladium-on-carbon and the resulting free amine coupled with N-CBZ-L-asparagine (165.1mg, 0.62mmol) in the presence of N-hydroxybenzotriazole (142.3mg, 0.93mmol) and EDC (130.7mg, 0.68mmol) to yield 161.7mg (41%) of [1S-[1R*(R*), 2S*]]- N¹[3-[[[(1,1-dimethylethyl)amino]carbonyl](2-napthylmethyl)amino]-2-hydroxy-1-(phenylmethyl)propyl]-2-[(phenylmethylcarbamoyl)amino]-butanediamide; mp 151-152°C (dec) and MH⁺ 668. This compound (91.0mg, 0.136mmol) was then deprotected by hydrogenation over 10% palladium-on-carbon and the resulting free amine coupled with 2-quinolinecarboxylic acid N-hydroxysuccinimide ester (36.8mg, 0.136mmol), in the presence of N-methylmorpholine, to afford 65.8mg (70%) of pure [1S-[1R*(R*), 2S*]]- N¹[3-[[[(1,1-dimethylethyl)amino]carbonyl](2-napthylmethyl)amino]-2-hydroxy-1-(phenylmethyl)propyl]-2-[(2-quinolinylcarbonyl)amino]-butanediamide; mp 119-120°C and MH⁺ 689.

### Example 4

The procedure described in Example 1, part C-H, was used to prepare [1S-[1R*(R*), 2S*]]- N¹[3-[[[(1,1-dimethylethyl)amino]carbonyl](2-phenylethyl)amino]-2-hydroxy-1-(phenylmethyl)propyl]-2-[(2-quinolinylcarbonyl)amino]-butanediamide.
a) From the reaction of 1.00g (3.36mmol) of N-benzyloxycarbonyl 3(S)-amino-1,2-(S)-epoxy-4-phenylbutane and 8.19g (67.0mmol) of 2-phenethyl amine, one obtains 1.10g (79%) of [2(R), 3(S)]-N-[[3-(phenylmethylcarbamoyl)amino]-2-hydroxy-4-phenylbutyl]N-[(2-phenylethyl)]amine, mp 137-138°C and MH⁺ 419. This amine (750mg, 1.79mmol) was then reacted with tert-butylisocyanate (178mg, 1.79mmol) to afford 897mg ■ (97%) of [2(R), 3(S)]-N-[[3-(phenylmethylcarbamoyl)amino]-2-hydroxy-4-phenyl]-1-[(2-phenylethyl)]amino-1-(1,1-dimethylethyl)amino]carbonyl]butane; as an oil, MH⁺ 518.
b) The CBZ protected compound (897mg, 1.73mmol) was then deprotected by hydrogenation over 10% palladium-on-carbon and the resulting free amine coupled with N-CBZ-L-asparagine (620.7mg, 2.33mmol) in the presence of N-hydroxybenzotriazole (509.5mg, 3.33mmol) and EDC (488.0mg, 2.55mmol) to yield 1.00g (92%) of [1S-[1R*(R*), 2S*]]- N¹[3[[[(1,1-dimethylethyl)amino]carbonyl](2-phenylethyl)amino]-2-hydroxy-1-(phenylmethyl)propyl]-2-[(phenylmethylcarbamoyl)amino]-butanediamide; mp 145°C (dec) and MH⁺ 632. This compound (860mg, 1.36mmol) was then deprotected by hydrogenation over 10% palladium-on-carbon and the resulting free amine coupled with 2-quinolinecarboxylic acid N-hydroxysuccinimide ester (338mg, 1.25mmol), in the presence of N-methylmorpholine, to afford 450.4mg (55%) of pure [1S-[1R*(R*), 2S*]]- N¹[3[[[(1,1-dimethylethyl)amino]carbonyl](2-phenylethyl)amino]-2-hydroxy-1-(phenylmethyl)propyl]-2-[(2-quinolinylcarbonyl)amino]-butanediamide; mp 139-140°C and MH⁺ 653.

### Example 5

The procedure described in Example 1, part C-H, was used to prepare [1S-[1R*(R*), 2S*]]- N¹[3-[[[(1,1-dimethylethyl)amino]carbonyl](2,2-dimethylpropyl)amino]-2-hydroxy-1-(phenylmethyl)propyl]-2-[(2-quinolinylcarbonyl)amino]-butanediamide.
a) From the reaction of 1.00g (3.36mmol) of N-benzyloxycarbonyl 3(S)-amino-1,2-(S)-epoxy-4-phenylbutane and 7.9mL (approx. 67mmol) of neopentyl amine, one obtains 0.69g (49%) of [2(R), 3(S)]-N-[[3-(phenylmethylcarbamoyl)amino]-2-hydroxy-4-phenylbutyl]N-[(2,2-dimethylpropyl)]amine, MH⁺ 385. This amine (686mg, 1.78mmol) was then reacted with tert-butylisocyanate (180mg, 1.78mmol) to afford 860mg (100%) of [2(R), 3(S)]-N-[[3-(phenylmethylcarbamoyl)amino]-2-hydroxy-4-phenyl]-1-[(2,2-dimethylpropyl)]amino-1-(1,1-dimethylethyl)amino]carbonyl]butane; MH⁺ 484.
b) The CBZ protected compound (860mg, 1.78mmol) was then deprotected by hydrogenation over 10% palladium-on-carbon and the resulting free amine coupled with N-CBZ-L-asparagine (471mg, 1.77mmol) in the presence of N-hydroxybenzotriazole (406mg, 2.66mmol) and EDC (374mg, 1.95mmol) to yield 326mg (34%) of [1S-(R*(R*), 2S*]]- N¹[3-[[[(1,1-dimethylethyl)amino]carbonyl](2,2-dimethylpropyl)amino]-2-hydroxy-1-(phenylmethyl)propyl]-2-[(phenyimethylcarbamoyl)amino]-butanediamide; mp 177-178°C and MH⁺ 598. This compound (245mg, 0.41mmol) was then deprotected by hydrogenation over 10% palladium-on-carbon and the resulting free amine coupled with 2-quinolinecarboxylic acid N-hydroxysuccinimide ester (111mg, 0.41mmol), in the presence of N-methylmorpholine, to afford 150mg (59%) of pure [1S-[R*(R*), 2S*]]- N¹[3-[[[(1,1-dimethylethyl)amino]carbonyl](2,2-dimethylpropyl)amino]-2-hydroxy-1-(phenylmethyl)propyl]-2-[(2-quinolinylcarbonyl)amino]-butanediamide; mp 115-117°C and MH⁺ 619.

### Example 6

The procedure described in Example 1, part C-H, was used to prepare [1S-[R*(R*), 2S*]]- N¹[3-[[[(1,1-dimethylethyl)amino]carbonyl](4-methoxyphenylmethyl)amino]-2-hydroxy-1-(phenylmethyl)propyl]-2-[(2-quinolinylcarbonyl)amino]-butanediamide;
a) From the reaction of 1.00g (3.36mmol) of N-benzyloxycarbonyl 3(S)-amino-1,2-(S)-epoxy-4-phenylbutane and 9.2g (67mmol) of 4-methoxybenzyl amine, one obtains 1.12g (76%) of [2(R), 3(S)]-N-[(3-(phenylmethylcarbamoyl)amino]-2-hydroxy-4-phenylbutyl]N-[(4-methoxyphenylmethyl)]amine, MH⁺ 435. This amine (1.12g, 2.58mmol) was then reacted with tert-butylisocyanate (260mg, 2.58mmol) to afford 1.35g (98%) of [2(R), 3(S)]-N-[[3-(phenylmethylcarbamoyl)amino]-2-hydroxy-4-phenyl]-1-[(4-methoxyphenylmethyl)]amino-1-(1,1-dimethylethyl)amino]carbonyl]butane; MH⁺ 534.
b) The CBZ protected compound (1.35g, 2.53mmol) was then deprotected by hydrogenation over 10% palladium-on-carbon and the resulting free amine coupled with N-CBZ-L-asparagine (684mg, 2.57mmol) in the presence of N-hydroxybenzotriazole (590mg, 3.85mmol) and EDC (543mg, 2.83mmol) to yield 442mg (29%) of [1S-[1R*(R*), 2S*]]- N¹[3-[[[(1,1-dimethylethyl)amino]carbonyl](4-methoxyphenylmethyl)amino]-2-hydroxy-1-(phenylmethyl)propyl]-2-[phenylmethylcarbamoyl)amino]-butanediamide; mp 175°C (dec) and MH⁺ 648. This compound (345mg, 0.53mmol) was then deprotected by hydrogenation over 10% palladium-on-carbon and the resulting free amine coupled with 2-quinolinecarboxylic acid N-hydroxysuccinimide ester (118mg, 0.44mmol), in the presence of N-methylmorpholine, to afford 108mg (31%) of pure [1S-[1R*(R*), 2S*]]- N¹[3-[[[(1,1-dimethylethyl)amino]carbonyl](4-methoxyphenylmethyl)amino]-2-hydroxy-1-(phenylmethyl)propyl]-2-[(2-quinolinylcarbonyl)amino]-butanediamide; mp 220°C (dec) and MLi⁺ 675.

### Example 7

The procedure described in Example 1, part C-H, was used to prepare [1S-[1R*(R*), 2S*]]- N¹[3-[[[(1,1-dimethylethyl)amino]carbonyl](n-butyl)amino]-2-hydroxy-1-(phenylmethyl)propyl]-2-[(2-quinolinylcarbonyl)amino]-butanediamide.
a) From the reaction of 1.48g (5.0mmol) of N-benzyloxycarbonyl 3(S)-amino-1,2-(S)-epoxy-4-phenylbutane and 7.314g (100.0mmol) of n-butyl amine, one obtains 1.50g (80%) of [2(R), 3(S)]-N-[[3-(phenylmethylcarbamoyl)amino]-2-hydroxy-4-phenylbutyl]N-[n-butyl)]amine. This amine (1.48g, 4.0mmol) was then reacted with tert-butylisocyanate (396mg, 4.0mmol) to afford 1.87g (100%) of [2(R), 3(S)]-N-[[3-(phenylmethylcarbamoyl)amino]-2-hydroxy-4-phenyl]-1-[(n-butyl)]amino-1-(1,1-dimethylethyl)amino]carbonyl] butane as an oil.
b) The CBZ protected compound (1.87g, 4.0mmol) was then deprotected by hydrogenation over 10% palladium-on-carbon and the resulting free amine coupled with N-CBZ-L-asparagine (1.05g, 3.96mmol) in the presence of N-hydroxybenzotriazole (535mg, 7.9mmol) and EDC (759mg, 3.96mmol) to yield 1.75g (76%) of [1S-[1R*(R*), 2S*]]- N¹[3-[[[(1,1-dimethylethyl)amino]carbonyl](n-butyl)amino]-2-hydroxy-1-(phenylmethyl)propyl]-2-[(phenylmethylcarbamoyl)amino]-butanediamide; mp 166-167°C and MH⁺ 584.

### Example 8

The procedure described in Example 1, part C-H, was used to prepare [1S-[1R*(R*), 2S*]]- N¹[3-[[[(1,1-dimethylethyl)amino]carbonyl](phenylmethyl)amino]-2-hydroxy-1-(phenylmethyl)propyl]-2-[(2-quinolinylcarbonyl)amino]-butanediamide.
a) From the reaction of 1.48g (5.0mmol) of N-benzyloxycarbonyl 3(S)-amino-1,2-(S)-epoxy-4-phenylbutane and 10.68g (100.0mmol) of benzyl amine, one obtains 1.88g (95%) of [2(R), 3(S)]-N-[[3-(phenylmethylcarbamoyl)amino]-2-hydroxy-4-phenylbutyl]N-[(phenylmethyl)]amine. This amine (1.88g, 4.65mmol) was then reacted with tert-butylisocyanate (460.0mg, 4.6mmol) to afford 2.24g (96%) of [2(R), 3(S)]-N-[[3-(phenylmethylcarbamoyl)amino]-2-hydroxy-4-phenyl]-1-[(phenylmethyl)]amino-1-(1,1-dimethylethyl)amino]carbonyl] butane.
b) The CBZ protected compound (2.22g, 4.4mmol) was then deprotected by hydrogenation over 10% palladium-on-carbon and the resulting free amine coupled with N-CBZ-L-asparagine (1.17g, 4.4mmol) in the presence of N-hydroxybenzotriazole (1.19g, 8.8mmol) and EDC (843mg, 4.4mmol) to yield 2.11g (78%) of [1S-[1R*(R*), 2S*]]- N¹[3-[[[(1,1-dimethylethyl)amino]carbonyl](phenylmethyl)amino]-2-hydroxy-1-(phenylmethyl)propyl]-2-[(phenylmethylcarbamoyl)amino]-butanediamide; mp 156-158°C and MH⁺ 618. This compound (1.0g, 1.62mmol) was then deprotected by hydrogenation over 10% palladium-on-carbon and the resulting free amine coupled with 2-quinolinecarboxylic acid N-hydroxysuccinimide ester (437mg, 1.62mmol), in the presence of N-methylmorpholine, to afford 640mg (62%) of pure [1S-[1R*(R*), 2S*]]- N¹[3-[[[(1,1-dimethylethyl)amino]carbonyl](phenylmethyl)amino]-2-hydroxy-1-(phenylmethyl)propyl]-2-[(2-quinolinylcarbonyl)amino]-butanediamide; mp 110.5-112.5°C and MH⁺ 639.

### EXAMPLE 9

Additional exemplary compounds of the present invention are listed in Table 1. These compounds were prepared according to the following general procedures.

### General Procedure for the Synthesis of 1,3-Diamino 4-phenyl Butan-2-ol Derivatives.

A mixture of the amine R³NH₂ (20 equiv.) in dry isopropyl alcohol (20mL/mmol of epoxide to be converted) was heated to reflux and then treated with an N-Cbz amino epoxide of the formula: 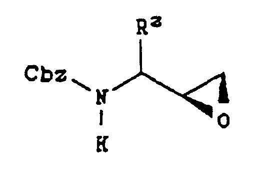 from a solids addition funnel over a 10-15 minute period. After the addition is complete the solution was maintained at reflux for an additional 15 minutes and the progress of the reaction monitored by TLC. The reaction mixture was then concentrated in vacuo to give an oil that was treated with n-hexane with rapid stirring whereupon the ring opened material precipitated from solution. Precipitation was generally complete within 1 hr and the product was then isolated by filtration on a Büchner funnel and then air dried. The product was further dried in vacuo. This method affords amino alcohols of sufficient purity for most purposes. General procedure for the Reaction of Amino Alcohols with Isocyanates: Preparation-of Ureas

A solution from the amino alcohol in tetrahydrofuran (THF) was treated at room temperature with the appropriate isocyanate of formula R⁴NCO via syringe under nitrogen. After the reaction has stirred for -5m the progress of the reaction was monitored by TLC. The solvent was removed in vacuo and the product obtained was of sufficient purity for most purposes. The product may be further purified by dissolution in ethyl acetate and washing with 5% aqueous citric acid, water, and brine. The solvent is dried over anhydrous magnesium sulfate, filtered and concentrated in vacuo to give the pure urea.

### General Procedure for the Removal of the Protecting Groups by Hydrogenolysis with Palladium on Carbon

### A. Alcohol Solvent

The Cbz-protected peptide derivative was dissolved in methanol (ca.2omL/mmol) and 10% palladium on carbon catalyst is added under a nitrogen atmosphere. The reaction vessel is sealed and flushed 5 times with nitrogen and then 5 times with hydrogen. The pressure is maintained at 50 psig for 1-16 hours and then the hydrogen replaced with nitrogen and the solution filtered through a pad of celite to remove the catalyst. The solvent is removed in vacuo to give the free amino derivative of suitable purity to be taken directly on to the next step.

### B. Acetic Acid Solvent

The Cbz-protected peptide derivative was dissolved in glacial acetic acid (20mL/mmol) and 10% palladium on carbon catalyst is added under a nitrogen atmosphere. The reaction vessel is flushed 5 times with nitrogen and 5 times with hydrogen and then maintained at 40 psig for about 2h. The hydrogen was then replaced with nitrogen and the reaction mixture filtered through a pad of celite to remove the catalyst. The filtrate was concentrated and the resulting product taken up in anhydrous ether and evaporated to dryness 3 times. The final product, the acetate salt, was dried in vacuo and is of suitable purity for subsequent conversion.

### General Procedure for Removal of Boc-protecting Group with 4N Hydrochloric Acid in Dioxane

The Boc-protected amino acid or peptide is treated with a solution of 4N HCl in dioxane with stirring at room temperature. Generally the deprotection reaction is complete within 15 minutes, the progress of the reaction is monitored by thin layer chromatography (TLC). Upon completion, the excess dioxane and HCl are removed by evaporation in vacuo. The last traces of dioxane and HCl are best removed by evaporation again from anhydrous ether or acetone. The hydrochloride salt thus obtained is thoroughly dried in vacuo and is suitable for further reaction.

### EDC/HOHt Coupling of Cbz-Asparagine (General Procedure)

N-CBZ-(L-asparagine (1.10eq) and N-hydroxybenzotriazole (HOBt) (1.5eq) are dissolved in dry dimethylformamide (DMF) (2-5mL/mmol) and cooled in an ice bath. 1-(3-dimethylaminopropyl)-3-ethylcarbodiimide hydrochloride (EDC) (1.10eq) is added to the stirring solution and maintained at 0°C for 10 minutes. A solution of the amino component (free amine), 1.0eq in DMF (1-2mL/mmol), is added. (In the case of the amine hydrochloride or acetate salt, an equivalent of N-methylmorpholine is also added.] The reaction mixture is stirred at 0°C for 1 hour and then at room temperature for -5-6 hours. The reaction mixture is then poured into a rapidly stirring solution of 60% saturated aqueous sodium bicarbonate (ca-50mL/mmol). An immediate white precipitate forms which is collected on a Büchner funnel and the solid washed thoroughly with saturated aqueous sodium bicarbonate, water, 5% aqueous citric acid solution and water. The product is thoroughly dried in vacuo and redissolved in DMF, filtered and reprecipitated by the addition to water. The precipitated product is isolated by filtration, washed again with water and dried in vacuo.

### General Procedure for Acylation with 2-Quinoline Carboxylic Acid N-Hydroxysuccinimide Ester

A solution of the free amine (or amine acetate salt) and 1.0 equivalent of N-hydroxysuccinimide 2-quinoline carboxylate in anhydrous dichloromethane was treated with 1.5 equivalents of N-methylmorpholine (NMM) at room temperature. The progress of the reaction was monitored by TLC and when the reaction was complete the reaction mixture was diluted with additional dichloromethane and the solution washed with saturated aqueous sodium bicarbonate, 5% aqueous citric acid, water and brine. The solution was dried over anhydrous magnesium sulfate, filtered and concentrated in vacuo. The product thus obtained was recrystallized from a mixture of acetone and hexane.

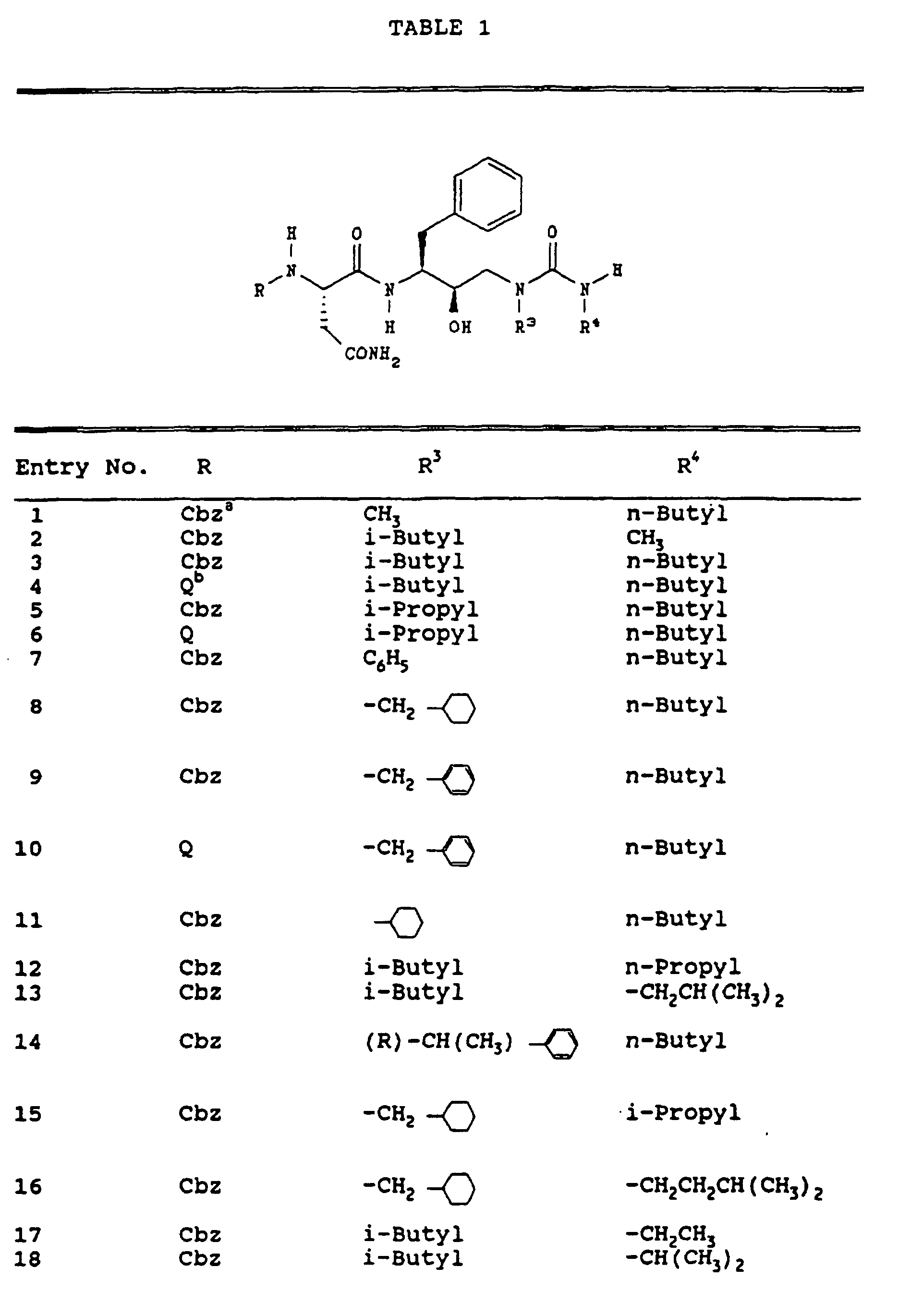

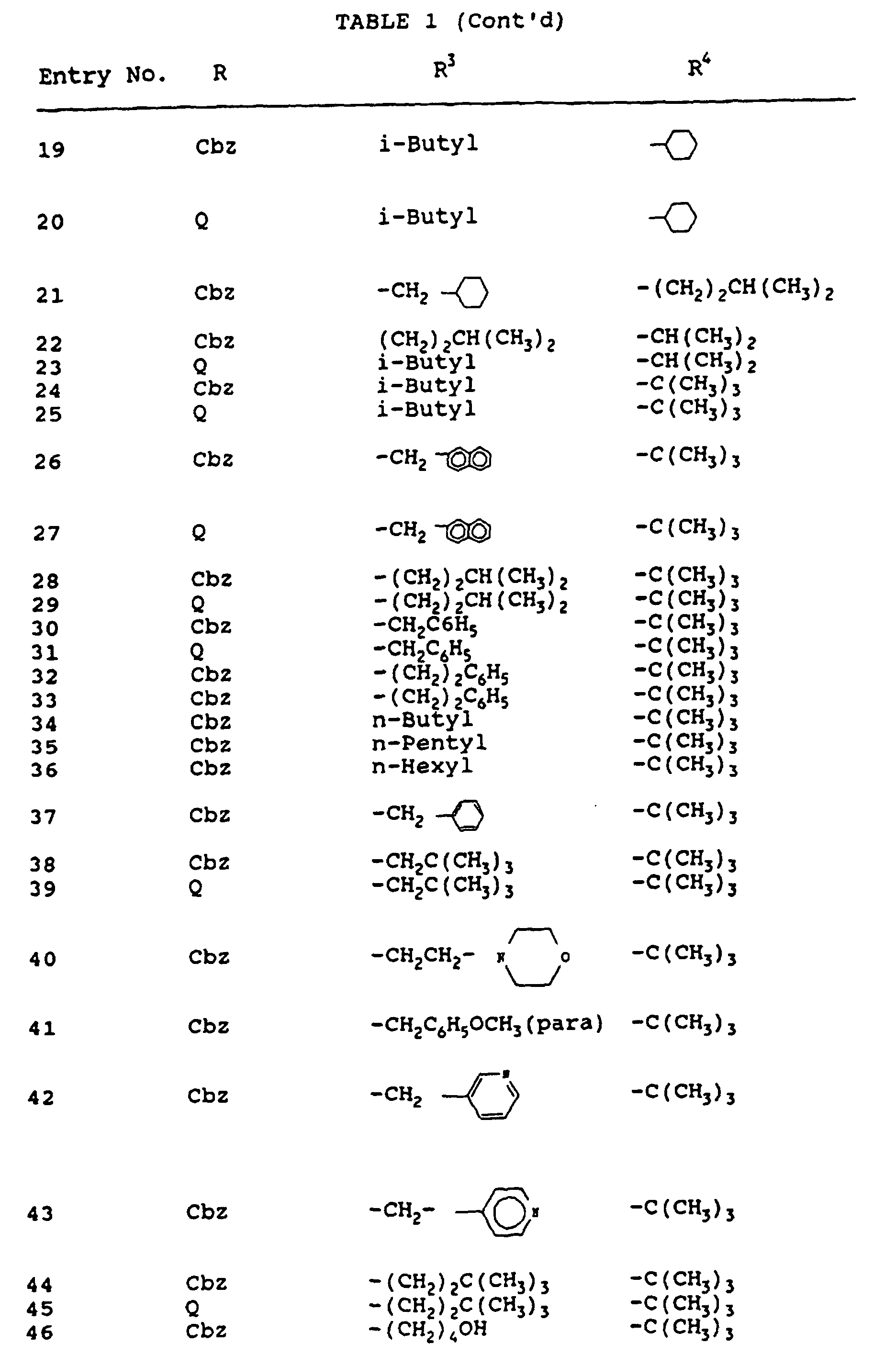

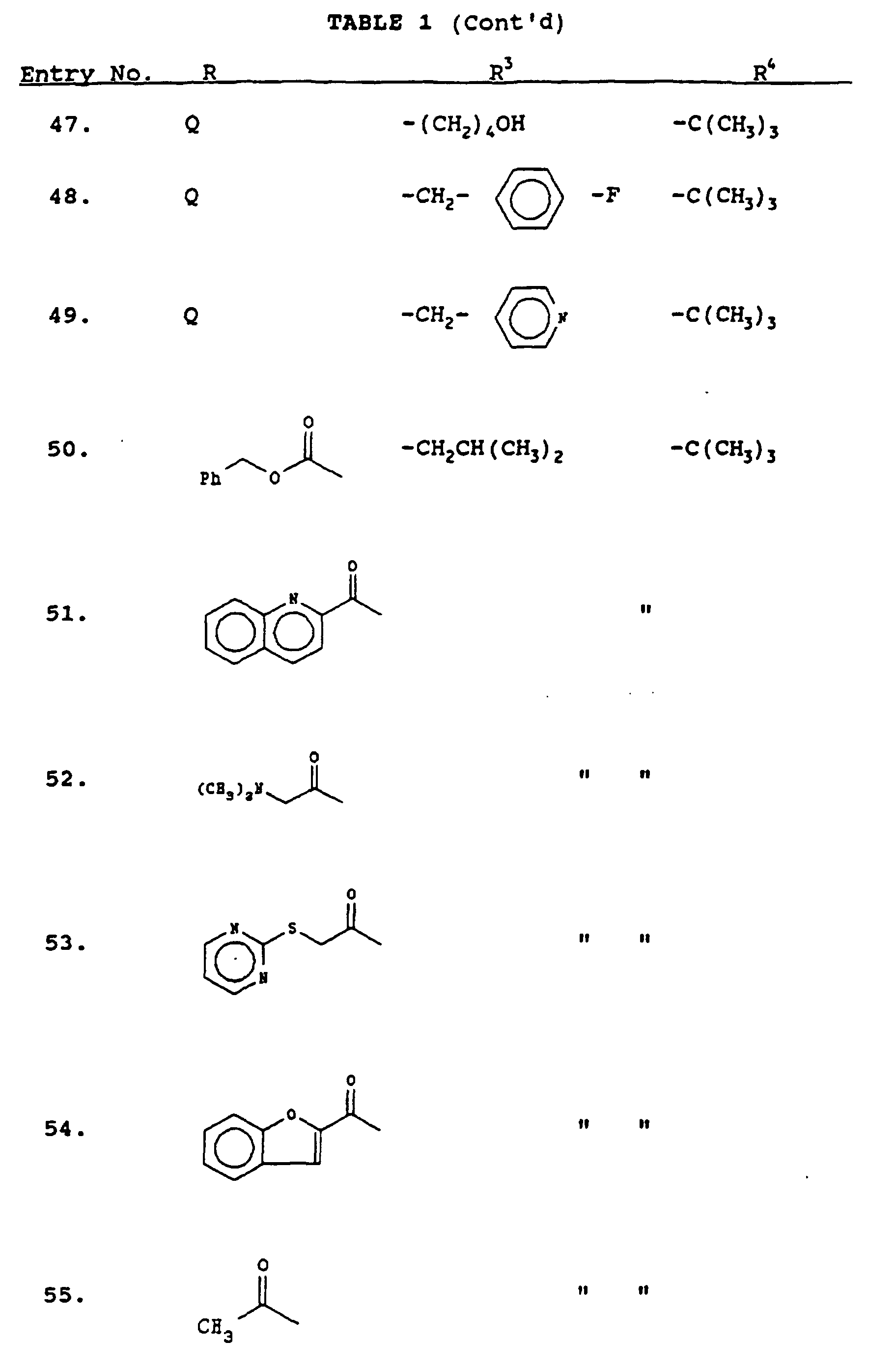

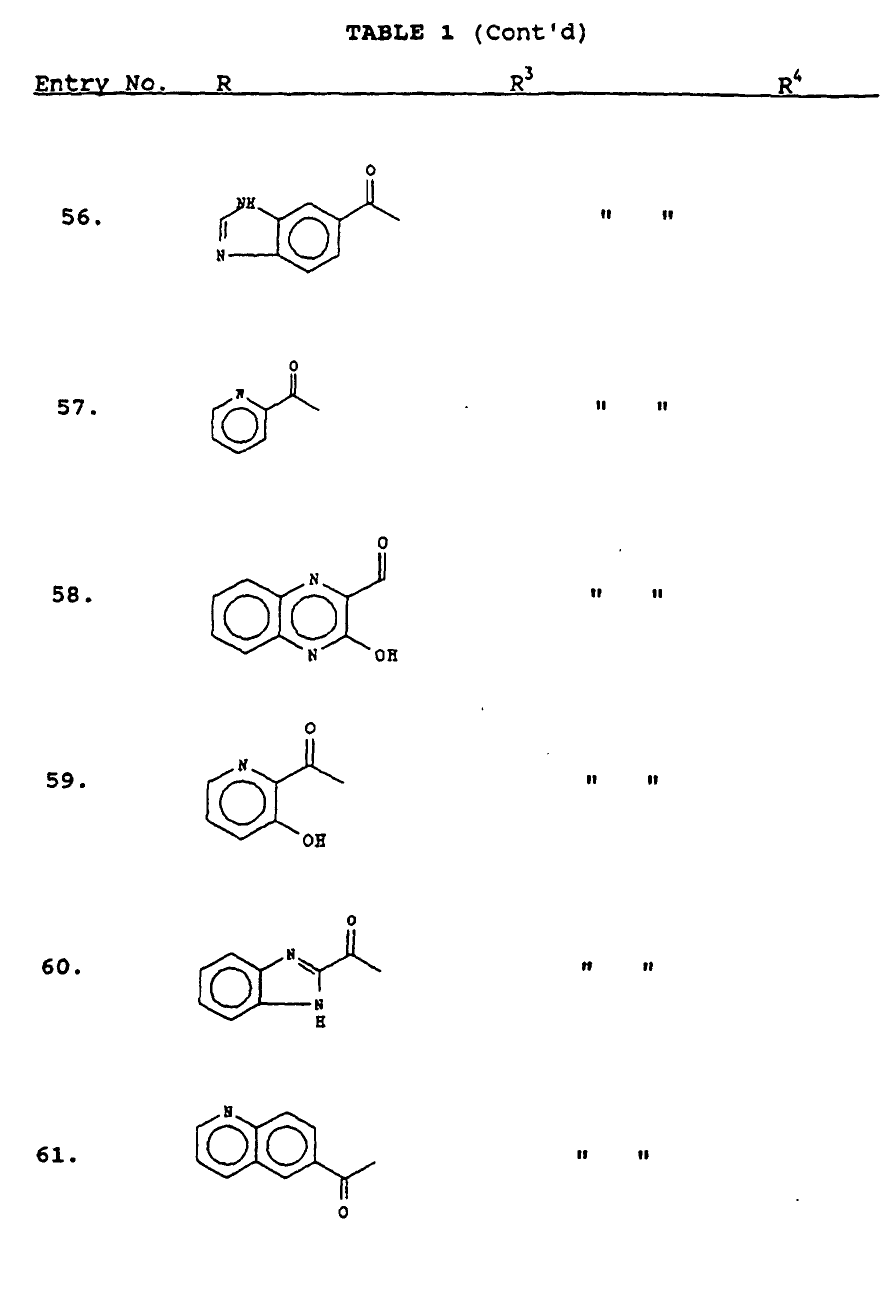

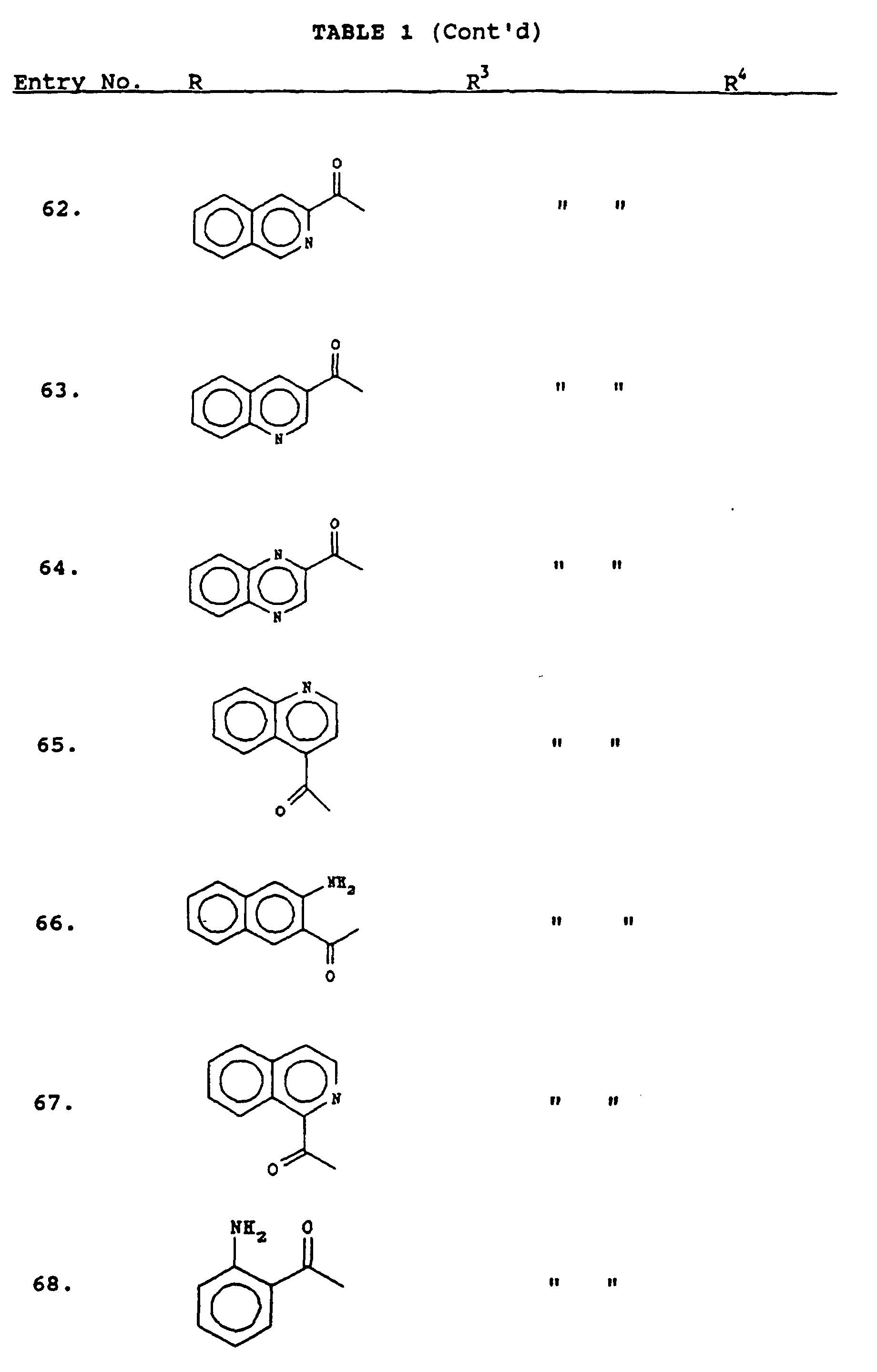

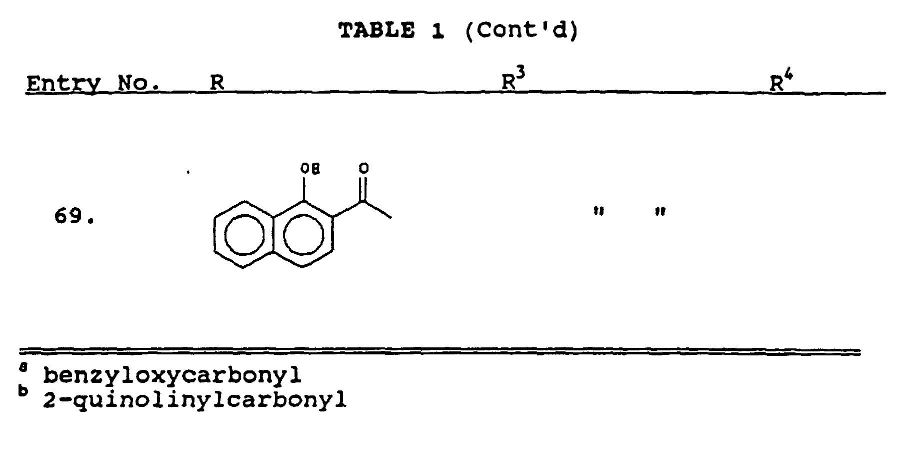

### EXAMPLE 10

Following the generalized procedures set forth in Example 9, the compounds set forth in Table 2 were prepared.

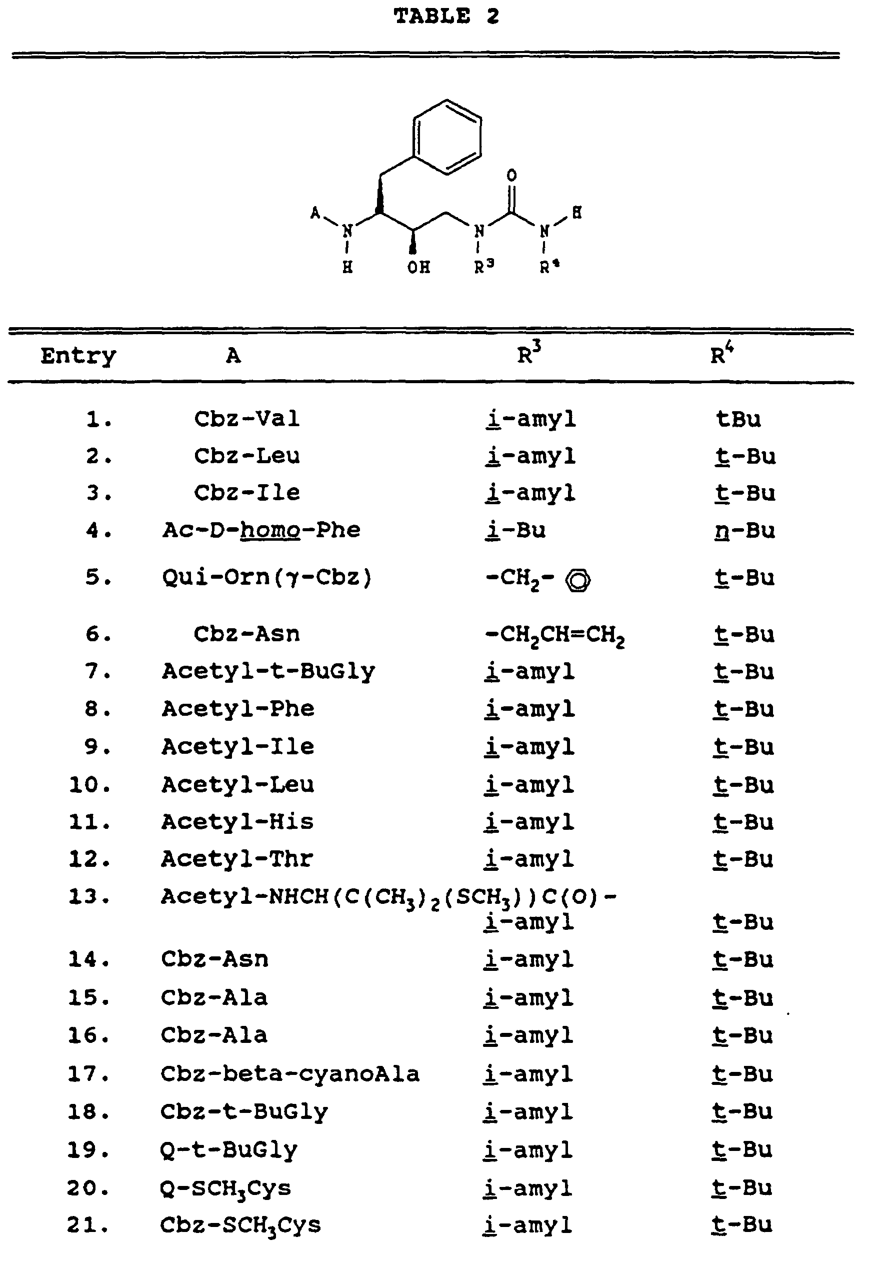

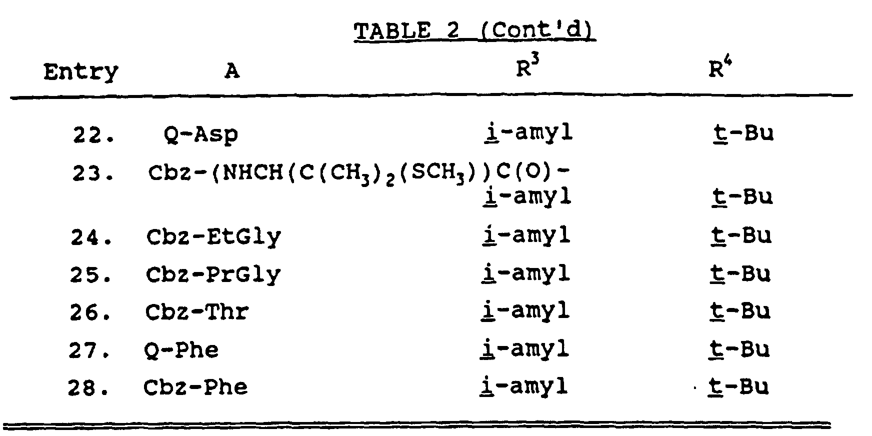

### EXAMPLE 11

Following the generalized procedure of Example 9, the compounds listed in Table 3 were prepared.

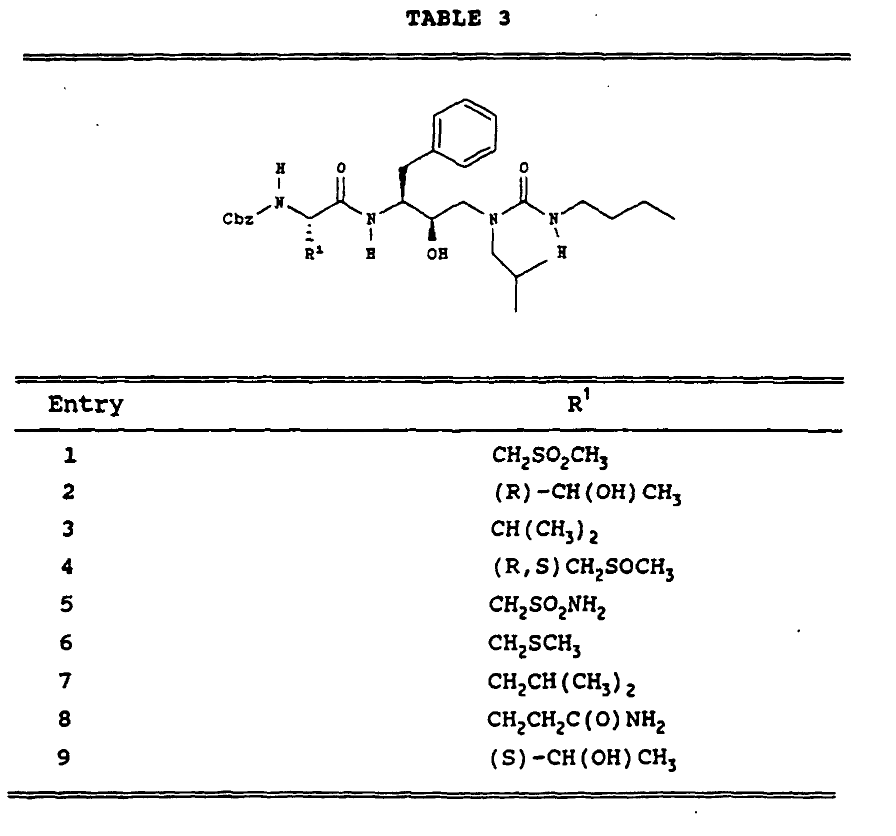

### EXAMPLE 12

Following the generalized procedures of Example 9, the compounds set forth in Table 4 were prepared.

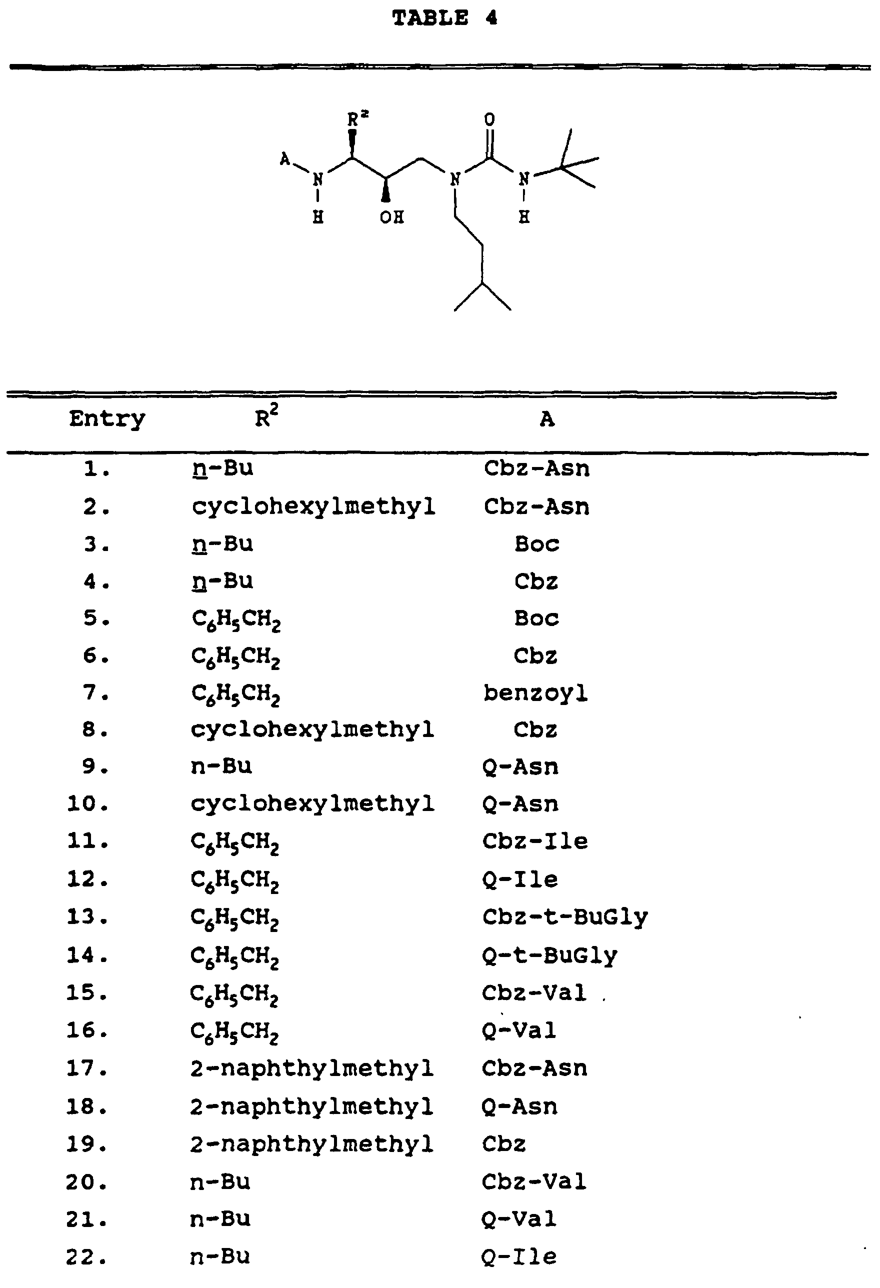

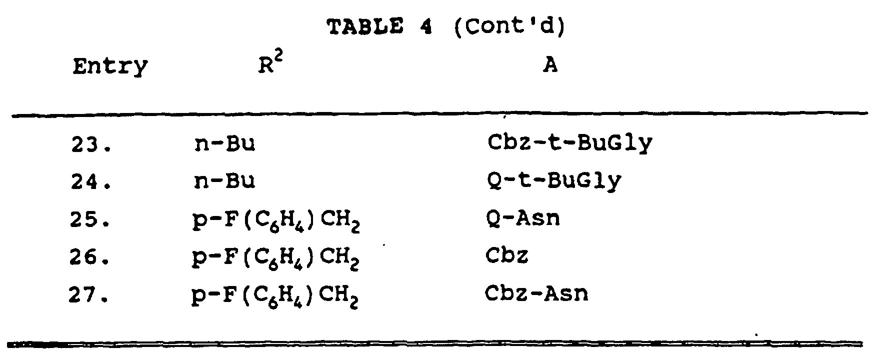

### EXAMPLE 13

The compounds listed in Table 5 were prepared according to the generalized procedures of Example 9.

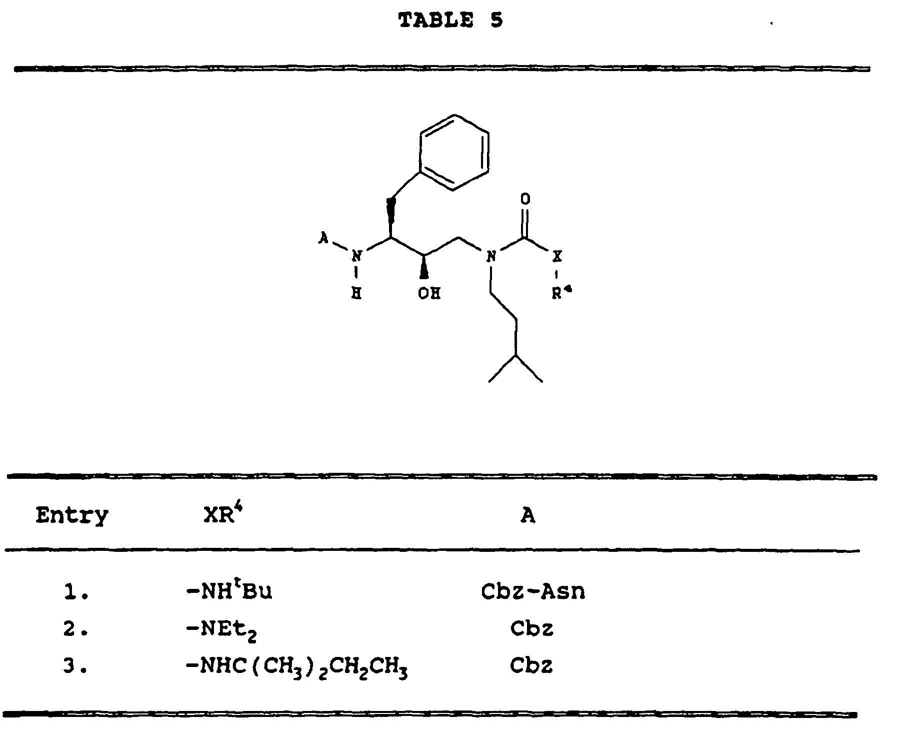

### EXAMPLE 14

The compounds of Table 6 were prepared according to the generalized procedures set forth in Example 9 except that instead of an isocyanate, an isothiocyanate equivalent was utilized.

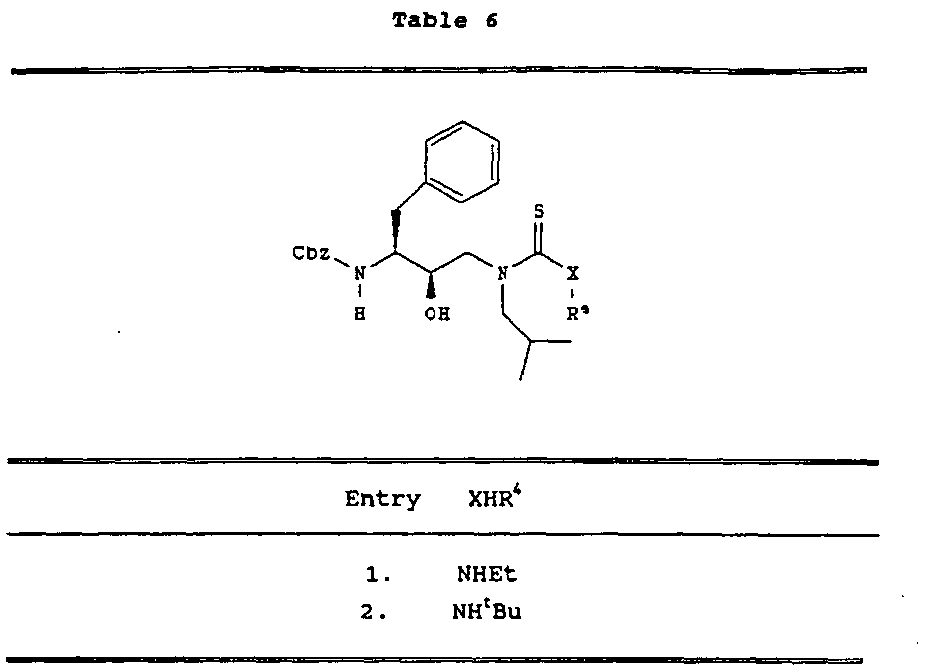

The Cbz group of the compounds shown in Examples 13 and 14 can be removed as described in Example 9 and the resulting compound can be coupled to a desired α- or β-amino acid or the like to produce compounds of the present invention.

### Example 15

The compounds shown in Table 7 were prepared according to the following general procedure. This general procedure represents a Curtius Rearrangement and reaction with the amino alcohol derivative as prepared following the general procedure in Example 9.

To a solution of 1 mmol of carboxylic acid in 12 mL of toluene and 3 mmol of triethylamine at 90°C under a nitrogen atmosphere, was added 1 mmol of diphenylphosphoryl azide. After 1 hour, a solution of 1 mmol of amino alcohol derivative in 3.5 mL of either N,N-dimethylformamide or toluene was added. After 1 hour, the solvent was removed under reduced pressure, ethyl acetate and water added and the layers separated. The organic layer was washed with 5% citric acid, sodium bicarbonate, brine, dried, filtered and concentrated to afford the crude product. This was then recrystallized or chromatographed on silica gel to afford the purified final compound.

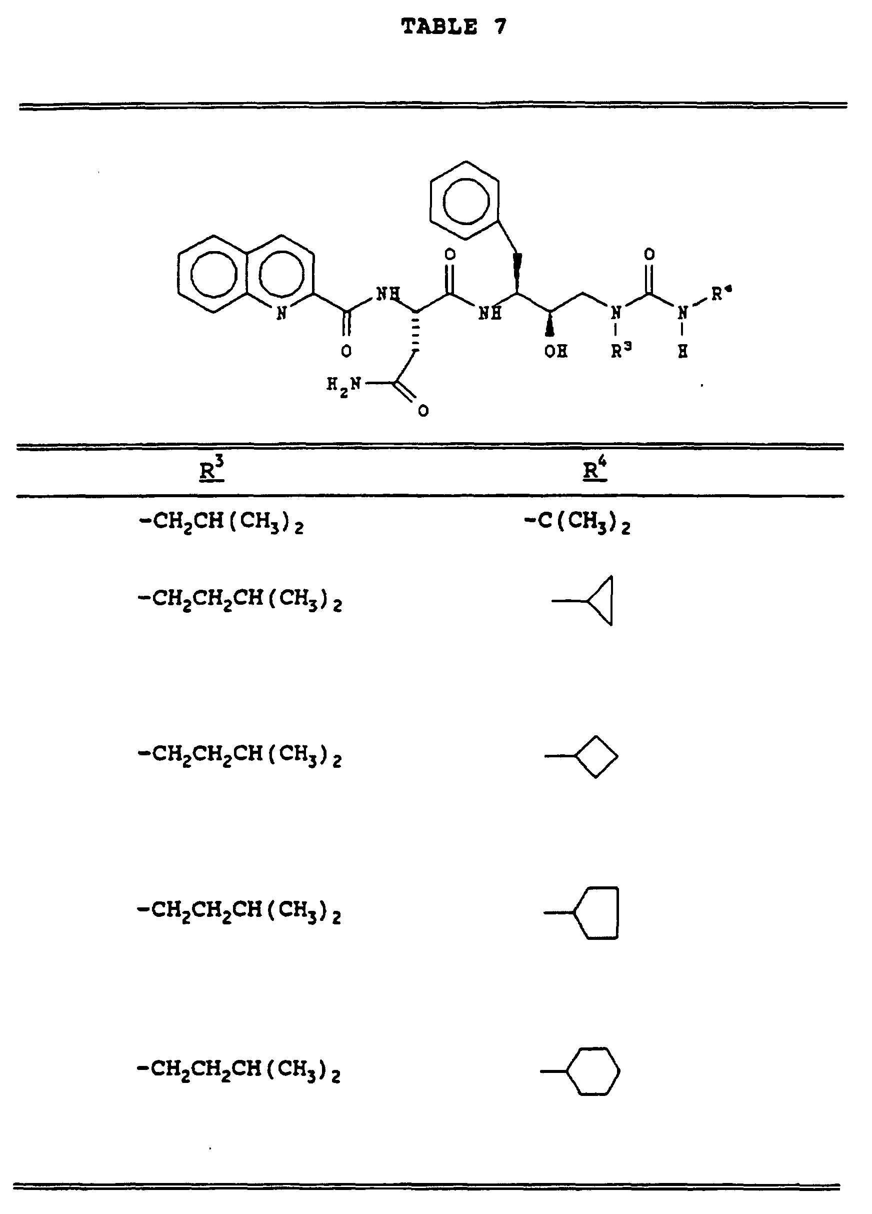

### Example 16

### A. Preparation of 4(4-methoxybenzyl)itaconate

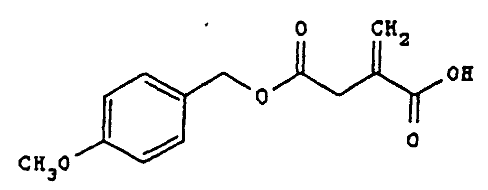

A 5 L three-necked round bottomed flask equipped with constant pressure addition funnel, reflux condenser, nitrogen inlet, and mechanical stirrer was charged with itaconic anhydride (660.8g, 5.88 mol) and toluene (2300 mL). The solution was warmed to reflux and treated with 4-methoxybenzyl alcohol (812.4g, 5.88 mol) dropwise over a 2.6h period. The solution was maintained at reflux for an additional 1.5h and then the contents were poured into three 2 L erlenmeyer flasks to crystallize. The solution was allowed to cool to room temperature whereupon the desired mono-ester crystallized. The product was isolated by filtration on a Buchner funnel and air dried to give 850.2g, 58% of material with mp 83-85°C, a second crop, 17% was isolated after cooling of the filtrate in an ice bath. ¹H NMR (CDCl₃) 300 MHz 7.32(d, J=8.7 Hz, 2H), 6.91(d, J=8.7 Hz, 2H), 6.49(s, 1H), 5.85(s, 1H), 5.12(s, 2H), 3.83(s, 3H), 3.40(s, 2H).

### B. Preparation of Methyl 4(4-methoxybenzyl) itaconate

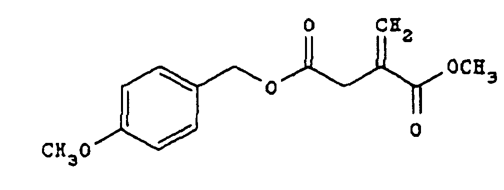

A 5 L three-necked round bottomed flask equipped with reflux condenser, nitrogen inlet, constant pressure addition funnel and mechanical stirrer was charged with 4(4-methoxybenzyl) itaconate (453.4g, 1.81 mol) and treated with 1,5-diazabicyclo[4.3.0]non-5-ene (275.6g, 1.81 mol), (DBU), dropwise so that the temperature did not rise above 15°C. To this stirring mixture was added a solution of methyl iodide (256.9g, 1.81 mol) in 250 mL of toluene from the dropping funnel over a 45m period. The solution was allowed to warm to room temperature and stirred for an additional 3.25h.

The precipitated DBU hydroiodide was removed by filtration, washed with toluene and the filtrate poured into a separatory funnel. The solution was washed with sat. aq. NaHCO₃ (2 X 500 mL), 0.2N HCl (1 X 500 mL), and brine (2 X 500 mL), dried over anhyd. MgSO₄, filtered, and the solvent removed *in vacuo*. This gave a clear colorless oil, 450.2g, 94% whose NMR was consistent with the assigned structure. ¹H NMR (CDCl₃) 300 MHz 7.30(d, J=8.7 Hz, 2H), 6.90(d, J=8.7 Hz, 2H), 6.34(s, 1H), 5.71(s, 1H), 5.09(s, 2H), 3.82(s, 3H), 3.73(s, 3H), 3.38(s, 2H). ¹³C NMR (CDCl₃) 170.46, 166.47, 159.51, 133.55, 129.97, 128.45, 127.72, 113.77, 66.36, 55.12, 51.94, 37.64.

### C. Preparation of Methyl 4(4-methoxybenzyl) 2(R)-methylsuccinate

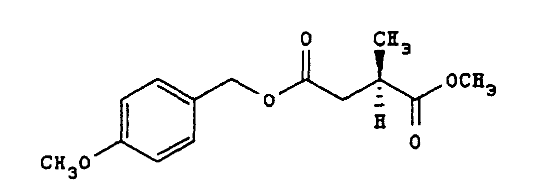

A 500 mL Fisher-Porter bottle was charged with methyl 4(4-methoxybenzyl) itaconate (71.1g, 0.269 mol), rhodium (R,R) DiPAMP catalyst (204mg, 0.269 mmol, 0.1 mol%) and degassed methanol (215 mL). The bottle was flushed 5 times with nitrogen and 5 times with hydrogen to a final pressure of 40 psig. The hydrogenation commenced immediately and after ca. 1h the uptake began to taper off, after 3h the hydrogen uptake ceased and the bottle was flushed with nitrogen, opened and the contents concentrated on a rotary evaporator to give a brown oil that was taken up in boiling *iso*-octane (ca. 200 mL, this was repeated twice), filtered through a pad of celite and the filtrate concentrated in vacuo to give 66.6g, 93% of a clear colorless oil, ¹H NMR (CDCl₃ 300 MHz 7.30(d, J=8.7 Hz, 2H), 6.91(d, J=8.7 Hz, 2H), 5.08(s, 2H), 3.82(s, 3H), 3.67(s, 3H), 2.95(ddq, J=5.7, 7.5, 8.7 Hz, 1H), 2.79(dd, J=8.1, 16.5 Hz, 1H), 2.45(dd, J=5.7, 16.5 Hz, 1H), 1.23(d, J=7.5 Hz, 3H).

### D. Preparation of Methyl 2(R)-methylsuccinate

A 3 L three-necked round-bottomed flask equipped with a nitrogen inlet, mechanical stirrer, reflux condenser and constant pressure addition funnel was charged with methyl 4(4-methoxybenzyl) 2(R)-methylsuccinate (432.6g, 1.65 mol) and toluene (1200 mL). The stirrer was started and the solution treated with trifluoroacetic acid (600 mL) from the dropping funnel over 0.25h. The solution turned a deep purple color and the internal temperature rose to 45°C. After stirring for 2.25h the temperature was 27°C and the solution had acquired a pink color. The solution was concentrated on a rotary evaporator. The residue was diluted with water (2200 mL) and sat. aq. NaHCO₃ (1000 mL). Additional NaHCO₃ was added until the acid had been neutralized. The aqueous phase was extracted with ethyl acetate (2 X 1000 mL) to remove the by-products and the aqueous layer was acidified to pH=1.8 with conc. HCl. This solution was extracted with ethyl acetate (4 X 1000 mL), washed with brine, dried over anhyd. MgSO₄, filtered and concentrated on a rotary evaporator to give a colorless liquid 251g, >100% that was vacuum distilled through a short path apparatus cut 1: bath temperature 120°C @ >1mm, bp 25-29°C; cut 2: bath temperature 140°C @ 0.5mm, bp 95-108°C, 151g, [α]_{D} @ 25°C=+1.38°C(c=15.475, MeOH), [α]_{D}=+8.48°C (neat); cut 3: bath temperature 140°C, bp 108°C, 36g, [α]_{D} @ 25°C=+1.49°C(c=15.00, MeOH), [α]_{D}=+8.98°C (neat). Cuts 2 and 3 were combined to give 189g, 78% of product, ¹H NMR (CDCl₃) 300 MHz 11.6(brs, 1H), 3.72(s, 3H), 2.92(ddq, J=5.7, 6.9, 8.0 Hz, 1H), 2.81(dd, J=8.0, 16.8 Hz, 1H), 2.47(dd, J=5.7, 16.8 Hz, 1H), 1.26(d, J=6.9 Hz, 3H).

### E. Preparation of Methyl Itaconate

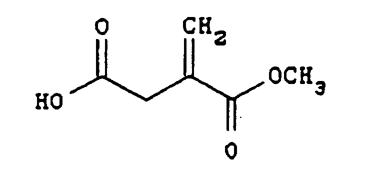

A 50 mL round bottomed flask equipped with reflux condenser, nitrogen inlet and magnetic stir bar was charged with methyl 4(4-methoxybenzyl) itaconate (4.00g, 16 mmol). The solution was kept at room temperature for 18 hours and then the volatiles were removed *in vacuo*. The residue was taken up in ethyl acetate and extracted three times with saturated aqueous sodium bicarbonate solution. The combined aqueous extract was acidified to pH=1 with aqueous potassium bisulfate and then extracted three times with ethyl acetate. The combined ethyl acetate solution was washed with saturated aqueous sodium chloride, dried over anhydrous magnesium sulfate, filtered, and concentrated *in vacuo*. The residue was then vacuum distilled to give 1.23g, 75% of pure product, bp 85-87 @ 0.1 mm. ¹H NMR (CDCl₃) 300 MHz 6.34(s, 1H), 5.73(s, 2H), 3.76(s, 3H), 3.38(s, 2H). ¹³C NMR (CDCl₃) 177.03, 166.65, 129.220, 132.99, 52.27, 37.46.

### F. Curtius Rearrangement of Methyl 2(R)-methylsuccinate: Preparation of Methyl N-Moz-α-methyl β-alanine.

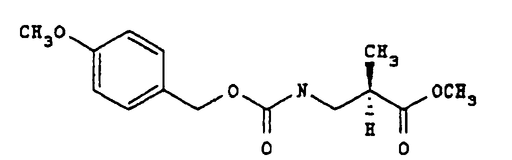

A 5L four necked round bottomed flask equipped with a nitrogen inlet, reflux condenser, mechanical stirrer, constant pressure addition funnel, and thermometer adapter was charged with methyl 2(R)-methylsuccinate (184.1g, 1.26 mol), triethylamine (165.6g, 218 mL, 1.64 mol, 1.3 equivalents), and toluene (1063 mL). The solution was warmed to 85°C and then treated dropwise with a solution of diphenylphosphoryl azide (346.8g, 1.26 mol) over a period of 1.2h. The solution was maintained at that temperature for an additional 1.0h and then the mixture was treated with 4-methoxybenzyl alcohol (174.1g, 1.26 mol) over a 0.33h period from the dropping funnel. The solution was stirred at 88°C for an additional 2.25h and then cooled to room temperature. The contents of the flask were poured into a separatory funnel and washed with sat. aq. NaHCO₃ (2 X 500 mL), 0.2N HCl (2 X 500 mL), brine (1 X 500 mL), dried over anhyd. MgSO₄, filtered, and concentrated *in vacuo* to give 302.3g, 85% of the desired product as a slightly brown oil. ₁H NMR (CDCl₃) 300 MHz 7.32(d, J=8.4 Hz, 2H), 6.91(d, J=8.4 Hz, 2H), 5.2(brm, 1H), 5.05(s, 2H), 3.83(s, 3H), 3.70(s, 3H), 3.35(m, 2H), 2.70(m, 2H), 1.20(d, J=7.2 Hz, 3H).

### G. Hydrolysis of Methyl N-Moz-α-methyl β-alanine: Preparation of α-methyl β-alanine Hydrochloride

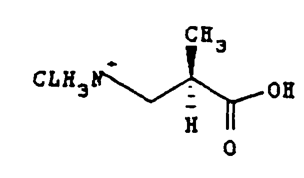

A 5 L three-necked round bottomed flask equipped with a reflux condenser, nitrogen inlet and mechanical stirrer was charged with methyl N-Moz-α-methyl β-alanine (218.6g, 0.78 mol), glacial acetic acid (975 mL) and 12N hydrochloric acid (1960 mL). The solution was then heated to reflux for 3h. After the solution had cooled to room temperature (ca. 1h) the aqueous phase was decanted from organic residue (polymer) and the aqueous phase concentrated on a rotary evaporator. Upon addition of acetone to the concentrated residue a slightly yellow solid formed that was slurried with acetone and the white solid was isolated by filtration on a Buchner funnel. The last traces of acetone were removed by evacuation to give 97.7g, 90% of pure product, mp 128.5-130.5°C [α]_{D} @ 25°C=9.0°C (c=2.535, Methanol). ¹H NMR (D₂O) 300 MHz 3.29(dd, J=8.6, 13.0 Hz, 1H), 3.16(dd, J=5.0, 13.0m Hz, 1H), 2.94(ddq, J=7.2, 5.0, 8.6 Hz, 1H), 1.30(d,J=7.2 Hz, 3H); ¹³C NMR (D₂O) 180.84, 44.56, 40.27, 17.49.

### H. Preparation of N-Boc α-Methyl β-Alanine

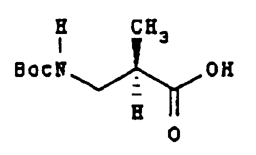

A solution of α-methyl β-alanine hydrochloride (97.7g, 0.70 mol) in water (1050 mL) and dioxane (1050 mL) the pH was adjusted to 8.9 with 2.9N NaOH solution. This stirring solution was then treated with di-*tert*-butyl pyrocarbonate (183.3g, 0.84 mol, 1.2 equivalents) all at once. The pH of the solution was maintained between 8.7 and 9.0 by the periodic addition of 2.5N NaOH solution. After 2.5h the pH had stabilized and the reaction was judged to be complete. The solution was concentrated on a rotary evaporator (the temperature was maintained at <40°C). The excess di-*tert*-butyl pyrocarbonate was removed by extraction with dichloromethane and then the aqueous solution was acidified with cold 1N HCl and immediately extracted with ethyl acetate (4 X 1000 mL). The combined ethyl acetate extract was washed with brine, dried over anhyd. MgSO₄, filtered and concentrated on a rotary evaporator to give a thick oil 127.3g, 90% crude yield that was stirred with n-hexane whereupon crystals of pure product formed, 95.65g, 67%, mp 76-78°C, [α]_{D} @ 25°C=-11.8°C (c=2.4, EtOH). A second crop was obtained by concentration of the filtrate and dilution with hexane, 15.4g, for a combined yield of 111.05g, 78%. ¹H NMR (acetone D₆) 300 MHz 11.7 (brs, 1H), 6.05 (brs 1H), 3.35 (m, 1H), 3.22 (m, 1H), 2.50 (m, 1H), 1.45(s, 9H), 1.19 (d, J=7.3 Hz, 3H); ¹³C NMR (acetone D₆) 177.01, 79.28, 44.44, 40.92, 29.08, 15.50. Elemental analysis calc'd. for C₉H₁₇NO₄: C, 53.19, H, 8.42; N, 6.89. Found: C, 53.36; H, 8.46; N, 6.99.

### I. Preparation of N-4-Methoxybenzyloxycarbonyl α-Methyl β-Alanine

A solution of N-4-methoxybenzyloxycarbonyl α-methyl β-alanine methyl ester (2.81g, 10.0 mmol) in 30 mL of 25% aqueous methanol was treated with lithium hydroxide (1.3 equivalents) at room temperature for a period of 2h. The solution was concentrated *in vacuo* and the residue taken up in a mixture of water and ether and the phases separated and the organic phase discarded. The aqueous phase was acidified with aqueous potassium hydrogen sulfate to pH=1.5 and then extracted three times with ether. The combined ethereal phase was washed with saturated aqueous sodium chloride solution, dried over anhydrous magnesium sulfate, filtered and concentrated *in vacuo* to give 2.60 g, 97% of N-4-Methoxybenzyloxycarbonyl α-methyl β-alanine (N-Moz-AMBA) which was purified by recrystallization from a mixture of ethyl acetate and hexane to give 2.44g, 91% of pure product, mp 96-97°C, MH+=268. ¹H NMR (D₆-acetone/300 MHz) 1.16 (3H, d, J=7.2Hz), 2.70 (1H, m), 3.31 (2H, m), 3.31 (3H, s), 4.99 (2H, s), 6.92 (2H, 4, J=8.7 Hz), 7.13 (2H, d, J=8.7 Hz).

### J. Preparation of Propanamide, 3-(4-methoxybenzyloxycarbonyl)-N [3-[[[(1,1-dimethylethyl)amine]carbonyl](3-methylbutyl)amino]-2-hydroxy-1-(phenylmethyl)propyl]-2-methyl-[IS-[IR*(S*), 2S*]]-

N-Moz-AMBA (468mg, 1.75mmol) was dissolved in 5mL of DMF, HOBT (355mg, 2.6mmol) was added and the solution was cooled to 0°C. The solution was treated with (336mg, 1.75mmol) EDC for 15 minutes. To this was added (612mg, 1.75mmol) of [2R,3S 3-amino-1-isoamyl-1-(t-butylcarbonyl)amino 4-phenyl-2-butanol in 10mL of DMF and the reaction stirred for 16 hours at room temperature. The DMF was concentrated to 5mL and the product was precipitated by addition to 60% saturated aqueous NaHCO₃. The solid was taken up in ethyl acetate and washed with KHSO₄, NaHCO₃, NaCl(saturated), dried over MgSO₄ and concentrated to yield 680mg of crude product which was crystallized from CH₂Cl₂, Et₂O, hexane, to yield 300mg of pure product.

### Example 17

The compounds of Table 8 were prepared according to the procedure listed below and that utilized in Example 16.

### Propaneamide, 3-[(1,1-dimethylethyl)butoxycarbonyl]amino-N-[3-[[[(1,1-dimethylethyl)amino]carbonyl](3-methylbutyl)amino]-2-hydroxy-1-(phenylmethyl)propyl]-2-methyl-,[1S-[1R*(S*),2S*]- (Table 8, Entry 11)

### Part A.

A solution of N-t-butyloxycarbonyl-2-(R)-methyl-3-aminopropionic acid (372 mg, 1.83 mmol) and N-hydroxybenzotriazole (371 mg, 2.75 mmol) in 5 mL of dimethylformamide was cooled to 0°C. To this was added EDC (351 mg, 1.83 mmol) and the solution was stirred for 15 minutes. To this chilled solution was added a solution of 3-[[[(1,1-dimethylethyl)amino]carbonyl](3-methylbutyl)amino]-2(R)-hydroxy-1(S)(phenylmethyl)propylamine in 5 mL of dimethylformamide and stirred for 15 hours. The dimethylformamide was removed and replaced with 50 mL of ethyl acetate, and the organic phase was extracted with 5% potassium hydrogen sulfate, saturated sodium bicarbonate and brine. The ethyl acetate layer was dried over magnesium sulfate, filtered and concentrated to yield 613 mg of product after recrystallization from ethyl acetate , hexanes. (63 % yield). M+Li 541

### Part B.

### Preparation of Propaneamide,_3-amino-N-[3-[[[(1,1-dimethylethyl)amino]carbonyl]-(3-methylbutyl)amino]-2-hydroxy-1-(phenylmethyl)propyl]-2-methyl-,[1S-[1R*(S*), 2S*]-hydrochloride

The product from part A. (577 mg, 1.08 mmol) was dissolved in 40 mL of 4N HCl in dioxane and the solution stirred for 2 hours, and concentrated to yield the hydrochloride salt in quantitative yield.

### Part C .

### Preparation of Propaneamide, 3-(2-methylpropanoylamino)-N-[3-[[[(1,1-dimethylethyl)amino]carbonyl](3-methylbutyl)amino]-2-hydroxy-1-(phenylmethyl)propyl]-2-methyl-,[1S-[1R*(S*),2S*]-

The product from part B. (236 mg, 0.5 mmol) was dissolved in anhydrous tetrahydrofuran and to this was added N-methylmorpholine (160 mg, 1.5 mmol) upon which time a precipitate formed. To this suspension was added isobutyryl chloride ( 53.5 mg, 0.5 mmol) and the suspension stirred for 15 hours. The suspension was diluted with ethyl acetate and washed with 5% potassium hydrogen sulfate, saturated sodium bicarbonate and brine. the organic layer was dried over magnesium sulfate, filtered and concentrated to yield 195 mg of crude product which was chromatographed on silica gel with 5% methanol methylene chloride to yield 121.5 mg (50 % yield) of pure product. M+Li 511

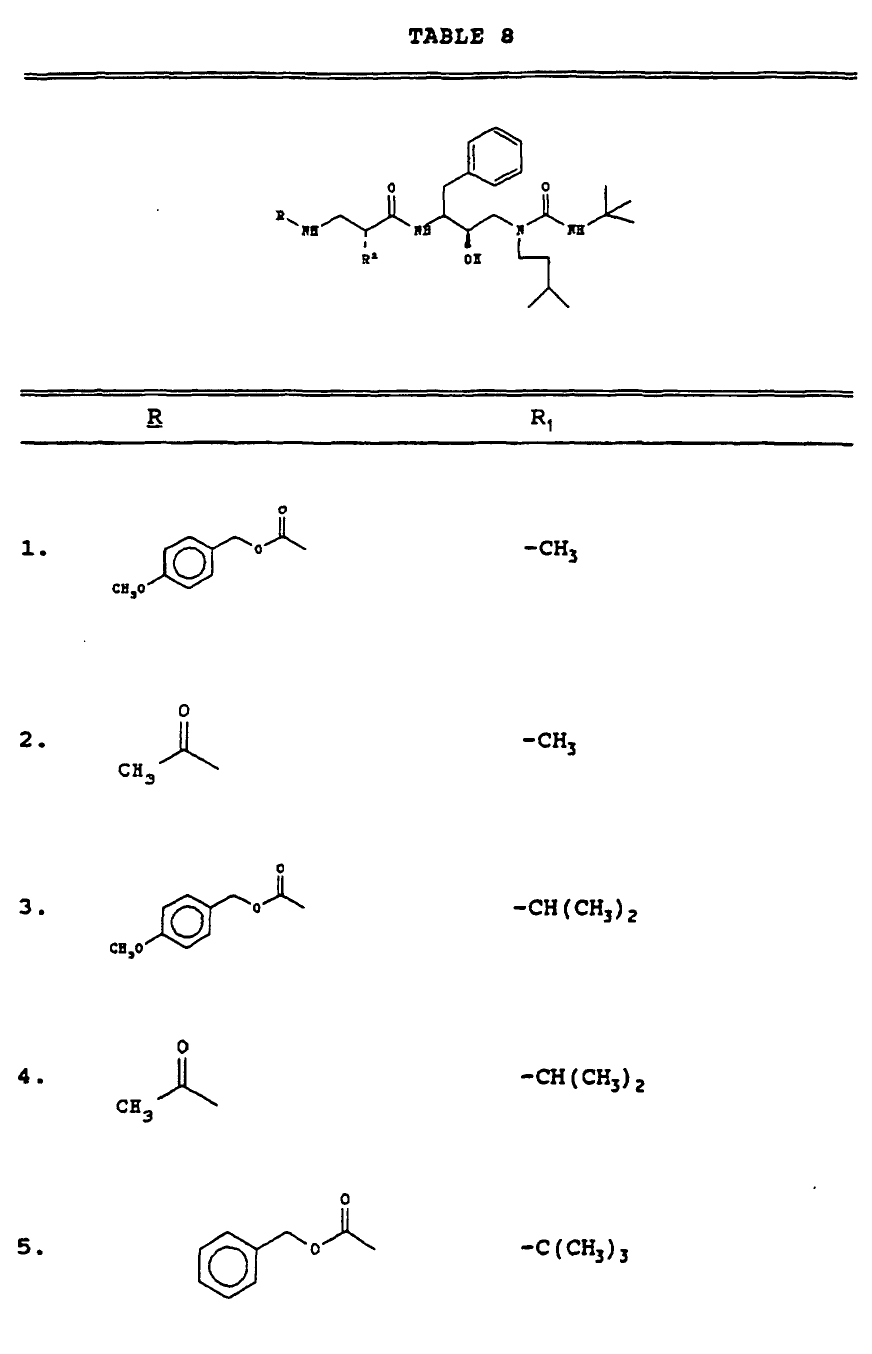

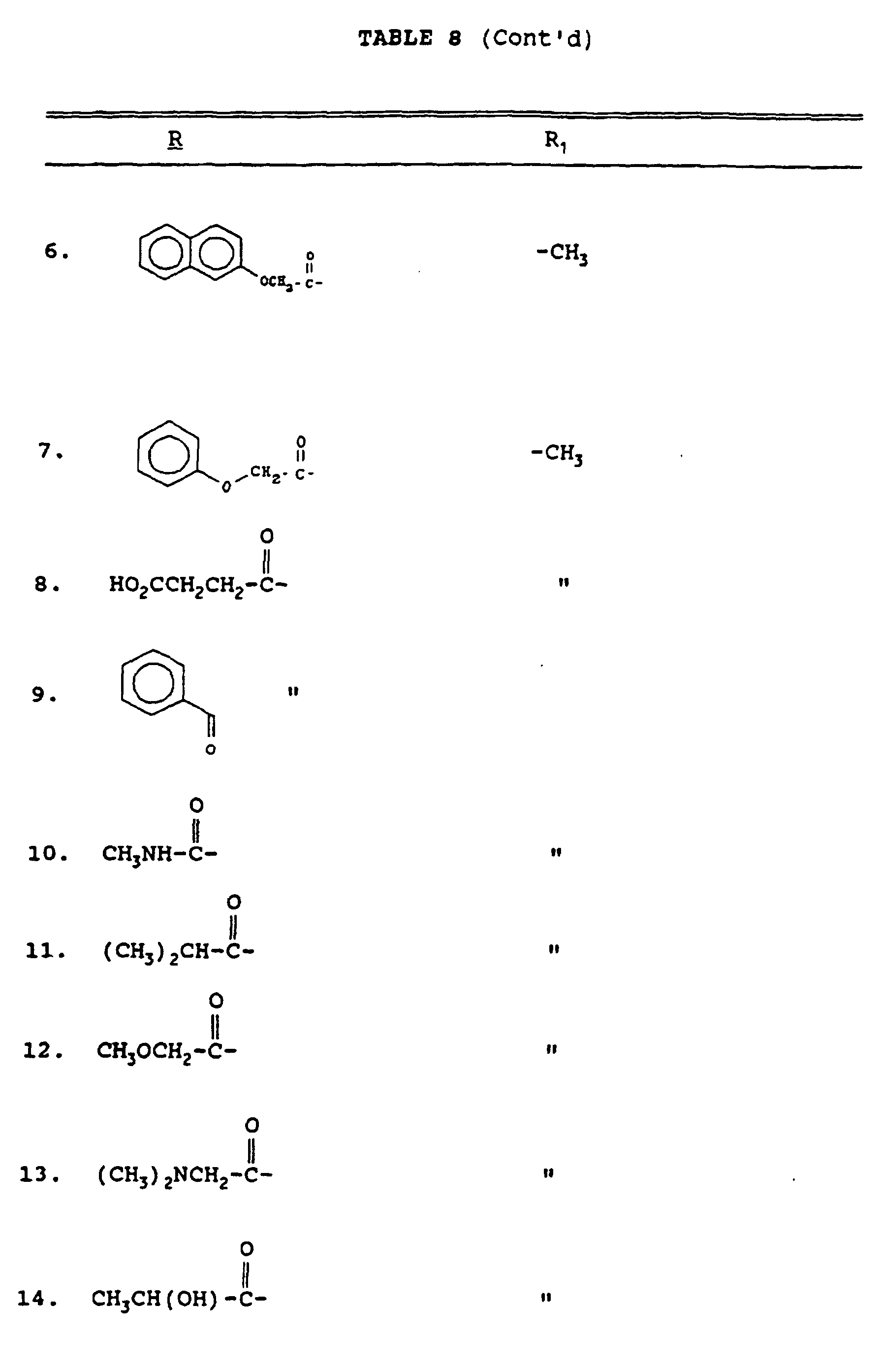

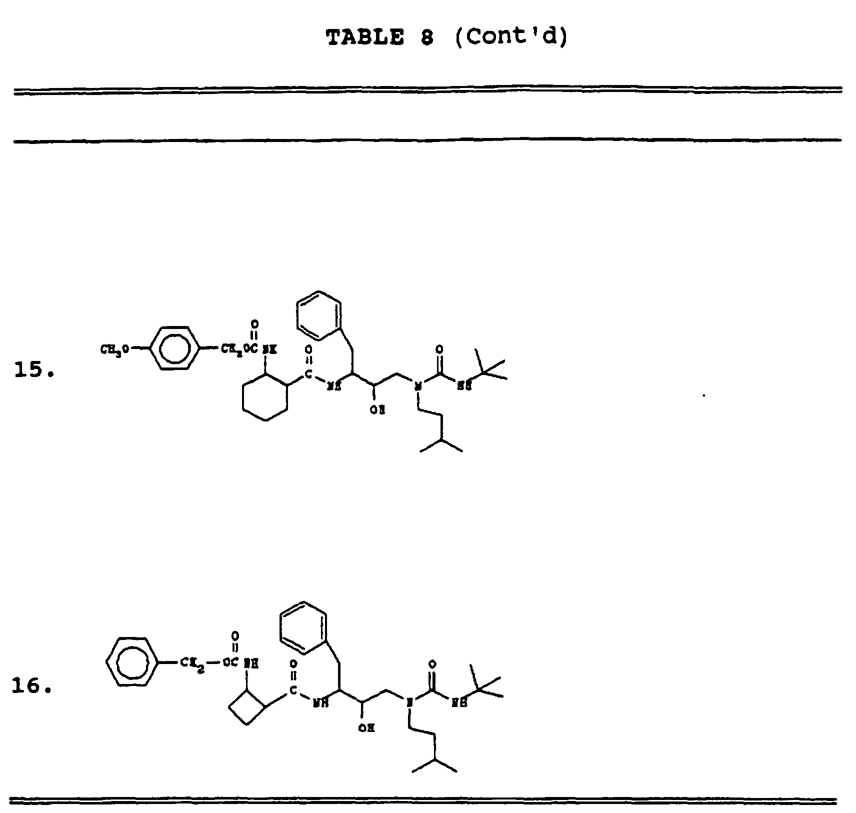

### Example 18

Following generally the procedure set forth in Example 16, the compounds shown in Table 9 were prepared.

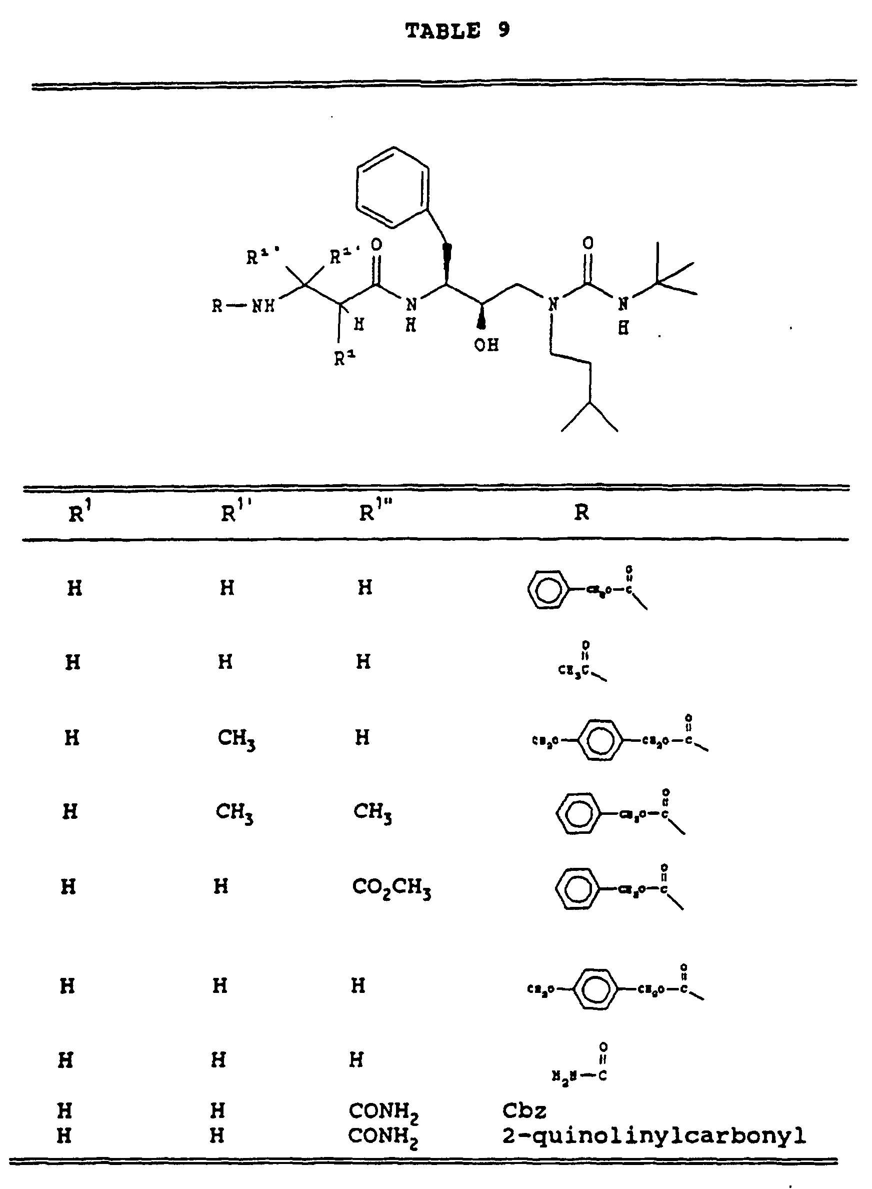

### Example 19

The procedure set forth below in Example 23was generally utilized to prepare the compounds shown in Table 10.

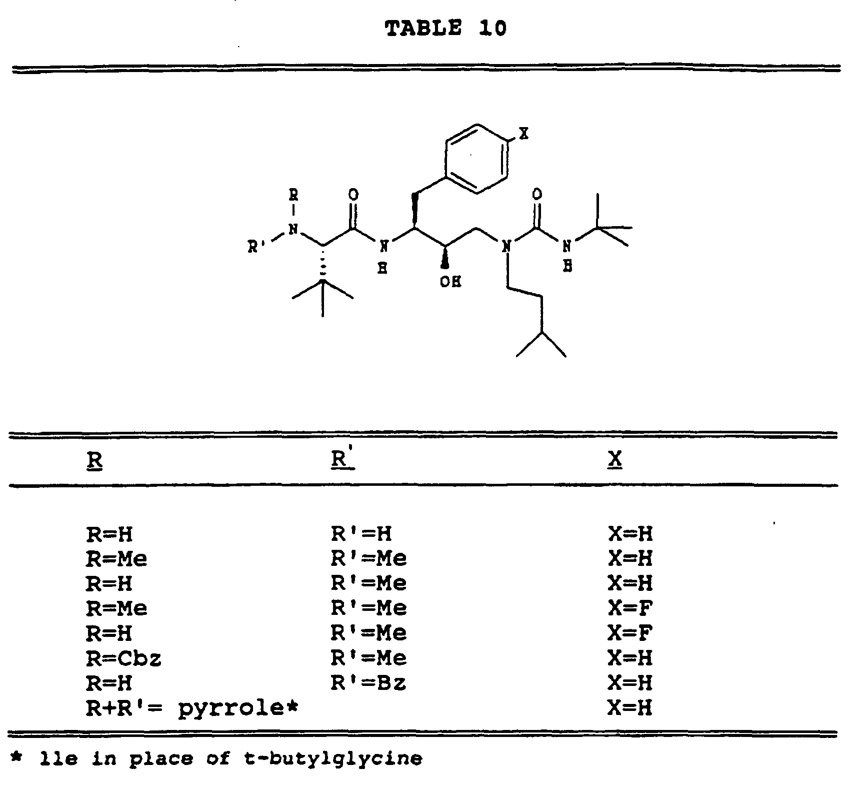

### Example 20

This example illustrates preparation of compounds wherein R⁴ and R⁵ together with N, forms a heterocycloalkyl radical.
**a) Pyrrolidine carbamoyl chloride.** 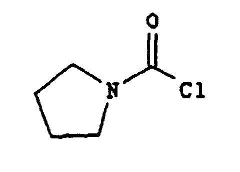 A stirring solution of triphosgene (27.78g, 0.103 mol) in 40 mL toluene was cooled to -20 °C in an ice/salt bath under a blanket of nitrogen and treated with a solution of N-methylmorpholine (27.3 g, 0.27 mol) in 20 mL of toluene dropwise over 1h. This solution was then treated with a solution of pyrrolidine (19.8 g, 0.27 mol) in 30 mL of toluene over a period of 30 m. The solution was allowed to warm to room temperature, filtered and the filtrate concentrated *in vacuo* to give an oil that was purified by vacuum distillation through a 12" Vigeraux column to give 20.7g, 56%, bp 58 °C @ 0.6 mm, of pure product.
**b) Butanediamide, N**^{**1**}**-[3-[[(4-fluorophenyl)methyl)](1-pyrrolidinylcarbonyl)amino]-2-hydroxy-1-(phenylmethyl)propyl]-2-[(2-quinolinylcarbonyl) amino]-[1S[1R*(R*),2S*]]-** 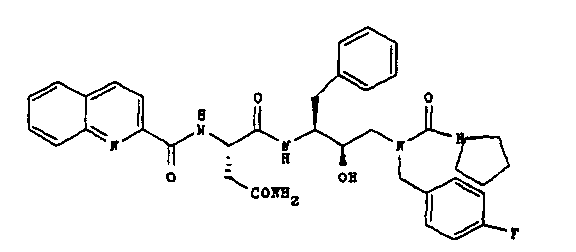 A stirring solution of [1S-[1R*(R*),2S*]]-N¹-[3-[[(4-fluorophenyl)methyl]amino]-2-hydroxy-1-(phenylmethyl)propyl]-2-[(2-quinolinylcarbonyl)aminobutanediamide (1.08 g, 1.91 mmol) (prepared as in Example 31) in 7 mL of anhydrous DMF was treated with pyrrolidine carbamoyl chloride (260 mg, 1.95 mmol), 4-dimethylaminopyridine (15 mg), and N-methylmorpholine (380 mg, 3.76 mmol). The solution was stirred at room temperature for 3h and then concentrated *in vacuo* to give a semi-solid that was dissolved in methanol/water ca. 2:1. A solid formed from this solid that was isolated by filtration on a Büchner funnel and washed with water, 5% aq. citric acid and water and air dried to give 130 mg of pure product, TLC on SiO₂ eluting with 7% methanol in ethyl acetate showed one spot with R_{f}=0.64, 11%.
**c) Butanediamide, N**^{**1**}**-[3-[[(4-fluorophenyl)methyl)](4-morpholinylcarbonyl)amino]-2-hydroxy-1-(phenylmethyl)propyl]-2-[(2-quinolinylcarbonyl) amino]-[1S(1R*(R*),2S*]]-** 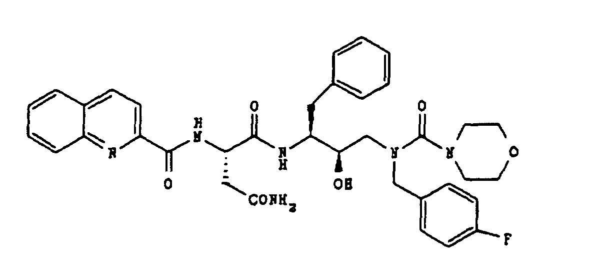 To a stirring solution of [1S-[1R*(R*),2S*]]-N¹-[3-[[(4-fluorophenyl)methyl]amino]-2-hydroxy-1-(phenylmethyl)propyl]-2-[(2-quinolinylcarbonyl)aminobutanediamide (520 mg, 0.922 mmol), triethylamine (172 mg, 1.70 mmol), 4-dimethylaminopyridine (50 mg), and morpholino carbamoyl chloride (157.3 mg, 1.05 mmol) in 5 mL of chloroform. The initially heterogeneous mixture was heated to reflux for 6 h. The solution was then diluted with additional chloroform, poured into a separatory funnel and washed with 1N KHSO₄, sat. aq. NaHCO₃, dried over anhyd. MgSO₄, filtered, and concentrated *in vacuo* to give a white solid that was purified by column chromatography on SiO₂ eluting with ethanol/ethyl acetate to give 380 mg, 61%, of pure product.

### Example 21

This example illustrates preparation of compounds wherein R⁴ and R⁵ are both other than H.

### Butanediamide, N¹-[3-[[(diethylamino)carbonyl](3-methylbutyl)amino]-2- hydroxy-1-(phenylmethyl)propyl]-2-[(2-quinolinylcarbonyl) amino]-[1S-[1R*(R*),2S*]]-

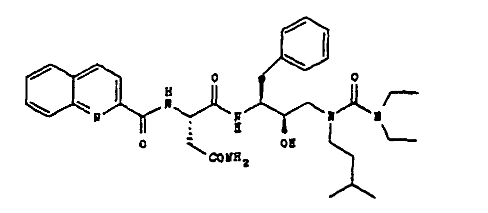

To a stirring solution of [1S-[1R*(R*),2S*]]-N¹-[3-(methylbutyl)amino]-2-hydroxy-1-(phenylmethyl)propyl]-2-[(2-quinolinylcarbonyl)amino-butane diamide] (119 mg, 0.21 mmol) triethylamine (59 mg, 0.58 mmol), 4-dimethylaminopyridine (9 mg), and diethyl carbamoyl chloride (157.3 mg, 1.05 mmol) in 4 mL of chloroform. The mixture was kept at room temperature for 26 h. The solution was then diluted with additional chloroform, poured into a separatory funnel and washed with 1N KHSO₄, sat. aq. NaHCO₃, dried over anhyd. MgSO₄, filtered, and concentrated *in vacuo* to give a white solid that was purified by column chromatography on SiO₂ eluting with methanol/CH₂Cl₂ to give 20 mg, 15%, of pure product.

### Example 22

Following the procedures set forth in Examples 20 and 21, the compounds listed in Table 11 were prepared.

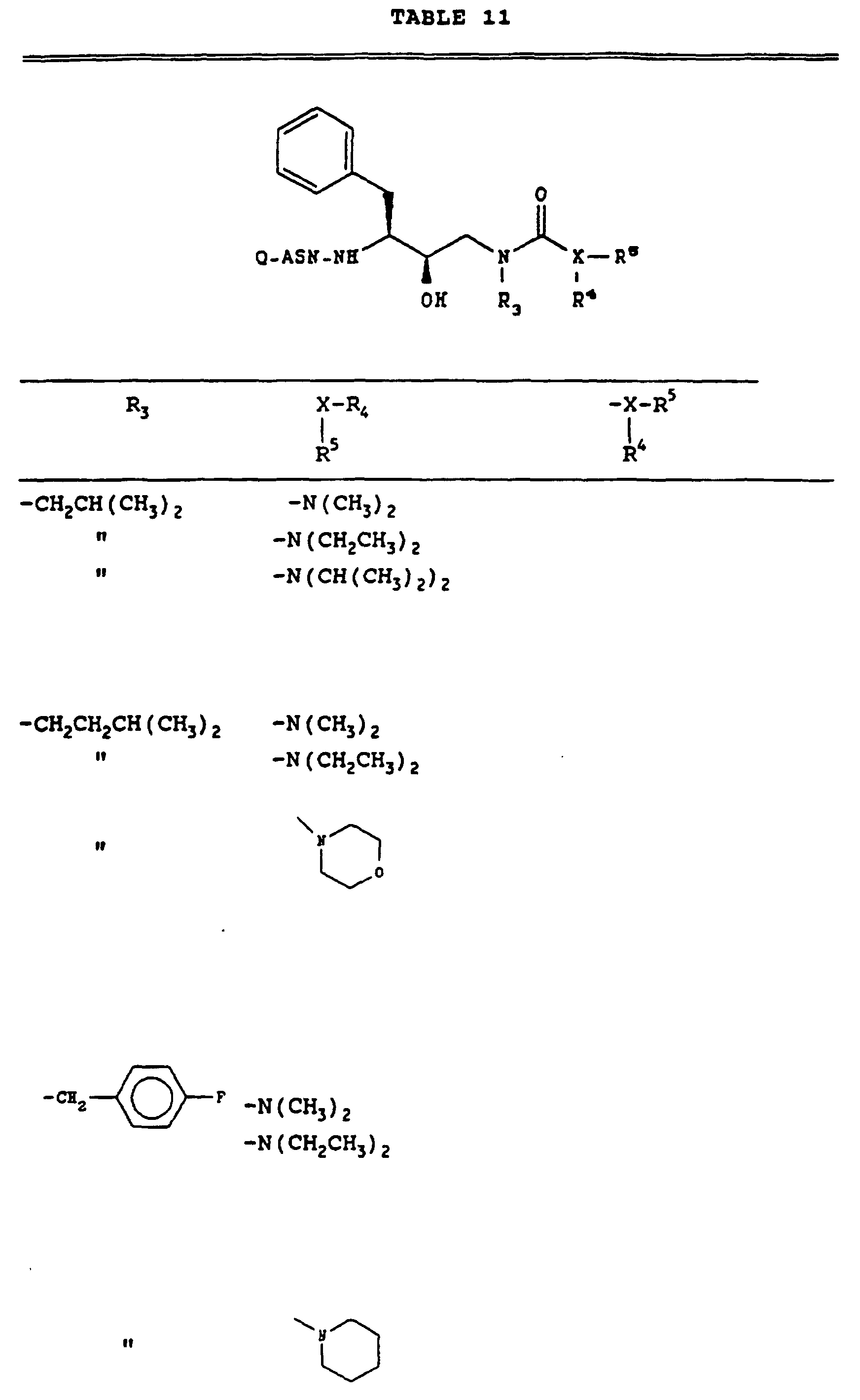

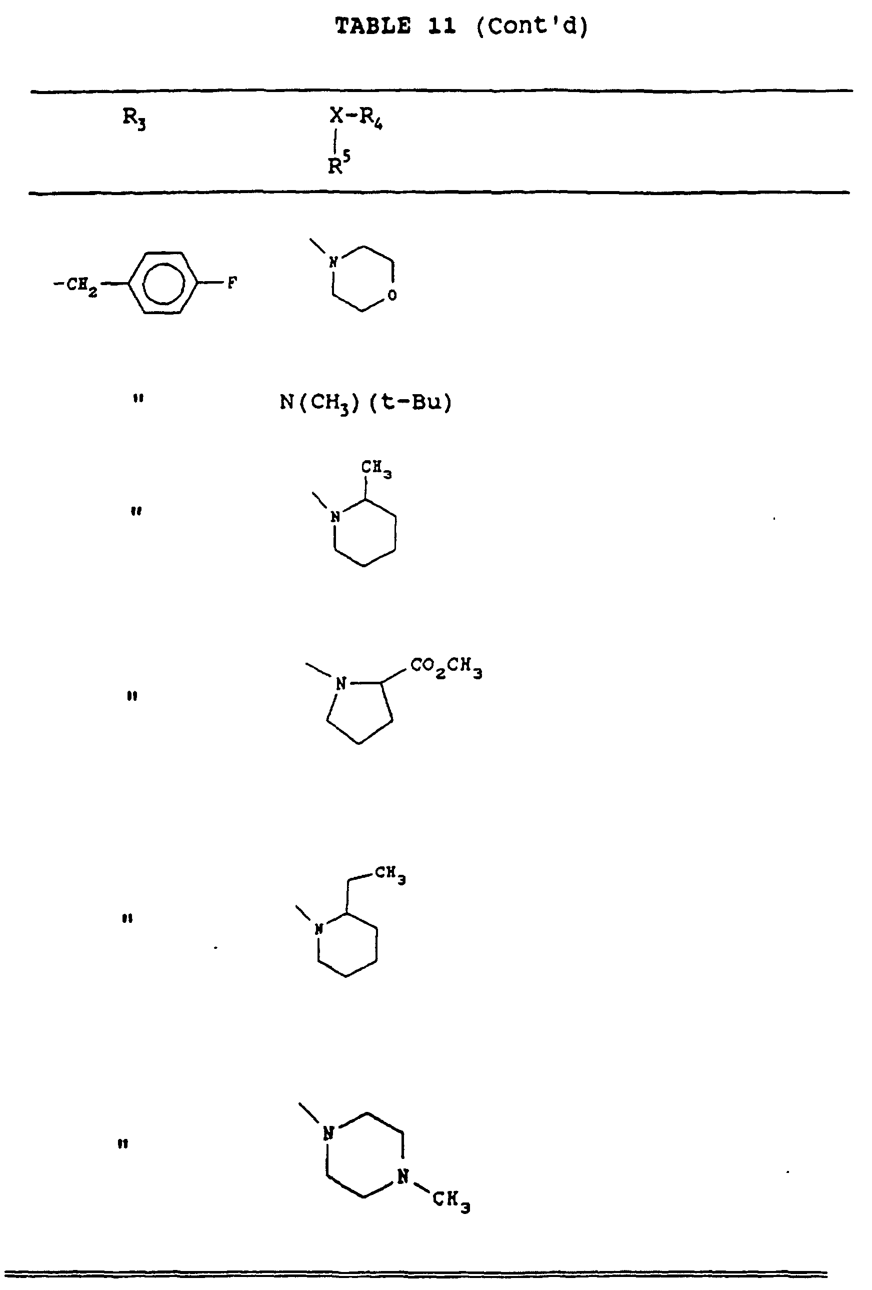

### Example 23

This example illustrates preparation of compounds of Formula II wherein R¹ is an alkyl group other than an alkyl group of a naturally occurring amino acid side chain. In particular R¹ is a t-butyl group.

### Part A:

3-[[(1,1-dimethylethyl)amino]carbonyl](3-methylbutyl)amino-2(R)-hydroxy-1(S)-[N-(benzyloxycarbonyl)(phenylmethyl()propyl amine] (4.7 gm, 9.7 mmol) was combined with 10% Pd on carbon (200 mg) and conc. HCl (3 mL) in ethanol (35 mL) and hydrogenated at 50 psi of hydrogen for 2.5 h. The reaction mixture was filtered through diatomaceous earth and concentrated on a rotary evaporator to a yellow hygroscopic solid; 3.7 gm, 100%.

### Part B:

### Butaneamide, 2-[(phenylmethyloxycarbonyl)amino]-N-[3-[[[(1,1-dimethylethyl)amino]carbonyl](3-methylbutyl)amino]-2-hydroxy-1-(phenylmethyl)propyl-3,3-dimethyl-[1S-[1R*(R*),25*]]-

N-Cbz-L-tert-leucine (172 mg, 0.65 mmol) and N-hydroxybenzotriazole (100 mg, 0.65 mmol) in DMF (3 mL) was cooled to 0°C and EDC (115 mg, 0.60 mmol) added. After 45 min the amine from Part A (193 mg, 0.50 mmol) and N-methylmorpholine (60 uL, 0.55 mmol) were added. The reaction was stirred at ambient temperature for 18 h and poured into a solution of 50% saturated NaHCO₃ (25 mL). The solid was collected by suction filtration, washed with water and dried in-vacuo. The solid was chromatographed on SiO₂ using 2% MeOH in CH₂Cl₂. The appropriate fractions were pooled and concentrated to afford a white solid; 220 mg, MH⁺ 597, TLC (SiO₂ 2%MeOH/CH₂Cl₂) R_{f} = .2 . CHN requires: C, 68.42, H, 8.78, N, 9.39; found: C, 68.03, H, 8.83, N, 9.33.

### Part C:

### Butaneamide, 2-amino-N-[3-[[[(1,1-dimethylethyl)amino]carbonyl](3-methylbutyl)amino]-2-hydroxy-1-(phenylmethyl)propyl]-3,3-dimethyl-, [1S-[1R*(R*), 2S*]-

The product from Part B (570 mg, 0.95 mmol) and 4% Pd on carbon (150 mg) in ethanol (30 mL) was hydrogenated at 5 psi for 2.75 h. The reaction mixture was filtered through diatomaceous earth and concentrated on a rotary evaporator to an oil; 438 mg, 100%.

### Part D:

### Butaneamide, 2-(acetylamino)-N-[3-[[[(1,1-dimethylethyl)amino]carbonyl](3-methylbutyl)amino]-2-hydroxy-1-(phenylmethyl)propyl]-3,3-dimethyl-, [1S-[1R*(R*), 2S*]-

The product from Part C (206 mg, 0.41 mmol) and N-methylmorpholine (45 uL, 0.41 mmol) were dissolved in CH₂Cl₂ (2.5 mL) and cooled to 0 C. Acetic anhydride (39 uL, 0.41 mmol) was then added and the reaction stirred 30 min at 0 C, then allowed to warm to ambient temperature and stir for 30 min. The solvent was removed on a rotary evaporator and the residue dissolved in ethanol (2 mL). The ethanolic solution was slowly poured into 50 % saturated NaHCO₃ (20 mL) and stirred vigorously. The solid was collected by suction filtration and washed with water, 5% citric acid, and again with water; 157 mg, 75%. CHN / 1.5 H₂O requires: C 63.24, H, 9.67, N, 10.54; found: C, 63.40, H, 9.41, N, 10.39.
Butaneamide, 2-amino-N-[3-[[[(1,1-dimethylethyl)amino]carbonyl](3-methylbutyl)amino]-2-hydroxy-1-(phenylmethyl)propyl]-3,3-dimethyl-, [1S-[1R*(R*), 2S*]- was also capped with the acyl groups shown in Table 12.

### EXAMPLE 24

The compounds of the present invention are effective HIV protease inhibitors. Utilizing an enzyme assay as described below, the compounds set forth in the examples herein disclosed inhibited the HIV enzyme. The preferred compounds of the present invention and their calculated IC₅₀ (inhibiting concentration 50%, i.e., the concentration at which the inhibitor compound reduces enzyme activity by 50%) values are shown in Table 11, together with compounds of similar structure on claimed in EP divisional patent applications EP 9 710 351.7 (publication number EP 815 856) and EP 97 105 352.5 (publication number EP 813 868). The enzyme method is described below. The substrate is 2-aminobenzoyl-Ile-Nle-Phe(p-NO₂)-Gln-ArgNH₂. The positive control is MVT-101 (Miller, M. et al, Science, 246, 1149 (1989)] The assay conditions are as follows:

| | |
|---|---|
| Assay buffer | 20 mM sodium phosphate, pH 6.4 |
| | 20% glycerol |
| | 1 mM EDTA |
| | 1 mM DTT |
| | 0.1% CHAPS |

The above described substrate is dissolved in DMSO, then diluted 10 fold in assay buffer. Final substrate concentration in the assay is 80 µM.

HIV protease is diluted in the assay buffer to a final enzyme concentration of 12.3 nanomolar, based on a molecular weight of 10,780.

The final concentration of DMSO is 14% and the final concentration of glycerol is 18%. The test compound is dissolved in DMSO and diluted in DMSO to 10x the test concentration; 10µl of the enzyme preparation is added, the materials mixed and then the mixture is incubated at ambient temperature for 15 minutes. The enzyme reaction is initiated by the addition of 40µl of substrate. The increase in fluorescence is monitored at 4 time points (0, 8, 16 and 24 minutes) at ambient temperature. Each assay is carried out in duplicate wells.

**TABLE 13**

| | Compound | IC₅₀ (nanomolar) |
|---|---|---|
| 1a) | Butanediamide, N¹-[3-[[[(1,1-dimethylethyl)amino]carbonyl](2-methylpropyl)amino]-2-hydroxy-1-(phenylmethyl)propyl]-2-[(N-benzyloxycarbonyl)amino]-, [1S-[1R*(R*), 2S*]]- | 35 |
| 1b) | Butanediamide, N¹-[3-[[[(1,1-dimethylethyl)amino]carbonyl](2-methylpropyl)amino]-2-hydroxy-1-(phenylmethyl)propyl]-2-[(2-quinolinylcarbonyl)amino]-, [1S-[1R*(R*),2S*]]- | 7.1 |
| 2a) | Butanediamide, N¹-[3-[[[(1,1-dimethylethyl)amino]carbonyl](3-methylbutyl)amino]-2-hydroxy-1-(phenylmethyl)propyl]-2-[(N-benzyloxycarbonyl)amino], [1S-[1R*(R*),2S*]]- | 13.4 |
| 2b) | Butanediamide, N¹-[3-[[[(1,1-dimethylethyl)amino]carbonyl](3-methylbutyl)amino]-2-hydroxy-1-(phenylmethyl)propyl]-2-[(2-quinolinylcarbonyl)amino]-, [1S-[1R*(R*),2S*]]- | 3.2 |
| 3a) | Butanediamide, N¹-[3-[[[(1,1-dimethylethyl)amino]carbonyl](2-naphthylmethyl)amino]-2-hydroxy-1-(phenylmethyl)propyl]-2-[(N-benzyloxycarbonyl)amino]-, [1S-[1R*(R*), 2S*]]- | 31.0 |
| 3b) | Butanediamide, N¹-[3-[[[(1,1-dimethylethyl)amino]carbonyl](2-naphthylmethyl)amino]-2-hydroxy-1-(phenylmethyl)propyl]-2-[(2-quinolinylcarbonyl)amino]-, [1S-[1R*(R*),2S*]]- | 2.9 |
| 4a) | Butanediamide, N¹-[3-[[[(1,1-dimethylethyl)amino]carbonyl](2-phenylethyl)amino]-2-hydroxy-1-(phenylmethyl)propyl]-2-[(N-benzyloxycarbonyl)amino]-, [1S-[1R*(R*),2S*]]- | 70 |
| 4b) | Butanediamide, N¹-[3-[[[(1,1-dimethylethyl)amino]carbonyl](2-phenylethyl)amino]-2-hydroxy-1-(phenylmethyl)propyl]-2-[(2-quinolinylcarbonyl)amino]-, [1S-[1R*(R*),2S*]]- | 10 |
| 5a) | Butanediamide, N¹-[3-[[[(1,1-dimethylethyl)amino]carbonyl](3,3-dimethylpropyl)amino]-2-hydroxy-1-(phenylmethyl)propyl]-2-[(N-benzyloxycarbonyl)amio], [1S-[1R*(R*),2S*]]- | 18 |
| 5b) | Butanediamide, N¹-[3-[[[(1,1-dimethylethyl)amino]carbonyl](3,3-dimethylpropyl)amino]-2-hydroxy-1-(phenylmethyl)propyl]-2-[(2-quinolinylcarbonyl)amino]-, [1S-[1R*(R*),2S*]]- | 3.7 |
| 6) | Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)-, [1S-[1R*(R*), 2S*]]- | 29 |
| 7) | Propanamide, 3-(acetylamino)-N-[3-[[[(11,-dimethylethyl)amino]carbonyl](3-methylbutyl)amino]-2-hydroxy-1-(phenylmethyl)propyl]-2-methyl-, [1S-[1R*(S*),2S*]]- | 100 |
| 8) | Propanamide, 3-(4-methoxybenzyloxy-carbonyl)amino-N-[3-[[[(11,-dimethylethyl)amino]carbonyl](3-methylbutyl)amino]-2-hydroxy-1-(phenylmethyl) propyl]-2-methyl-, [1S-[1R*(S*),2S*]]- | 6 |
| 9) | Butanediamide, N¹-[2-hydroxy-3-[(4-fluorophenylmethyl)(1-pyrrolidinylcarbonyl)amino]-1-(phenylmethyl)propyl]-2-[(2-quinolinylcarbonyl) amino]-, [1S-[1R*(R*),2S*]]- | 7 |
| 10) | Butanediamide, N¹-[3-[[[(1,1-dimethylethyl)amino]carbonyl](3-methylbutyl)amino]-2-hydroxy-1-(phenylmethyl)propyl]-2,3,3-trimethyl-, [1S-[1R*(2S*),2S*]]- | 12 |
| 11) | Butanoic acid, 4-[[3-[[[(1,1-dimethylethyl)amino]carbonyl] (3-methylbutyl)amino]-2-hydroxy-1-(phenylmethyl)propyl]amino-2,2, 3-trimethyl-4-oxo-, [1S-[1R*(3S*), 2S*]]- | 8 |
| 12) | Butaneamide,2-(acetylamino)-N-[3-[[[(1,1-dimethylethyl)amino]-carbonyl](3-methylbutyl)amino]-2-hydroxy-1-(phenylmethyl)propyl]-3,3-dimethyl-, [1S-[1R*(R*),2S*]]- | 18 |

### Example 47

The effectiveness of the compounds listed in Table 9 were determined in the above-described enzyme assay and in a CEM cell assay.

The HIV inhibition assay method of acutely infected cells is an automated tetrazolium based colorimetric assay essentially that reported by Pauwles et al, J. Virol. Methods 20, 309-321 (1988). Assays were performed in 96-well tissue culture plates. CEM cells, a CD4⁺ cell line, were grown in RPMI-1640 medium (Gibco) supplemented with a 10% fetal calf serum and were then treated with polybrene (2µg/ml). An 80 µl volume of medium containing 1 x 10⁴ cells was dispensed into each well of the tissue culture plate. To each well was added a 100µl volume of test compound dissolved in tissue culture medium (or medium without test compound as a control) to achieve the desired final concentration and the cells were incubated at 37°C for 1 hour. A frozen culture of HIV-1 was diluted in culture medium to a concentration of 5 x 10⁴ TCID₅₀ per ml (TCID₅₀ = the dose of virus that infects 50% of cells in tissue culture), and a 20µL volume of the virus sample (containing 1000 TCID₅₀ of virus) was added to wells containing test compound and to wells containing only medium (infected control cells). Several wells received culture medium without virus (uninfected control cells). Likewise, the intrinsic toxicity of the test compound was determined by adding medium without virus to several wells containing test compound. In summary, the tissue culture plates contained the following experiments:

| | Cells | Drug | Virus |
|---|---|---|---|
| 1. | + | - | - |
| 2. | + | + | - |
| 3. | + | - | + |
| 4. | + | + | + |

In experiments 2 and 4 the final concentrations of test compounds were 1, 10, 100 and 500 µg/ml. Either azidothymidine (AZT) or dideoxyinosine (ddI) was included as a positive drug control. Test compounds were dissolved in DMSO and diluted into tissue culture medium so that the final DMSO concentration did not exceed 1.5% in any case. DMSO was added to all control wells at an appropriate concentration.

Following the addition of virus, cells were incubated at 37°C in a humidified, 5% CO₂ atmosphere for 7 days. Test compounds could be added on days 0, 2 and 5 if desired. On day 7, post-infection, the cells in each well were resuspended and a 100µl sample of each cell suspension was removed for assay. A 20µL volume of a 5 mg/ml solution of 3-(4,5-dimethylthiazol-2-yl)-2,5-diphenyltetrazolium bromide (MTT) was added to each 100µL cell suspension, and the cells were incubated for 4 hours at 27°C in a 5% CO₂ environment. During this incubation, MTT is metabolically reduced by living cells resulting in the production in the cell of a colored formazan product. To each sample was added 100µl of 10% sodium dodecylsulfate in 0.01 N HCl to lyse the cells, and samples were incubated overnight. The absorbance at 590 nm was determined for each sample using a Molecular Devices microplate reader. Absorbance values for each set of wells is compared to assess viral control infection, uninfected control cell response as well as test compound by cytotoxicity and antiviral efficacy.

**TABLE 14**

| Compound | IC₅₀ | EC₅₀ | TD₅₀ |
|---|---|---|---|
| butanediamide N¹[3-[[[n-butylamino]carbonyl](2-methylpropyl)amino]-2-hydroxy-1-(phenylmethyl)propyl]-2-[(phenylmethylcarbamoyl) amino],[1S-[1R*(R*), 2S*]]--. | 940nM | 17µM | 170-180µM |
| butanediamide N¹[3-[[[n-butylamino]carbonyl](2-methylpropyl)amino]-2-hydroxy-1-(phenylmethyl)propyl]-2-[(2-quinolinylcarbonyl)amino]-[1S-[1R*(R*), 2S*]]-. | 126nM | 26nM | 53µM |
| butanediamide N¹[3-[[[(1,1-dimethylethyl)amino]-carbonyl](2-phenylethyl)amino]-2-hydroxy-1-(phenylmethyl)propyl]-2-[(2-quinolinylcarbonyl)amino] [1S-[1R*(R*), 2S*]]-. | 10nM | 28nM | 13µM |
| butanediamide N¹[3-[[[(1,1-dimethylethyl)amino]-carbonyl](2-methylpropyl)amino-2-hydroxy-1-(phenylmethyl)propyl]-2-[(2-quinolinylcarbonyl)amino] [1S-[1R*(R*), 2S*]]-. | 7nM | 10nM | 16µM |

Utilizing the procedures set forth above in the examples along with the general description, it is contemplated that the compounds listed below could be prepared and that such compounds would have activities as HIV protease inhibitors substantially similar to the activities of the compounds set forth in the examples.
Butanediamide, N¹-[3-[[[(1,1-dimethylethyl)amino]carbonyl]pentylamino]-2-hydroxy-1-(phenylmethyl)propyl]-2-[(2-quinolinylcarbonyl)amino]-, [1S-[1R*(R*),2S*]]-
Butanediamide, N¹-[3-[[[(1,1-dimethylethyl)amino]carbonyl]hexylamino]-2-hydroxy-1-(phenylmethyl)propyl]-2-[(2-quinolinylcarbonyl)amino]-, [1S-[1R*(R*),2S*]]-
Propanamide, N-[3-[[[(1,1-dimethylethyl)amino]carbonyl]butylamino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](phenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](4-fluorophenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](4-methoxyphenylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](cyclohexylmethyl)amino]-2-hydroxy-1-(phenylmethyl)propyl]-2-methyl-3-(methylsulfonyl)-, [1S-[1R*(R*), 2S*]]-
Propanamide, N-[3-[[[(1,1-dimethylethyl)amino]carbonyl](3-methylbutyl)amino]-2-hydroxy-1-(cyclohexylmethyl)propyl]-2-methyl-3-(methylsulfonyl)-, [1S-[1R*(R*), 2S*]]-
Butanediamide, N¹-[2-hydroxy-3-[(3-methylbutyl)(1-pyrrolidinylcarbonyl)amino]-1-(phenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-pyrrolidinylcarbonyl)amino]-1-(4-fluorophenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-pyrrolidinylcarbonyl)amino]-1-(cyclohexylmethyl)propyl]-2-[(2-quinolinylcarbonyl)amino]-, [1S-[1R*(R*),2S*]]-
Butanediamide, N¹-[2-hydroxy-3-[(phenylmethyl)(4-morpholinylcarbonyl)amino]-1-(phenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-piperidinylcarbonyl)amino]-1-(phenylmethyl)propyl]-2-[(2-quinolinylcarbonyl)amino]-, [1S-[1R*(R*),2S*]]-
Butanediamide, N¹-[2-hydroxy-3-[(phenylmethyl)(1-piperidinylcarbonyl)amino]-1-(phenylmethyl)propyl]-2-[(2-quinolinylcarbonyl)amino]-, [1S-[1R*(R*),2S*]]-
Butanediamide, N-[3-[[[(1,1-dimethylethyl)amino]carbonyl]butylamino]-2-hydroxy-1-(phenylmethyl)propyl]-2,3,3-trimethyl-, [1S-[1R*(2S*),2S*]]-
Butanediamide, N-[3-[[[(1,1-dimethylethyl)amino]carbonyl](phenylmethyl)amino]-2-hydroxy-1-(phenylmethyl)propyl]-2,3,3-trimethyl-, [1S-[1R*(2S*),2S*]]-
Butanediamide, N-[3-[[[(1,1-dimethylethyl)amino]carbonyl](4-fluorophenylmethyl)amino]-2-hydroxy-1-(phenylmethyl)propyl]-2,3,3-trimethyl-, [1S-[1R*(2S*),2S*]]-
Butanediamide, N-[3-[[[(1,1-dimethylethyl)amino]carbonyl](cyclohexylmethyl)amino]-2-hydroxy-1-(phenylmethyl)propyl]-2,3,3-trimethyl-, [1S-[1R*(2S*),2S*]]-
Butanediamide, N-[3-[[[(1,1-dimethylethyl)amino]carbonyl](3-methylbutyl)amino]-2-hydroxy-1-(cyclohexylmethyl)propyl]-2,3,3-trimethyl-, [1S-[1R*(2S*),2S*]]-
Butanoic acid, 4-[[3-[[[(1,1-dimethylethyl)amino]carbonyl]butylamino]-2-hydroxy-1-(phenylmethyl)propyl]amino-2,2,3-trimethyl-4-oxo-, [1S-[1R*(3S*),2S*]]-
Butanoic acid, 4-[[3-[[[(1,1-dimethylethyl)amino]carbonyl](phenylmethyl)amino]-2-hydroxy-1-(phenylmethyl)propyl]amino-2,2,3-trimethyl-4-oxo-, [1S-[1R*(3S*),2S*]]-
Butanoic acid, 4-[[3-[[[(1,1-dimethylethyl)amino]carbonyl](4-fluorophenylmethyl)amino]-2-hydroxy-1-(phenylmethyl)propyl]amino-2,2,3-trimethyl-4-oxo-, [1S-[1R*(3S*),2S*]]-
Butanoic acid, 4-[[3-[[[(1,1-dimethylethyl)amino]carbonyl](cyclohexylmethyl)amino]-2-hydroxy-1-(phenylmethyl)propyl]amino-2,2,3-trimethyl-4-oxo-, [1S-[1R*(3S*),2S*]]-
Butaneamide, 2-(acetylamino)-N-[3-[[[(1,1-dimethylethyl)amino]carbonyl](3-methylbutyl)amino]-2-hydroxy-1-(cyclohexylmethyl)propyl]-3,3-dimethyl-, [1S-[1R*(R*),2S*]]-
Butaneamide, 2-(acetylamino)-N-[3-[[[(1,1-dimethylethyl)amino]carbonyl]butylamino]-2-hydroxy-1-(phenylmethyl)propyl]-3,3-dimethyl-, [1S-[1R*(R*),2S*]]-
Butaneamide, 2-(acetylamino)-N-[3-[[[(1,1-dimethylethyl)amino]carbonyl](phenylmethyl)amino]-2-hydroxy-1-(phenylmethyl)propyl]-3,3-dimethyl-, [1S-[1R*(R*),2S*]]-
Butaneamide, 2-(acetylamino)-N-[3-[[[(1,1-dimethylethyl)amino]carbonyl](cyclohexylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](3-methylbutyl)amino]-2-hydroxy-1-(4-fluorophenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](3-methylbutyl)amino]-2-hydroxy-1-(cyclohexylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl]butylamino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](phenylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](4-fluorophenylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](cyclohexylmethyl)amino]-2-hydroxy-1-(phenylmethyl)propyl]-3,3-dimethyl-, [1S-[1R*(R*),2S*]]-

The compounds of the present invention are effective antiviral compounds and, in particular, are effective retroviral inhibitors as shown above. Thus, the subject compounds are effective HIV protease inhibitors. It is contemplated that the subject compounds will also inhibit other viruses such as HIV, human T-cell leukemia virus, respiratory syncitial virus, hepadnavirus, cytomegalovirus and picornavirus.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

The foregoing is merely illustrative of the invention and is not intended to limit the invention to the disclosed compounds.

The preceding examples can be repeated with similar success by substituting the generically or specifically described reactants and/or operating conditions of this invention for those used in the preceding examples.

## Claims

1. Use of a compound represented by the formula: 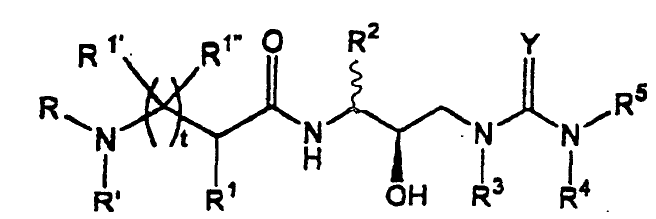 for preparing a medicament for inhibiting a retroviral protease wherein:
R represents hydrogen, alkoxycarbonyl, aralkoxycarbonyl, alkylcarbonyl, cycloalkylcarbonyl, cycloalkylalkoxycarbonyl, cycloalkylalkanoyl, alkanoyl, aralkanoyl, aroyl, aryloxycarbonyl, aryloxyalkanoyl, heterocyclocarbonyl, heterocyclyloxycarbonyl, heterocyclylalkanoyl, heterocyclylalkoxycarbonyl, heteroaralkoxycarbonyl, heteroaryloxycarbonyl, heteroaroyl, alkyl,aryl, aralkyl, aryloxyalkyl, heteroaryloxyalkyl, hydroxyalkyl, alkylaminocarbonyl, arylaminocarbonyl, aralkylaminoalkylcarbonyl, and aminoalkanoyl radicals; alkylaminoalkylcarbonyl and mono- and disubstituted aminoalkanoyl radicals wherein the substituents are selected from alkyl, aryl, aralkyl, cycloalkyl, cycloalkylalkyl, heteroaryl, heteroaralkyl, heteroalkyl, heterocycloalkylalkyl radicals;
R' represents hydrogen and radicals as defined for R³, or R and R' together with the nitrogen to which they are attached form a heterocycloalkyl or heteroaryl radical;
R¹ represents -CH₂SO₂NH₂, hydrogen, -CO₂CH₃, -CONH₂, alkyl and cycloalkyl radicals, and amino acid side chains selected from the group of asparagine, S-methyl cysteine and the sulfoxide (SO) and sulfone (SO₂) derivatives thereof, histidine, norleucine, glutamine, glycine, allo-isoleucine, alanine, threonine, isoleucine, leucine, tert-leucine, phenylalanine, ornithine, allothreonine, serine, aspartic acid, beta-cyano alanine and valine side chains;
R¹' and R¹" independently represent hydrogen and radicals as defined for R¹ ;
R² represents alkyl, aryl, cycloalkyl, cycloalkylalkyl, and aralkyl radicals, which radicals are optionally substituted with a group selected f rom halogen radicals and -NO₂, -OR⁹ and -SR⁹ wherein R⁹ represents hydrogen and alkyl radicals;
R³ represents alkyl, alkenyl, hydroxyalkyl, cycloalkyl, cycloalkylalkyl, heterocycloalkyl, heterocycloalkylalkyl, aryl, aralkyl, heteroaryl and heteroaralkyl radicals;
R⁴ and R⁵ independently represent hydrogen and radicals as defined by R³, or together with the nitrogen atom to which they are bonded represent a heterocycloalkyl or heteroaryl radical,
t represents, 0 or 1.; and
Y represents O or S.
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro,
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom.
- heteroaryl is an aromatic monocyclic, bicyclic, or tricyclic heterocycle which contains the hetero atoms and is optionally substituted as defined above with respect to the definition of heterocycloalkyl.
- heteroaralkyl is an alkyl radical having 1 to 10 carbon atoms in which one hydrogen is replaced by a heteroaryl.

2. The use recited in Claim 1 wherein R represents aralkoxycarbonyl and heteroaroyl radicals; R¹ represents alkyl radicals and amino acid side chains selected from the group consisting of asparagine, valine, threonine, allo-threonine, isoleucine, S-methyl cysteine and the sulfone and sulfoxide derivatives thereof, alanine, and allo-isoleucine; and R³, R⁴, and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals.

3. The use recited in Claim 1 wherein R represents carbobenzoxy, 2-benzofurancarbonyl and 2-quinolinylcarbonyl radicals, R¹ represents methyl, t-butyl, isopropyl and sec-butyl radicals, and amino acid side chains selected from the group consisting of asparagine, valine, S-methyl cysteine, allo-iso-leucine, iso-leucine, threonine, srine, aspartic acid, beta-cyano alanine, and allo-threonine side chains; R² represents CH₃SCH₂CH₂-, iso-butyl, n-butyl, benzyl, 4-fluorobenzyl, 2-naphthylmethyl and cyclohexylmethyl radicals; and R³, R⁴ and R⁵ independently represent alkyl radicals having from about 2 to about 5 carbon atoms, cycloalkylalkyl radicals, aralkyl radicals, heterocycloalkylalkyl radicals and heteroaralkyl radicals.

4. The use recited in Claim 1 wherein t is 0.

5. The use recited in Claim 1 wherein t is 1.

6. The use recited in Claim 3 wherein R³ represents benzyl, para-fluorobenzyl, para-methoxybenzyl, para-methylbenzyl, and 2-naphthylmethyl radicals and R⁴ represents t-butyl.

7. The use recited in Claim 1 wherein R⁴ and R⁵ together with the nitrogen atom to which they are attached represent a 5 or 6-membered heterocycle radical optionally substituted with an alkyl radical having from 1 to about 3 carbon atoms.

8. The use recited in Claim 7 wherein R⁴ and R⁵ together with the nitrogen to which they are bonded represent pyrrolidinyl, piperidinyl, morpholinyl, and piperazinyl radicals.

9. The use recited in Claim 1 wherein the retroviral protease is HIV protease.

10. Use of a compound represented by the formula: 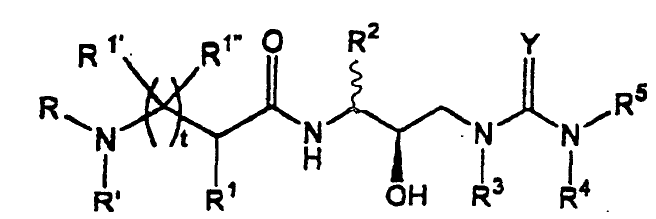 for preparing a medicament for treating a retroviral infection wherein:
R represents hydrogen, alkoxycarbonyl, aralkoxycarbonyl, alkylcarbonyl, cycloalkylcarbonyl, cycloalkylalkoxycarbonyl, cycloalkylalkanoyl, alkanoyl, aralkanoyl, aroyl, aryloxycarbonyl, aryloxyalkanoyl, heterocyclocarbonyl, heterocyclyloxycarbonyl, heterocyclylalkanoyl, heterocyclylallcoxycarbonyl, heteroarallcoxycarbonyl, heteroaryloxycarbonyl, heteroaroyl, alkyl,aryl, aralkyl, aryloxyalkyl, heteroaryloxyalkyl, hydroxyalkyl, alkylaminocarbonyl, arylaminocarbonyl, aralkylaminoalkylcarbonyl, and aminoalkanoyl radicals; alkylaminoalkylcarbonyl and mono- and disubstituted aminoalkanoyl radicals wherein the substituents are selected from alkyl, aryl, aralkyl, cycloalkyl, cycloalkylalkyl, heteroaryl, heteroaralkyl, heteroalkyl, heterocycloalkylalkyl radicals;
R' represents hydrogen and radicals as defined for R³, or R and R' together with the nitrogen to which they are attached form a heterocycloalkyl or heteroaryl radical;
R¹ represents -CH₂SO₂NH₂, hydrogen, -CO₂CH₃, -CONH₂, alkyl and cycloalkyl radicals, and amino acid side chains selected from the group of asparagine, S-methyl cysteine and the sulfoxide (SO) and sulfone (SO₂) derivatives thereof, histidine, norleucine, glutamine, glycine, allo-isoleucine, alanine, threonine, isoleucine, leucine, tert-leucine, phenylalanine, ornithine, allo-threonine, serine, aspartic acid, beta-cyano alanine and valine side chains;
R^{1'} and R^{1''} independently represent hydrogen and radicals as defined for R¹ ;
R² represents alkyl, aryl, cycloalkyl, cycloalkylalkyl, and aralkyl radicals, which radicals are optionally substituted with a group selected f rom halogen radicals and -NO₂, -OR⁹ and -SR⁹ wherein R⁹ represents hydrogen and alkyl radicals;
R³ represents alkyl, alkenyl, hydroxyalkyl, cycloalkyl, cycloalkylalkyl, heterocycloalkyl, heterocycloalkylalkyl, aryl, aralkyl, heteroaryl and heteroaralkyl radicals;
R⁴ and R⁵ independently represent hydrogen and radicals as defined by R³, or together with the nitrogen atom to which they are bonded represent a heterocycloalkyl or heteroaryl radical,
t represents, 0 or 1.; and
Y represents O or S,
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro,
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is a alkyl radical having 1 to 10 carbon atoms in which one hydrogen is replaced by a heteroaryl.

11. The use recited in Claim 10 wherein R represents aralkoxycarbonyl and heteroaroyl radicals; R¹ represents alkyl radicals and amino acid side chains selected from the group consisting of asparagine, valine, threonine, allo-threonine, isoleucine, S-methyl cysteine and the sulfone and sulfoxide derivatives thereof, alanine, and allo-isoleucine; and R³, R⁴, and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals.

12. The use recited in Claim 10 wherein R represents carbobenzoxy, 2-benzofurancarbonyl and 2-quinolinylcarbonyl radicals, R¹ represents methyl, t-butyl, isopropyl and sec-butyl radicals, and amino acid side chains selected from the group consisting of asparagine, valine, S-methyl cysteine, allo-iso-leucine, iso-leucine, threonine, srine, aspartic acid, beta-cyano alanine, and allo-threonine side chains; R² represents CH₃SCH₂CH₂-, iso-butyl, n-butyl, benzyl, 4-fluorobenzyl, 2-naphthylmethyl and cyclohexylmethyl radicals; and R³, R⁴ and R⁵ independently represent alkyl radicals having from about 2 to about 5 carbon atoms, cycloalkylalkyl radicals, aralkyl radicals, heterocycloalkylalkyl radicals and heteroaralkyl radicals.

13. The use recited in Claim 10 wherein t is 0.

14. The use recited in Claim 10 wherein t is 1.

15. The use recited in Claim 12 wherein R³ represents benzyl, para-fluorobenzyl, para-methoxybenzyl, para-methylbenzyl, and 2-naphthylmethyl radicals and R⁴ represents t-butyl.

16. The use recited in Claim 10 wherein R⁴ and R⁵ together with the nitrogen atom to which they are attached represent a 5 or 6-membered heterocycle radical optionally substituted with an alkyl radical having from 1 to about 3 carbon atoms.

17. The use recited in Claim 16 wherein R⁴ and R⁵ together with the nitrogen to which they are bonded represent pyrrolidinyl, piperidinyl, morpholinyl, and piperazinyl radicals.

18. The use recited in Claim 10 wherein the retroviral infection is an HIV infection.

19. Use of a compound represented by the formula: 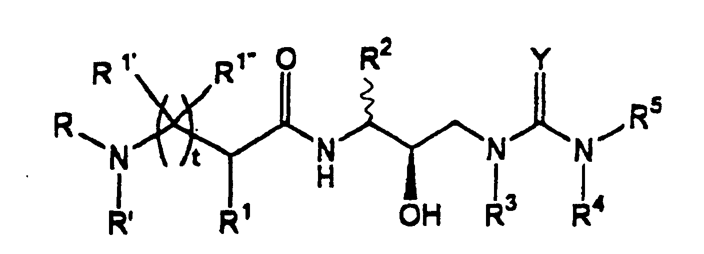 for preparing a medicament for treating AIDS wherein:
R represents hydrogen, alkoxycarbonyl, aralkoxycarbonyl, alkylcarbonyl, cycloalkylcarbonyl, cycloalkylalkoxycarbonyl, cycloalkylalkanoyl, alkanoyl, aralkanoyl, aroyl, aryloxycarbonyl, aryloxyalkanoyl, heterocyclocarbonyl, heterocyclyloxycarbonyl, heterocyclylalkanoyl, heterocyclylalkoxycarbonyl, heteroaralkoxycarbonyl, heteroaryloxycarbonyl, heteroaroyl, alkyl,aryl, aralkyl, aryloxyalkyl, heteroaryloxyalkyl, hydroxyalkyl, alkylaminocarbonyl, arylaminocarbonyl, aralkylaminoallcylcarbonyl, and aminoalkanoyl radicals; alkylaminoalkylcarbonyl and mono- and disubstituted aminoalkanoyl radicals wherein the substituents are selected from alkyl, aryl, aralkyl, cycloalkyl, cycloalkylalkyl, heteroaryl, heteroaralkyl, heteroalkyl, heterocycloalkylalkyl radicals;
R' represents hydrogen and radicals as defined for R³, or R and R' together with the nitrogen to which they are attached form a heterocycloalkyl or heteroaryl radical;
R¹ represents -CH₂SO₂NH₂, hydrogen, -CO₂CH₃, -CONH₂, alkyl and cycloalkyl radicals, and amino acid side chains selected from the group of asparagine, S-methyl cysteine and the sulfoxide (SO) and sulfone (SO₂) derivatives thereof, histidine, norleucine, glutamine, glycine, allo-isoleucine, alanine, threonine, isoleucine, leucine, tert-leucine, phenylalanine, omithine, allo-threonine, serine, aspartic acid, beta-cyano alanine and valine side chains;
R^{1'} and R^{1"} independently represent hydrogen and radicals as defined for R¹ ;
R² represents alkyl, aryl, cycloalkyl, cycloalkylalkyl, and aralkyl radicals, which radicals are optionally substituted with a group selected f rom halogen radicals and -NO₂, -OR⁹ and -SR⁹ wherein R⁹ represents hydrogen and alkyl radicals;
R³ represents alkyl, alkenyl, hydroxyalkyl, cycloalkyl, cycloalkylalkyl, heterocycloalkyl, heterocycloalkylalkyl, aryl, aralkyl, heteroaryl and heteroaralkyl radicals;
R⁴ and R⁵ independently represent hydrogen and radicals as defined by R³, or together with the nitrogen atom to which they are bonded represent a heterocycloalkyl or heteroaryl radical,
t represents, 0 or 1.; and
Y represents O or S,
and wherein
- alkyl means alkyl having from 1 to 10 carbon atoms,
- cycloalkyl means cycloalkyl having from 3 to 8 carbon atoms,
- aralkyl means an alkyl having from 1 to 10 carbon atoms in which one hydrogen is replaced by an aryl,
- aryl is phenyl or naphthyl, which optionally carries one or more substituents selected from alkyl, alkoxy, halogen, hydroxy, amino and nitro
- heterocycloalkyl is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy or oxo, and/or on a secondary nitrogen atom by alkyl, aralkoxycarbonyl, alkanoyl, phenyl or phenylalkyl or on a tertiary nitrogen atom by oxido and which is attached via a carbon atom,
- heteroaryl is an aromatic monocyclic, bicyclic, or tricyclic heterocycle which contains the hetero atoms and is optionally substituted as defined above with respect to the definition of heterocycloalkyl,
- heteroaralkyl is an alkyl radical having 1 to 10 carbon atoms in which one hydrogen is replaced by a heteroaryl.

20. The use recited in Claim 19 wherein R represents aralkoxycarbonyl and heteroaroyl radicals; R¹ represents alkyl radicals and amino acid side chains selected from the group consisting of asparagine, valine, threonine, allo-threonine, isoleucine, S-methyl cysteine and the sulfone and sulfoxide derivatives thereof, alanine, and allo-isoleucine; and R³, R⁴, and R⁵ independently represent alkyl, alkenyl, hydroxyalkyl, cycloalkyl, cycloalkylalkyl, heterocycloalkyl, heterocycloalkylalkyl, aryl, aralkyl and heteroaralkyl radicals.

21. The use recited in Claim 19 wherein R represents carbobenzoxy, 2-benzofurancarbonyl and 2-quinolinylcarbonyl radicals, R¹ represents methyl, t-butyl, isopropyl and sec-butyl radicals, and amino acid side chains selected from the group consisting of asparagine, valine, S-methyl cysteine, allo-iso-leucine, iso-leucine, threonine, srine, aspartic acid, beta-cyano alanine, and allo-threonine side chains; R² represents CH₃SCH₂CH₂-, iso-butyl, n-butyl, benzyl, 4-fluorobenzyl, 2-naphthylmethyl and cyclohexylmethyl radicals; and R³, R⁴ and R⁵ independently represent alkyl radicals having from about 2 to about 5 carbon atoms, cycloalkylalkyl radicals, aralkyl radicals, heterocycloalkylalkyl radicals and heteroaralkyl radicals.

22. The use recited in Claim 19 wherein t is 0.

23. The use recited in Claim 19 wherein t is 1.

24. The use recited in Claim 21 wherein R³ represents benzyl, para-fluorobenzyl, para-methoxybenzyl, para-methylbenzyl, and 2-naphthylmethyl radicals and R⁴ represents t-butyl.

25. The use recited in Claim 19 wherein R⁴ and R⁵ together with the nitrogen atom to which they are attached represent a 5 or 6-membered heterocycle radical optionally substituted with an alkyl radical having from 1 to about 3 carbon atoms.

26. The use recited in Claim 25 wherein R⁴ and R⁵ together with the nitrogen to which they are bonded represent pyrrolidinyl, piperidinyl, morpholinyl, and piperazinyl radicals.

27. The use recited in any of the claims 1-26 wherein the compound is selected among the group consisting of the compounds in the following tables

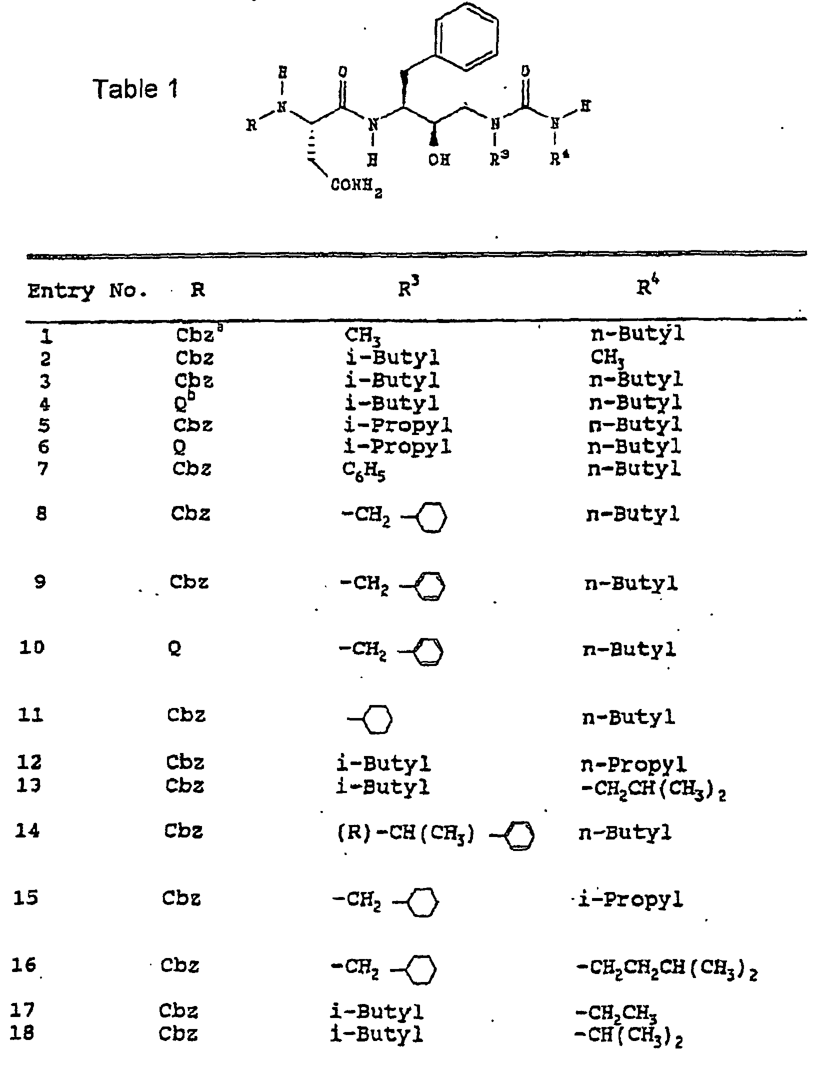

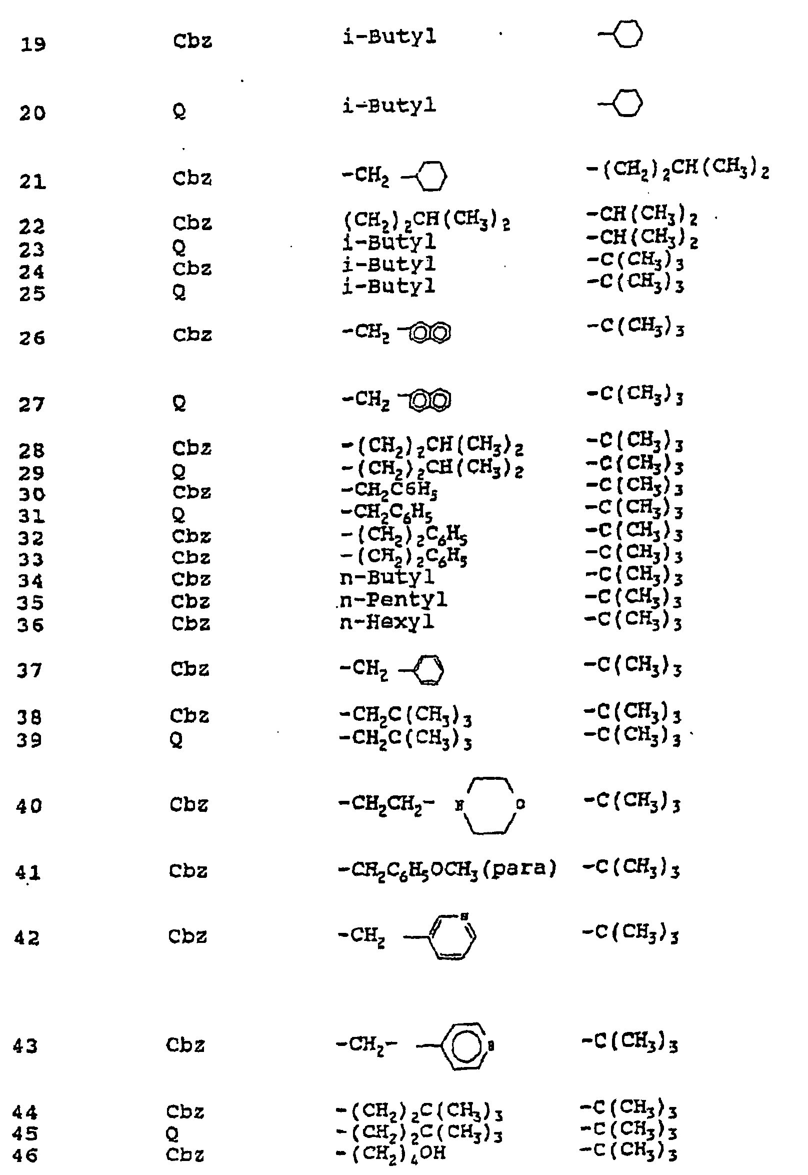

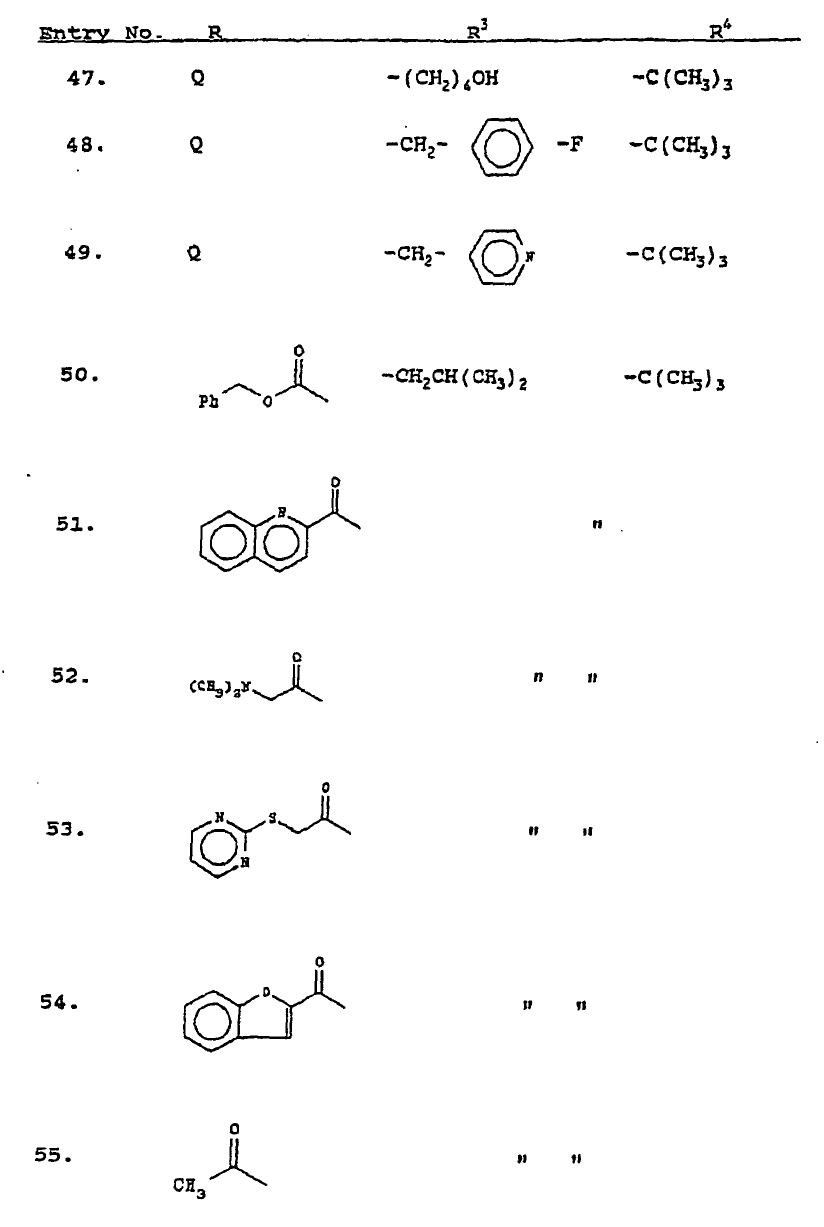

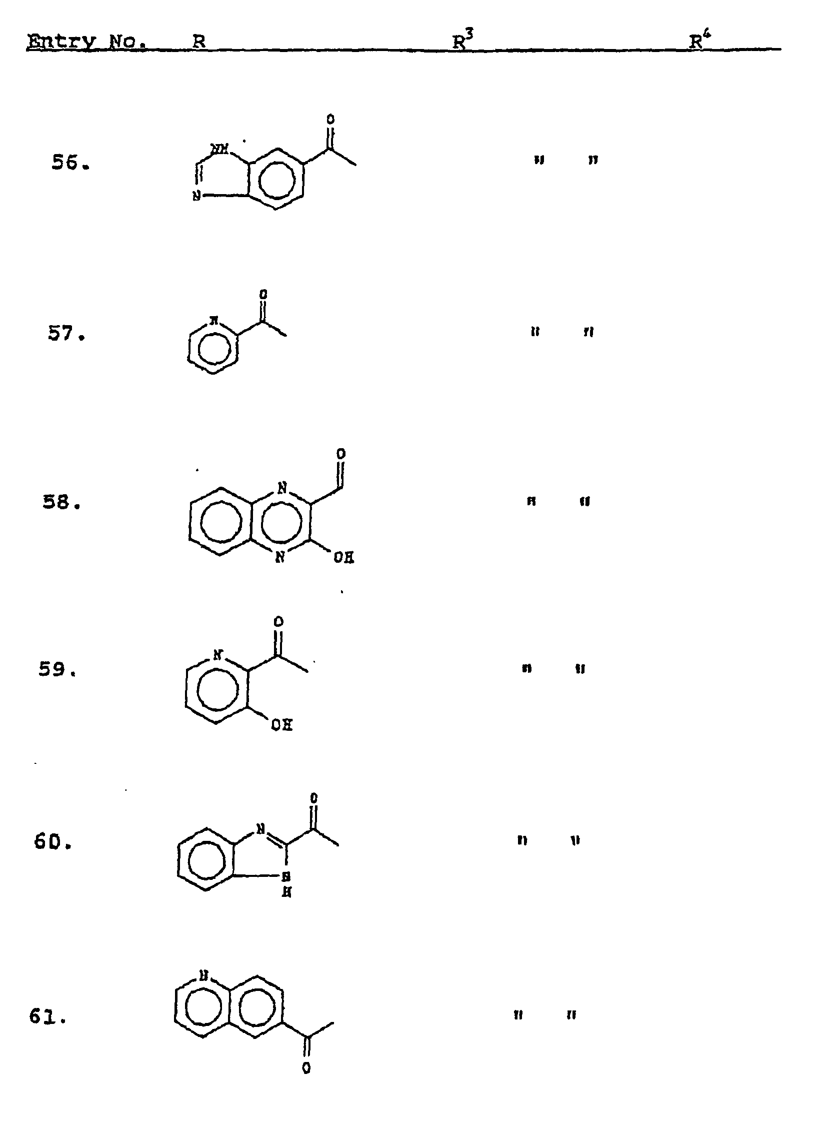

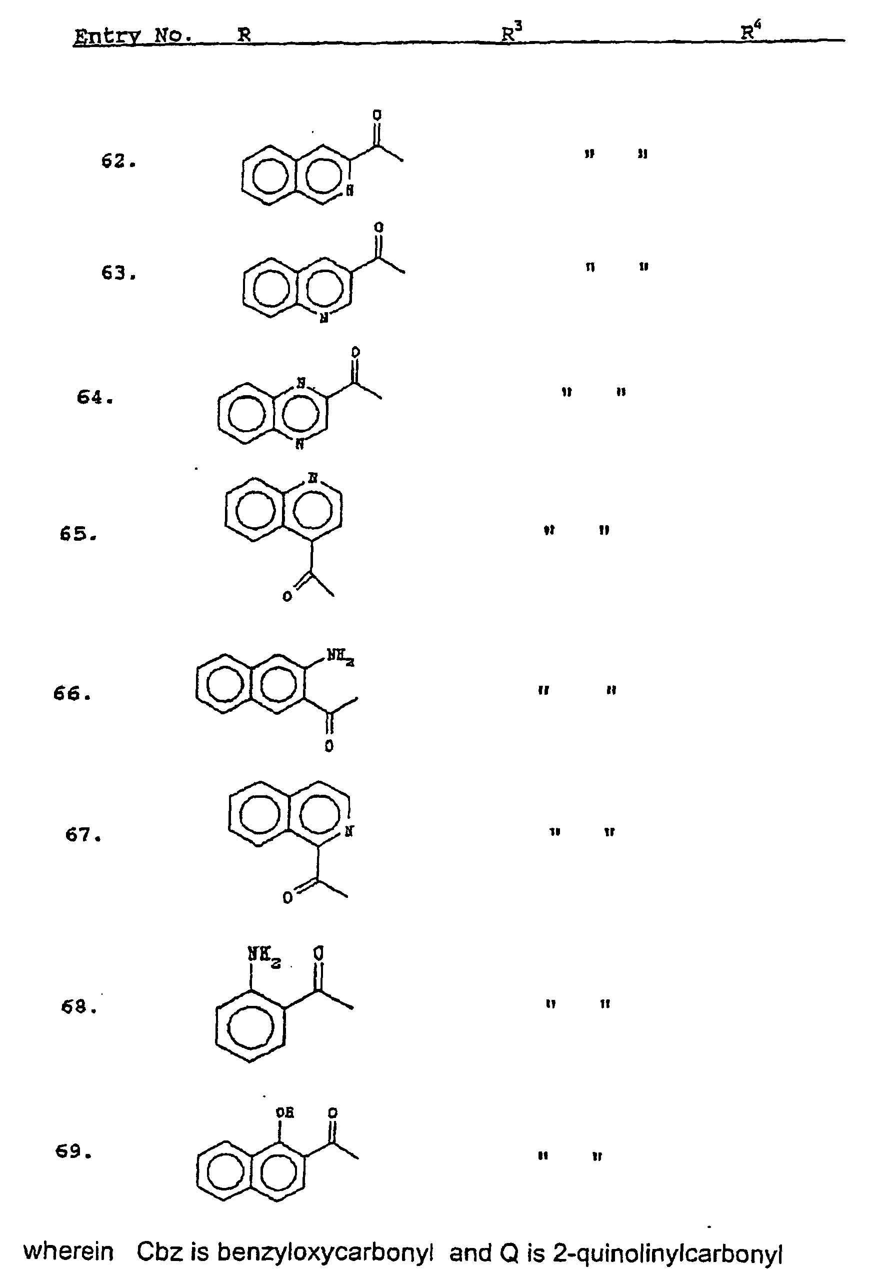

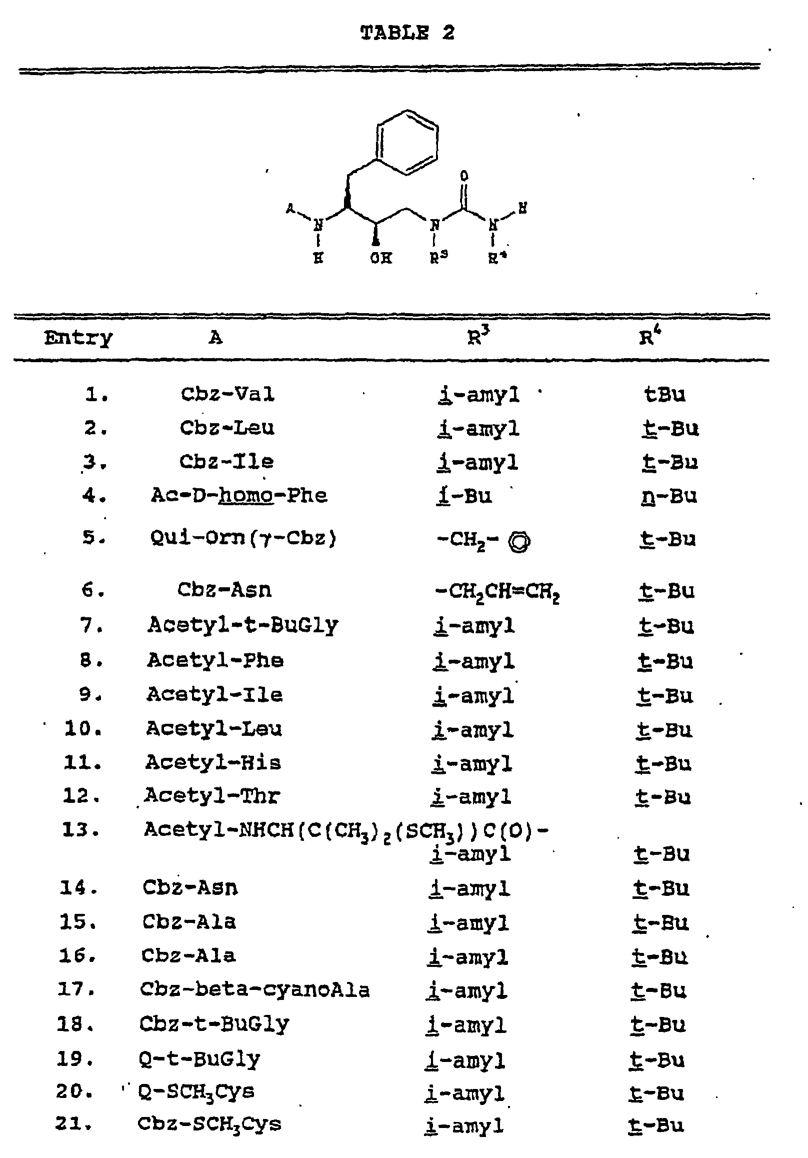

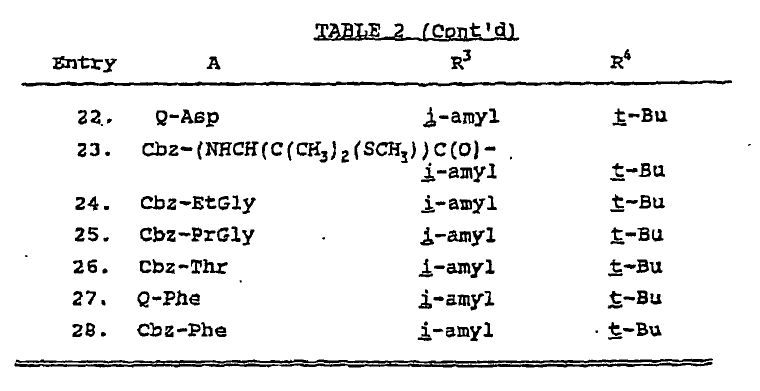

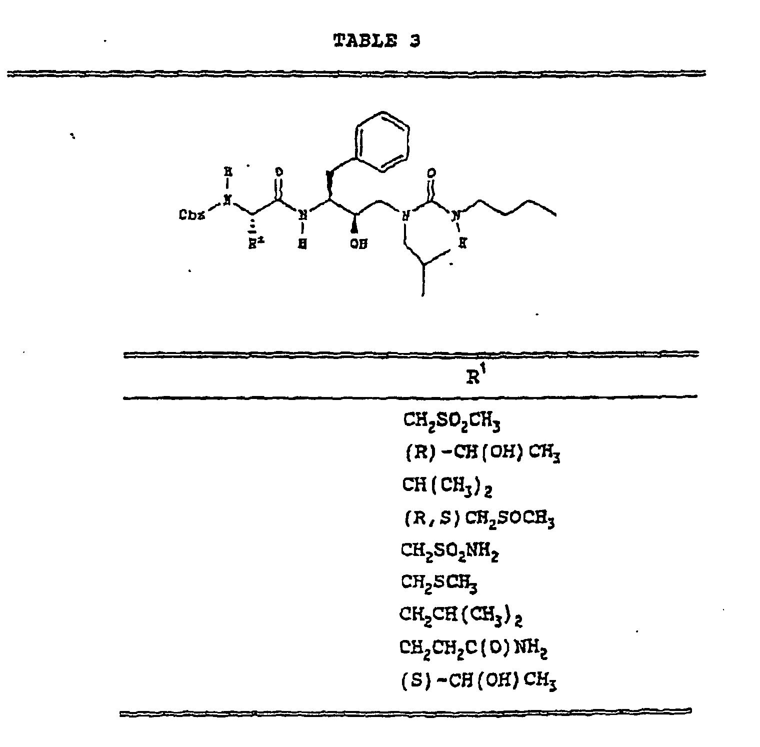

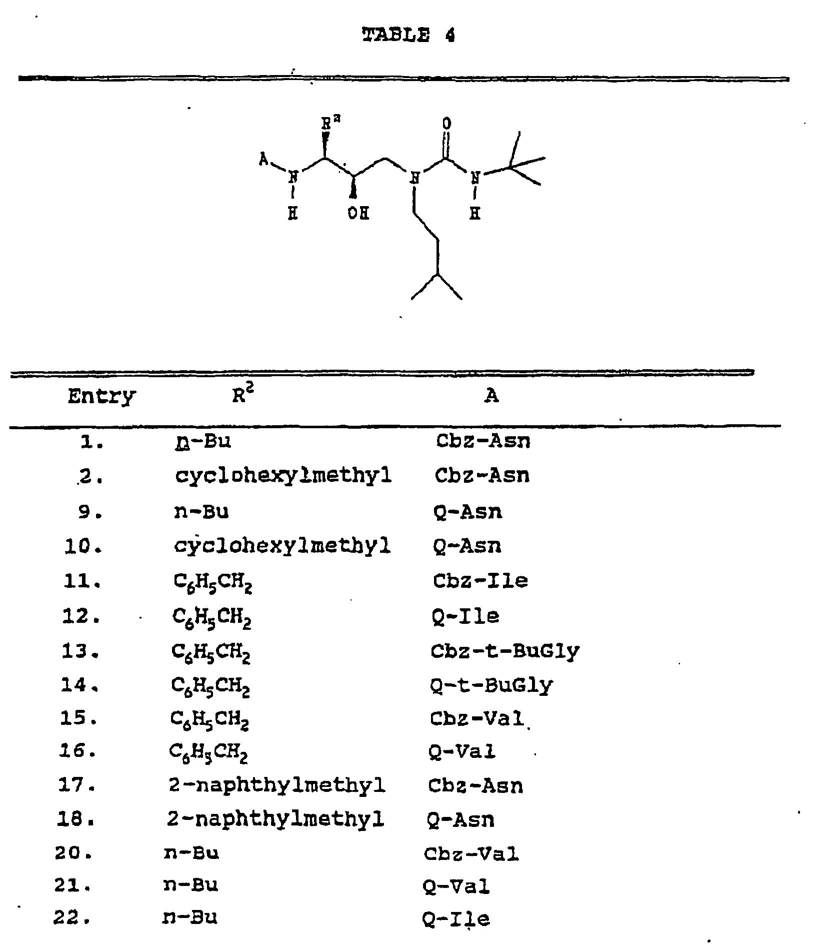

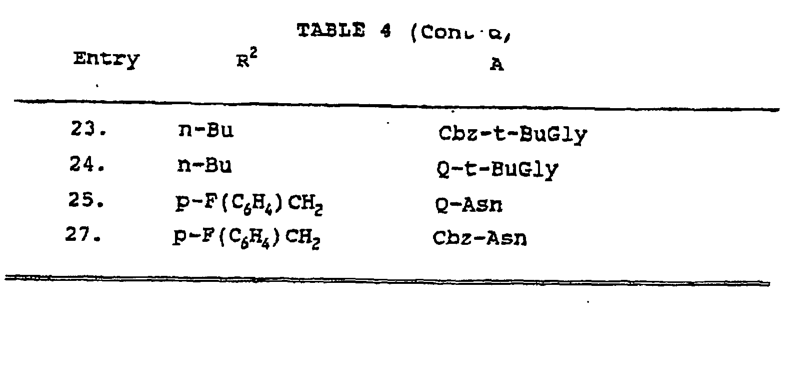

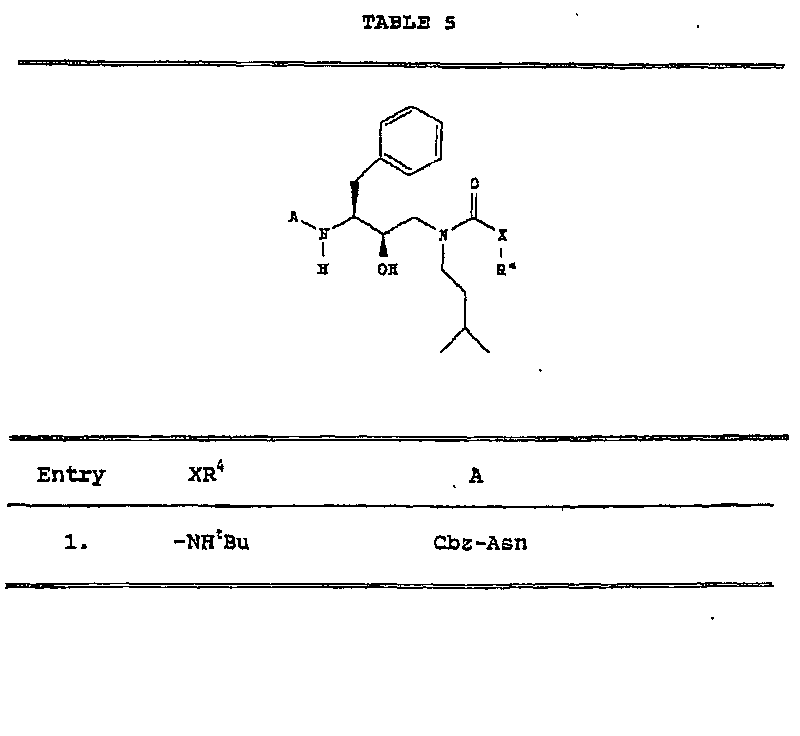

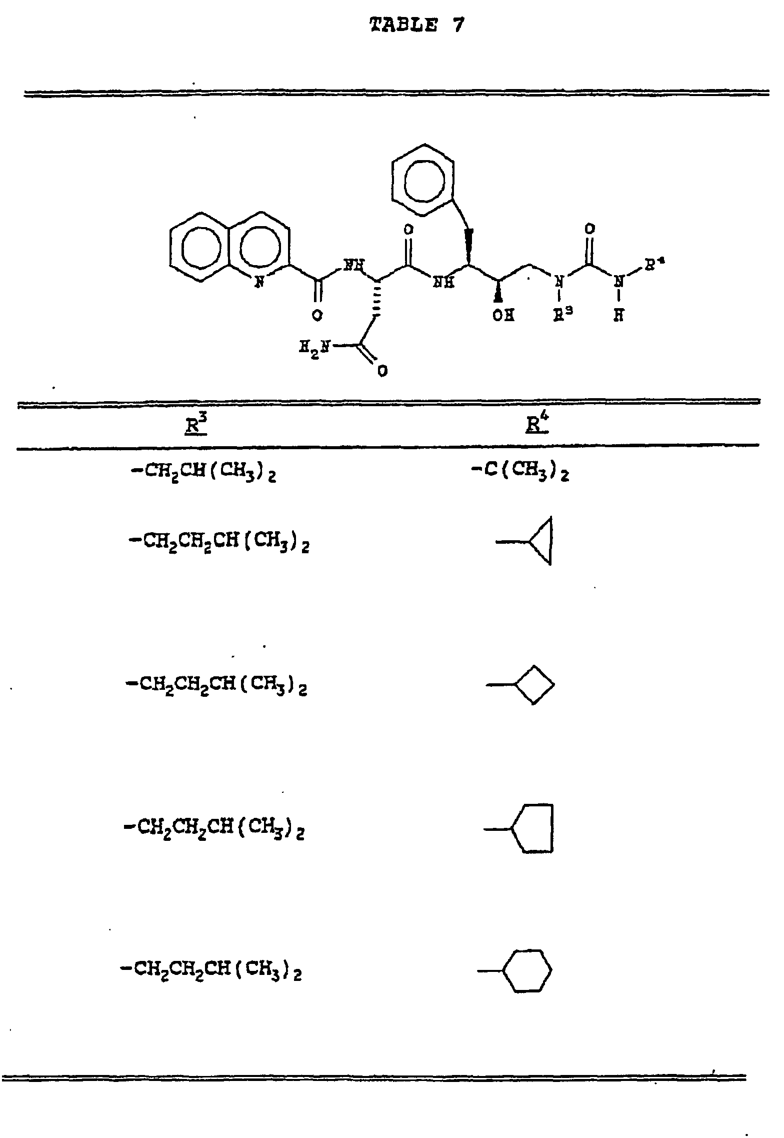

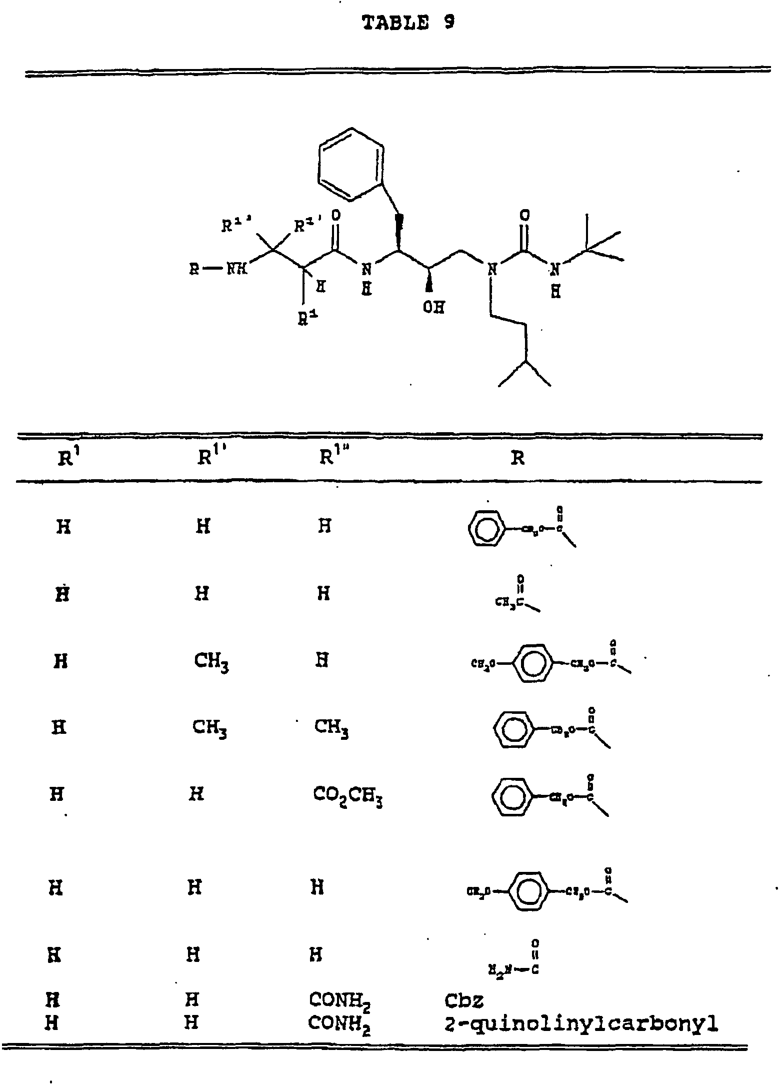

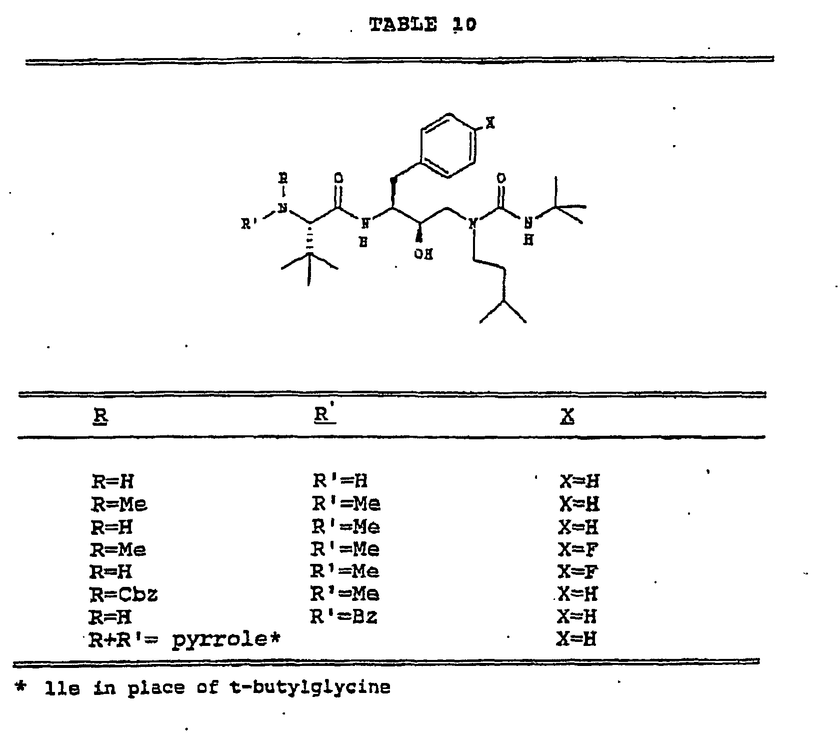

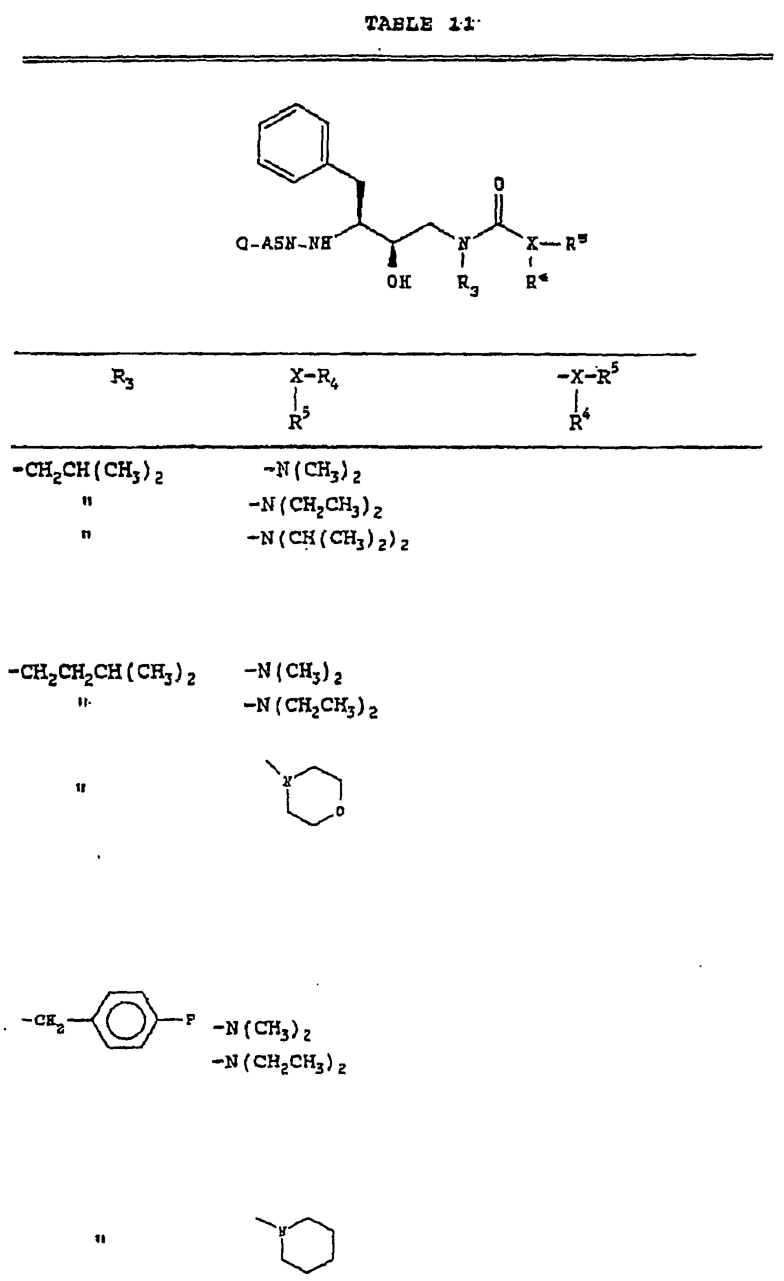

28. The use recited in any of the claims 1-26 wherein the compound is selected among the group consisting of
Butanediamide, N¹-[3-[[[(1,1-dimethylethyl)amino]carbonyl] (2-methylpropyl)amino]-2-hydroxy-1-(phenylmethyl)propyl]-2-[(N-benzyl-oxycarbonyl)amino]-, [1S-[1R*(R*), 2S*]]-
Butanediamide, N¹-[3-[[[(1,1-dimethylethyl)amino]carbonyl] (2-methylpropyl)amino]-2-hydroxy-1-(phenylmethyl)propyl]-2-[(2-quinolinylcarbonyl)amino]-, [1S-[1R*(R*),2S*]]-
Butanediamide, N¹-[3-[[[(1,1-dimethylethyl)amino]carbonyl](3-methylbutyl)amino]-2-hydroxy-1-(phenylmethyl)propyl]-2-[(N-benzyloxycarbonyl)amino], [1S-[1R*(R*),2S*]]-
Butanediamide, N¹-[3-[[[(1,1-dimethylethyl)amino]carbonyl](3-methylbutyl)amino]-2-hydroxy-1-(phenylmethyl)propyl]-2-[(2-quinolinylcarbonyl)amino]-, [1S-[1R*(R*),2S*]]-
Butanediamide, N¹-[3-[[[(1,1-dimethylethyl)amino]carbonyl](2-naphthylmethyl)amino]-2-hydroxy-1-(phenylmethyl)propyl]-2-[(N-benzyloxycarbonyl)amino]-, [1S-[1R*(R*), 2S*]]-
Butanediamide, N¹-[3-[[[(1,1-dimethylethyl)amino]carbonyl](2-naphthylmethyl)amino]-2-hydroxy-1-(phenylmethyl)propyl]-2-[(2-quinolinylcarbonyl)amino]-, [1S-[1R*(R*),2S*]]-
Butanediamide, N¹-[3-[[[(1,1-dimethylethyl)amino]carbonyl](2-phenylethyl)amino]-2-hydroxy-1-(phenylmethyl)propyl]-2-[(N-benzyloxycarbonyl)amino]-, [1S-[1R*(R*),2S*]]-
Butanediamide, N¹-[3-[[[(1,1-dimethylethyl)amino]carbonyl] (2-phenylethyl)amino]-2-hydroxy-1-(phenylmethyl)propyl]-2-[(2-quinolinylcarbonyl)amino]-, [1S-[1R*(R*),2S*]]-
Butanediamide, N¹-[3-[[[(1,1-dimethylethyl)amino]carbonyl](3,3-dimethylpropyl)amino]-2-hydroxy-1-(phenylmethyl)propyl]-2-[(N-benzyloxycarbonyl)amio], [1S-[1R*(R*),2S*]]-
Butanediamide, N¹-[3-[[[(1,1-dimethylethyl)amino]carbonyl](3,3-dimethylpropyl)amino]-2-hydroxy-1-(phenylmethyl)propyl]-2-[(2-quinolinylcarbonyl)amino]-, [1S-[1R*(R*),2S*]]-
Propanamide, 3-(acetylamino)-N-[3-[[[(11,-dimethylethyl)amino]carbonyl] (3-methylbutyl)amino]-2-hydroxy-1-(phenylmethyl)propyl]-2-methyl-, [1S-[1R*(S*),2S*]]-
Propanamide, 3-(4-methoxybenzyloxycarbonyl)amino-N-[3-[[[(11,-dimethylethyl)amino]carbonyl](3-methylbutyl) amino]-2-hydroxy-1-(phenylmethyl) propyl]-2-methyl-, [1S-[1R*(S*),2S*]]-
Butanediamide, N¹-[2-hydroxy-3-[(4-fluorophenylmethyl)(1-pyrrolidinylcarbonyl)amino]-1-(phenylmethyl) propyl]-2-[(2-quinolinylcarbonyl) amino]-, [1S-[1R*(R*),2S*]]-
Butaneamide,2-(acetylamino)-N-[3-[[[(1,1-dimethylethyl)amino]carbonyl](3-methylbutyl)amino]-2-hydroxy-1-(phenylmethyl)propyl]-3,3-dimethyl-, [1S-[1R*(R*),2S*]]-
butanediamide N¹[3-[[[n-butylamino]carbonyl](2-methylpropyl)amino]-2-hydroxy-1-(phenylmethyl)propyl]-2-[(phenylmethylcarbamoyl) amino],[1S-[1R*(R*), 2S*]]--.
butanediamide N¹[3-[[[n-butylamino]carbonyl](2-methylpropyl)amino]-2-hydroxy-1-(phenylmethyl)propyl]-2-[(2-quinolinylcarbonyl)amino]-[1S-[1R*(R*), 2S*]]-.
butanediamide N¹[3-[[[(1,1-dimethylethyl)amino]carbonyl](2-phenylethyl)amino]-2-hydroxy-1-(phenylmethyl)propyl]-2-[(2-quinolinylcarbonyl)amino] [1S-[1R*(R*), 2S*]]-.
butanediamide N¹[3-[[[(1,1-dimethylethyl)amino]carbonyl](2-methylpropyl)amino-2-hydroxy-1-(phenylmethyl)propyl]-2-[(2-quinolinylcarbonyl)amino] [1S-[1R*(R*), 2S*]]-.
Butanediamide, N¹-[3-[[[(1,1-dimethylethyl)amino]carbonyl]pentylanino]-2-hydroxy-1-(phenylmethyl)propyl]-2-[(2-quinolinylcarbonyl)amino]-, [1S-[1R*(R*),2S*]]-
Butanediamide, N¹-[3-[[[(1,1-dimethylethyl)amino]carbonyl]hexylamino]-2-hydroxy-1-(phenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-pyrrolidinylcarbonyl)amino]-1-(phenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-pyrrolidinylcarbonyl)amino]-1-(4-fluorophenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-pyrrolidinylcarbonyl)amino]-1-(cyclohexylmethyl)propyl]-2-[(2-quinolinylcarbonyl)amino]-, [1S-[1R*(R*),2S*]]-
Butanediamide, N¹-[2-hydroxy-3-[(phenylmethyl)(4-morpholinylcarbonyl)amino]-1-(phenylmethyl)propyl]-2-[(2-quinolinylcarbonyl)amino]-, [1S-[1R*(R*),2S*]]-
Butanediamide, N¹-[2-hydroxy-3-[(3-methylbutyl)(1-piperidinylcarbonyl)amino]-1-(phenylmethyl)propyl]-2-[(2-quinolinylcarbonyl)amino]-, [1S-[1R*(R*),2S*]]-
Butanediamide, N¹-[2-hydroxy-3-[(phenylmethyl)(1-piperidinylcarbonyl)amino]-1-(phenylmethyl)propyl]-2-[(2-quinolinylcarbonyl)amino]-, [1S-[1R*(R*),2S*]]-
Butaneamide, 2-(acetylamino)-N-[3-[[[(1,1-dimethylethyl)amino]carbonyl](3-methylbutyl)amino]-2-hydroxy-1-(cyclohexylmethyl)propyl]-3,3-dimethyl-, [1S-[1R*(R*),2S*]]-
Butaneamide, 2-(acetylamino)-N-[3-[[[(1,1-dimethylethyl)amino]carbonyl]butylamino]-2-hydroxy-1-(phenylmethyl)propyl]-3,3-dimethyl-, [1S-[1R*(R*),2S*]]-
Butaneamide, 2-(acetylamino)-N-[3-[[[(1,1-dimethylethyl)amino]carbonyl](phenylmethyl)amino]-2-hydroxy-1-(phenylmethyl)propyl]-3,3-dimethyl-, [1S-[1R*(R*),2S*]]-
Butaneamide, 2-(acetylamino)-N-[3-[[[(1,1-dimethylethyl)amino]carbonyl](cyclohexylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](3-methylbutyl)amino]-2-hydroxy-1-(4-fluorophenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](3-methylbutyl)amino]-2-hydroxy-1-(cyclohexylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl]butylamino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](phenylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](4-fluorophenylmethyl)amino]-2-hydroxy-1-(phenylmethyl)propyl]-3,3-dimethyl-, [1S-[1R*(R*),2S*]]-
Butaneamide, 2-[(2,2-dimethylaminoacetyl)amino]-N-[3-[[[(1,1-dimethylethyl)amino]carbonyl](cyclohexylmethyl)amino]-2-hydroxy-1-(phenylmethyl)propyl]-3,3-dimethyl-, [1S-[1R*(R*),2S*]]-

## Patentansprüche

1. Verwendung einer Verbindung mit der Formel 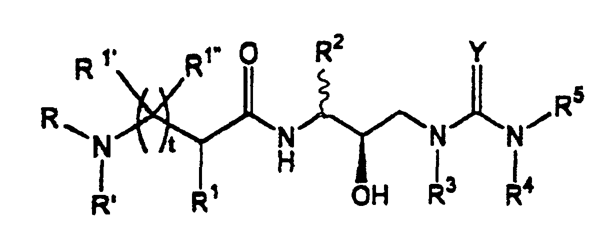 für die Herstellung eines Arzneimittels zum Inhibieren einer Retrovirusprotease, worin
- R Wasserstoff, einen Alkoxycarbonyl-, Aralkoxycarbonyl-, Alkylcarbonyl-, Cycloalkylcarbonyl-, Cycloalkylalkoxycarbonyl-, Cycloalkylalkanoyl-, Alkanoyl-, Aralkanoyl-, Aroyl-, Aryloxycarbonyl-, Aryloxyalkanoyl-, Heterocyclocarbonyl-, Heterocyclyloxycarbonyl-, Heterocyclylalkanoyl-, Heterocyclylalkoxycarbonyl-, Heteroaralkoxycarbonyl-, Heteroaryloxycarbonyl-, Heteroaroyl-, Alkyl-, Aryl-, Aralkyl-, Aryloxyalkyl-, Heteroaryloxyalkyl-, Hydroxyalkyl-, Alkylaminocarbonyl-, Arylaminocarbonyl-, Aralkylaminoalkylcarbonyl- und Aminoalkanoylrest, Alkylaminoalkylcarbonyl- und einen mono- und disubstituierten Aminoalkanoylrest, worin die Substituenten aus einem Alkyl-, Aryl-, Aralkyl-, Cycloalkyl-, Cycloalkylalkyl-, Heteroaryl-, Heteroaralkyl-, Heteroalkyl- und Heterocycloalkylalkylrest ausgewählt sind, bedeutet,
- R' Wasserstoff und einen wie für R³ definierten Rest bedeutet oder R und R' zusammen mit dem Stickstoff, an welchem sie befestigt sind, einen Heterocycloalkyl- oder Heteroarylrest bilden,
- R¹ -CH₂SO₂NH₂, Wasserstoff, -CO₂CH₃, -CONH₂, einen Alkyl- und Cycloalkylrest und eine Aminosäureseitenkette bedeutet, die aus der Gruppe ausgewählt ist, die aus einer Asparagin, S-Methylcystein- und deren Sulfoxid-(SO-) und Sulfon-(SO₂-)Derivaten, Histidin-, Norleucin-, Glutamin-, Glycin-, Alloisoleucin-, Alanin-, Threonin-, Isoleucin-, Leucin-, *tert*.-Leucin-, Phenylalanin-, Ornithin-, Allothreonin-, Serin-, Asparaginsäure-, β-Cyanoalanin- und Valinseitenkette besteht,
- R^{1'} und R^{1"} unabhängig voneinander Wasserstoff und einen wie für R¹ definierten Rest bedeuten,
- R² einen Alkyl-, Aryl-, Cycloalkyl-, Cycloalkylalkyl- und Aralkylrest, wobei die Reste wahlweise mit einer Gruppe substituiert sind, die aus Halogenresten und -NO₂, -OR⁹ und -SR⁹ ausgewählt ist, worin R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl-, Heteroaryl- und Heteroaralkylrest bedeutet und
- R⁴ und R⁵ unabhängig voneinander Wasserstoff und einen wie für R³ definierten Rest bedeuten oder zusammen mit dem Stickstoffatom, an welches sie gebunden sind, einen Heterocycloalkyl- oder Heteroarylrest bilden,
- t 0 oder 1 und
- Y O oder S bedeutet und
- Alkyl einen Alkylrest mit 1 bis 10 Kohlenstoffatomen,
- Cycloalkyl einen Cycloalkylrest mit 3 bis 8 Kohlenstoffatomen,
- Aralkyl einen Alkylrest mit 1 bis 10 Kohlenstoffatomen, worin ein Wasserstoff durch einen Arylrest ersetzt ist,
- Aryl einen Phenyl- oder Naphthylrest, der wahlweise einen oder mehrere Substituenten trägt, die aus Alkyl, Alkoxy, Halogen, Hydroxy, Amino und Nitro ausgewählt sind,
- Heterocycloalkyl einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, und welcher wahlweise an einem oder mehreren Kohlenstoffatomen mit Halogen, Alkyl, Alkoxy oder Oxo und/oder an einem sekundären Stickstoffatom mit Alkyl, Aralkoxycarbonyl, Alkanoyl, Phenyl oder Phenylalkyl oder an einem tertiären Stickstoffatom durch Oxido substituiert ist und welcher über ein Kohlenstoffatom befestigt ist,
- Heteroaryl einen aromatischen monocyclischen, bicyclischen oder tricyclischen Heterocyclus, der die Heteroatome enthält und wahlweise wie zuvor hinsichtlich dem Heterocycloalkyl definiert substituiert ist, und
- Heteroaralkyl einen Alkylrest mit 1 bis 10 Kohlenstoffatomen, in welchem ein Wasserstoffatom durch einen Heteroarylrest ersetzt ist,
bedeutet.

2. Verwendung nach Anspruch 1, worin R einen Aralkoxycarbonyl- und Heteroaroylrest und R¹ einen Alkylrest und eine Aminosäureseitenkette bedeutet, die aus der Gruppe ausgewählt ist, die aus Asparagin, Valin, Threonin, Allothreonin, Isoleucin, S-Methylcystein und deren Sulfon- und Sulfoxidderivaten, Alanin und Alloisoleucin besteht, und R³, R⁴ und R⁵ unabhängig voneinander einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- und Heteroaralkylrest bedeuteten.

3. Verwendung nach Anspruch 1, worin R einen Carbobenzoxy-, 2-Benzofurancarbonylund 2-Chinolinylcarbonylrest, R¹ einen Methyl-, *tert*.-Butyl-, Isopropyl- und *sek*.-Butylrest und eine Aminosäureseitenkette, die aus der Gruppe ausgewählt ist, die aus einer Asparagin-, Valin-, S-Methylcystein-, Alloisoleucin-, Isoleucin-, Threonin-, Serin-, Asparaginsäure-, β-Cyanoalanin- und Allothreoninseitenkette besteht, und R² einen CH₃SCH₂CH₂-, Iso-Butyl-, n-Butyl-, Benzyl-, 4-Fluorbenzyl-, 2-Naphthylmethyl- und Cyclohexylmethylrest bedeutet und R³, R⁴ und R⁵ unabhängig voneinander einen Alkylrest mit etwa 2 bis etwa 5 Kohlenstoffatomen, einen Cycloalkylalkyl-, Aralkyl-, Heterocycloalkylalkyl- und Heteroaralkylrest bedeuten.

4. Verwendung nach Anspruch 1, worin t 0 bedeutet.

5. Verwendung nach Anspruch 1, worin t 1 bedeutet.

6. Verwendung nach Anspruch 3, worin R³ einen Benzyl-, p-Fluorbenzyl-, p-Methoxybenzyl-, p-Methylbenzyl- und 2-Naphthylmethylrest und R⁴ einen *tert*.-Butylrest bedeutet.

7. Verwendung nach Anspruch 1, wobei R⁴ und R⁵ zusammen mit dem Stickstoffatom, an welches sie gebunden sind, einen 5- oder 6-gliedrigen Heterocyclusrest bedeuten, der wahlweise mit einem Alkylrest mit 1 bis etwa 3 Kohlenstoffatomen substituiert ist.

8. Verwendung nach Anspruch 7, worin R⁴ und R⁵ zusammen mit dem Stickstoffatom, an welches sie gebunden sind, einen Pyrrolidinyl-, Piperidinyl-, Morpholinyl- und Piperazinylrest bedeuten.

9. Verwendung nach Anspruch 1, worin die Retrovirusprotease HIV-Protease ist.

10. Verwendung einer Verbindung mit der Formel 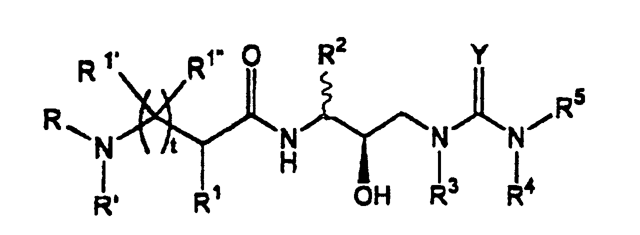 für die Herstellung eines Arzneimittels zur Behandlung einer Retrovirusinfektion, worin:
- R Wasserstoff, einen Alkoxycarbonyl-, Aralkoxycarbonyl-, Alkylcarbonyl-, Cycloalkylcarbonyl-, Cycloalkylalkoxycarbonyl-, Cycloalkylalkanoyl-, Alkanoyl-, Aralkanoyl-, Aroyl-, Aryloxycarbonyl-, Aryloxyalkanoyl-, Heterocyclocarbonyl-, Heterocyclyloxycarbonyl-, Heterocyclylalkanoyl-, Heterocyclylalkoxycarbonyl-, Heteroaralkoxycarbonyl-, Heteroaryloxycarbonyl-, Heteroaroyl-, Alkyl-, Aryl-, Aralkyl-, Aryloxyalkyl-, Heteroaryloxyalkyl-, Hydroxyalkyl-, Alkylaminocarbonyl-, Arylaminocarbonyl-, Aralkylaminoalkylcarbonyl- und Aminoalkanoylrest, Alkylaminoalkylcarbonyl- und einen mono- und disubstituierten Aminoalkanoylrest, worin die Substituenten aus einem Alkyl-, Aryl-, Aralkyl-, Cycloalkyl-, Cycloalkylalkyl-, Heteroaryl-, Heteroaralkyl-, Heteroalkyl- und Heterocycloalkylalkylrest ausgewählt sind, bedeutet,
- R' Wasserstoff und einen wie für R³ definierten Rest bedeutet oder R und R' zusammen mit dem Stickstoff, an welchem sie befestigt sind, einen Heterocycloalkyl- oder Heteroarylrest bilden,
- R¹ -CH₂SO₂NH₂, Wasserstoff, -CO₂CH₃, -CONH₂, einen Alkyl- und Cycloalkylrest und eine Aminosäureseitenkette bedeutet, die aus der Gruppe ausgewählt ist, die aus einer Asparagin, S-Methylcystein- und deren Sulfoxid-(SO-) und Sulfon-(SO₂-)Derivaten, Histidin-, Norleucin-, Glutamin-, Glycin-, Alloisoleucin-, Alanin-, Threonin-, Isoleucin-, Leucin-, *tert.*-Leucin-, Phenylalanin-, Ornithin-, Allothreonin-, Serin-, Asparaginsäure-, β-Cyanoalanin- und Valinseitenkette besteht,
- R^{1'} und R^{1"} unabhängig voneinander Wasserstoff und einen wie für R' definierten Rest bedeuten,
- R² einen Alkyl-, Aryl-, Cycloalkyl-, Cycloalkylalkyl- und Aralkylrest, wobei die Reste wahlweise mit einer Gruppe substituiert sind, die aus Halogenresten und -NO₂, -OR⁹ und -SR⁹ ausgewählt ist, worin R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl-, Heteroaryl- und Heteroaralkylrest bedeutet und
- R⁴ und R⁵ unabhängig voneinander Wasserstoff und einen wie für R³ definierten Rest bedeuten oder zusammen mit dem Stickstoffatom, an welches sie gebunden sind, einen Heterocycloalkyl- oder Heteroarylrest bilden,
- t 0 oder 1 und
- Y O oder S bedeutet und
- Alkyl einen Alkylrest mit 1 bis 10 Kohlenstoffatomen,
- Cycloalkyl einen Cycloalkylrest mit 3 bis 8 Kohlenstoffatomen,
- Aralkyl einen Alkylrest mit 1 bis 10 Kohlenstoffatomen, worin ein Wasserstoff durch einen Arylrest ersetzt ist,
- Aryl einen Phenyl- oder Naphthylrest, der wahlweise einen oder mehrere Substituenten trägt, die aus Alkyl, Alkoxy, Halogen, Hydroxy, Amino und Nitro ausgewählt sind,
- Heterocycloalkyl einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, und welcher wahlweise an einem oder mehreren Kohlenstoffatomen mit Halogen, Alkyl, Alkoxy oder Oxo und/oder an einem sekundären Stickstoffatom mit Alkyl, Aralkoxycarbonyl, Alkanoyl, Phenyl oder Phenylalkyl oder an einem tertiären Stickstoffatom durch Oxido substituiert ist und welcher über ein Kohlenstoffatom befestigt ist,
- Heteroaryl einen aromatischen monocyclischen, bicyclischen oder tricyclischen Heterocyclus, der die Heteroatome enthält und wahlweise wie zuvor hinsichtlich dem Heterocycloalkyl definiert substituiert ist, und
- Heteroaralkyl einen Alkylrest mit 1 bis 10 Kohlenstoffatomen, in welchem ein Wasserstoffatom durch einen Heteroarylrest ersetzt ist,
bedeutet.

11. Verwendung nach Anspruch 10, worin R einen Aralkoxycarbonyl- und Heteroaroylrest und R¹ einen Alkylrest und eine Aminosäureseitenkette bedeutet, die aus der Gruppe ausgewählt ist, die aus Asparagin, Valin, Threonin, Allothreonin, Isoleucin, S-Methylcystein und deren Sulfon- und Sulfoxidderivaten, Alanin und Alloisoleucin besteht, und R³, R⁴ und R⁵ unabhängig voneinander einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- und Heteroaralkylrest bedeuteten.

12. Verwendung nach Anspruch 10, worin R einen Carbobenzoxy-, 2-Benzofurancarbonyl- und 2-Chinolinylcarbonylrest, R¹ einen Methyl-, *tert*.-Butyl-, Isopropyl- und *sek*.-Butylrest und eine Aminosäureseitenkette, die aus der Gruppe ausgewählt ist, die aus einer Asparagin-, Valin-, S-Methylcystein-, Alloisoleucin-, Isoleucin-, Threonin-, Serin-, Asparaginsäure-, β-Cyanoalanin- und Allothreoninseitenkette besteht, und R² einen CH₃SCH₂CH₂-, Iso-Butyl-, n-Butyl-, Benzyl-, 4-Fluorbenzyl-, 2-Naphthylmethyl- und Cyclohexylmethylrest bedeutet und R³, R⁴ und R⁵ unabhängig voneinander einen Alkylrest mit etwa 2 bis etwa 5 Kohlenstoffatomen, einen Cycloalkylalkyl-, Aralkyl-, Heterocycloalkylalkyl- und Heteroaralkylrest bedeuten.

13. Verwendung nach Anspruch 10, worin t 0 bedeutet.

14. Verwendung nach Anspruch 10, worin t 1 bedeutet.

15. Verwendung nach Anspruch 12, worin R³ einen Benzyl-, p-Fluorbenzyl-, p-Methoxybenzyl-, p-Methylbenzyl- und 2-Naphthylmethylrest und R⁴ einen *tert*.-Butylrest bedeutet.

16. Verwendung nach Anspruch 10, wobei R⁴ und R⁵ zusammen mit dem Stickstoffatom, an welches sie gebunden sind, einen 5- oder 6-gliedrigen Heterocyclusrest bedeuteten, der wahlweise mit einem Alkylrest mit 1 bis etwa 3 Kohlenstoffatomen substituiert ist.

17. Verwendung nach Anspruch 16, worin R⁴ und R⁵ zusammen mit dem Stickstoffatom, an welches sie gebunden sind, einen Pyrrolidinyl-, Piperidinyl-, Morpholinyl- und Piperazinylrest bedeuten.

18. Verwendung nach Anspruch 10, worin die Retrovirusinfektion eine HIV-Infektion ist.

19. Verwendung einer Verbindung mit der Formel: 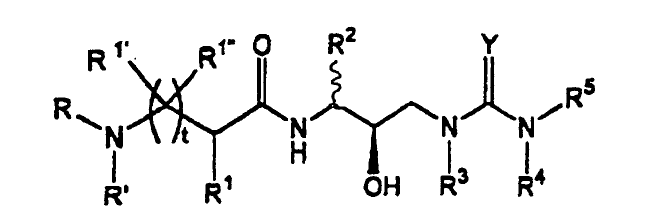 für die Herstellung eines Arzneimittels zur Behandlung von Aids, worin:
- R Wasserstoff, einen Alkoxycarbonyl-, Aralkoxycarbonyl-, Alkylcarbonyl-, Cycloalkylcarbonyl-, Cycloalkylalkoxycarbonyl-, Cycloalkylalkanoyl-, Alkanoyl-, Aralkanoyl-, Aroyl-, Aryloxycarbonyl-, Aryloxyalkanoyl-, Heterocyclocarbonyl-, Heterocyclyloxycarbonyl-, Heterocyclylalkanoyl-, Heterocyclylalkoxycarbonyl-, Heteroaralkoxycarbonyl-, Heteroaryloxycarbonyl-, Heteroaroyl-, Alkyl-, Aryl-, Aralkyl-, Aryloxyalkyl-, Heteroaryloxyalkyl-, Hydroxyalkyl-, Alkylaminocarbonyl-, Arylaminocarbonyl-, Aralkylaminoalkylcarbonyl- und Aminoalkanoylrest, Alkylaminoalkylcarbonyl- und einen mono- und disubstituierten Aminoalkanoylrest, worin die Substituenten aus einem Alkyl-, Aryl-, Aralkyl-, Cycloalkyl-, Cycloalkylalkyl-, Heteroaryl-, Heteroaralkyl-, Heteroalkyl- und Heterocycloalkylalkylrest ausgewählt sind, bedeutet,
- R' Wasserstoff und einen wie für R³ definierten Rest bedeutet oder R und R' zusammen mit dem Stickstoff, an welchem sie befestigt sind, einen Heterocycloalkyl- oder Heteroarylrest bilden,
- R' -CH₂SO₂NH₂, Wasserstoff, -CO₂CH₃, -CONH₂, einen Alkyl- und Cycloalkylrest und eine Aminosäureseitenkette bedeutet, die aus der Gruppe ausgewählt ist, die aus einer Asparagin, S-Methylcystein- und deren Sulfoxid-(SO-) und Sulfon-(SO₂-)Derivaten, Histidin-, Norleucin-, Glutamin-, Glycin-, Alloisoleucin-, Alanin-, Threonin-, Isoleucin-, Leucin-, *tert*.-Leucin-, Phenylalanin-, Ornithin-, Allothreonin-, Serin-, Asparaginsäure-, β-Cyanoalanin- und Valinseitenkette besteht,
- R^{1'} und R^{1"} unabhängig voneinander Wasserstoff und einen wie für R¹ definierten Rest bedeuten,
- R² einen Alkyl-, Aryl-, Cycloalkyl-, Cycloalkylalkyl- und Aralkylrest, wobei die Reste wahlweise mit einer Gruppe substituiert sind, die aus Halogenresten und -NO₂, -OR⁹ und -SR⁹ ausgewählt ist, worin R⁹ Wasserstoff und einen Alkylrest bedeutet, und
- R³ einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl-, Heteroaryl- und Heteroaralkylrest bedeutet und
- R⁴ und R⁵ unabhängig voneinander Wasserstoff und einen wie für R³ definierten Rest bedeuten oder zusammen mit dem Stickstoffatom, an welches sie gebunden sind, einen Heterocycloalkyl- oder Heteroarylrest bilden,
- t 0 oder 1 und
- Y O oder S bedeutet und
- Alkyl einen Alkylrest mit 1 bis 10 Kohlenstoffatomen,
- Cycloalkyl einen Cycloalkylrest mit 3 bis 8 Kohlenstoffatomen,
- Aralkyl einen Alkylrest mit 1 bis 10 Kohlenstoffatomen, worin ein Wasserstoff durch einen Arylrest ersetzt ist,
- Aryl einen Phenyl- oder Naphthylrest, der wahlweise einen oder mehrere Substituenten trägt, die aus Alkyl, Alkoxy, Halogen, Hydroxy, Amino und Nitro ausgewählt sind,
- Heterocycloalkyl einen gesättigten oder teilweise ungesättigten monocyclischen, bicyclischen oder tricyclischen Heterocyclus, der ein oder mehrere Heteroatome enthält, die aus Stickstoff, Sauerstoff und Schwefel ausgewählt sind, und welcher wahlweise an einem oder mehreren Kohlenstoffatomen mit Halogen, Alkyl, Alkoxy oder Oxo und/oder an einem sekundären Stickstoffatom mit Alkyl, Aralkoxycarbonyl, Alkanoyl, Phenyl oder Phenylalkyl oder an einem tertiären Stickstoffatom durch Oxido substituiert ist und welcher über ein Kohlenstoffatom befestigt ist,
- Heteroaryl einen aromatischen monocyclischen, bicyclischen oder tricyclischen Heterocyclus, der die Heteroatome enthält und wahlweise wie zuvor hinsichtlich dem Heterocycloalkyl definiert substituiert ist, und
- Heteroaralkyl einen Alkylrest mit 1 bis 10 Kohlenstoffatomen, in welchem ein Wasserstoffatom durch einen Heteroarylrest ersetzt ist,
bedeutet.

20. Verwendung nach Anspruch 19, worin R einen Aralkoxycarbonyl- und Heteroaroylrest und R¹ einen Alkylrest und eine Aminosäureseitenkette bedeutet, die aus der Gruppe ausgewählt ist, die aus Asparagin, Valin, Threonin, Allothreonin, Isoleucin, S Methylcystein und deren Sulfon- und Sulfoxidderivaten, Alanin und Alloisoleucin besteht, und R³, R⁴ und R⁵ unabhängig voneinander einen Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- und Heteroaralkylrest bedeuteten.

21. Verwendung nach Anspruch 19, worin R einen Carbobenzoxy-, 2-Benzofurancarbonyl- und 2-Chinolinylcarbonylrest, R¹ einen Methyl-, *tert.*-Butyl-, Isopropyl- und *sek*.-Butylrest und eine Aminosäureseitenkette, die aus der Gruppe ausgewählt ist, die aus einer Asparagin-, Valin-, S-Methylcystein-, Alloisoleucin-, Isoleucin-, Threonin-, Serin-, Asparaginsäure-, β-Cyanoalanin- und Allothreoninseitenkette besteht, und R² einen CH₃SCH₂CH₂-, Iso-Butyl-, n-Butyl-, Benzyl-, 4-Fluorbenzyl-, 2-Naphthylmethyl- und Cyclohexylmethylrest bedeutet und R³, R⁴ und R⁵ unabhängig voneinander einen Alkylrest mit etwa 2 bis etwa 5 Kohlenstoffatomen, einen Cycloalkylalkyl-, Aralkyl-, Heterocycloalkylalkyl- und Heteroaralkylrest bedeuten.

22. Verwendung nach Anspruch 19, worin t 0 bedeutet.

23. Verwendung nach Anspruch 19, worin t 1 bedeutet.

24. Verwendung nach Anspruch 21, worin R³ einen Benzyl-, p-Fluorbenzyl-, p-Methoxybenzyl-, p-Methylbenzyl- und 2-Naphthylmethylrest und R⁴ einen *tert*.-Butylrest bedeutet.

25. Verwendung nach Anspruch 19, wobei R⁴ und R⁵ zusammen mit dem Stickstoffatom, an welches sie gebunden sind, einen 5- oder 6-gliedrigen Heterocyclusrest bedeuteten, der wahlweise mit einem Alkylrest mit 1 bis etwa 3 Kohlenstoffatomen substituiert ist.

26. Verwendung nach Anspruch 25, worin R⁴ und R⁵ zusammen mit dem Stickstoffatom, an welches sie gebunden sind, einen Pyrrolidinyl-, Piperidinyl-, Morpholinyl- und Piperazinylrest bedeuten.

27. Verwendung nach einem der Ansprüche 1 bis 26, worin die Verbindung aus der Gruppe ausgewählt ist, die aus den Verbindungen in den folgenden Tabellen besteht:

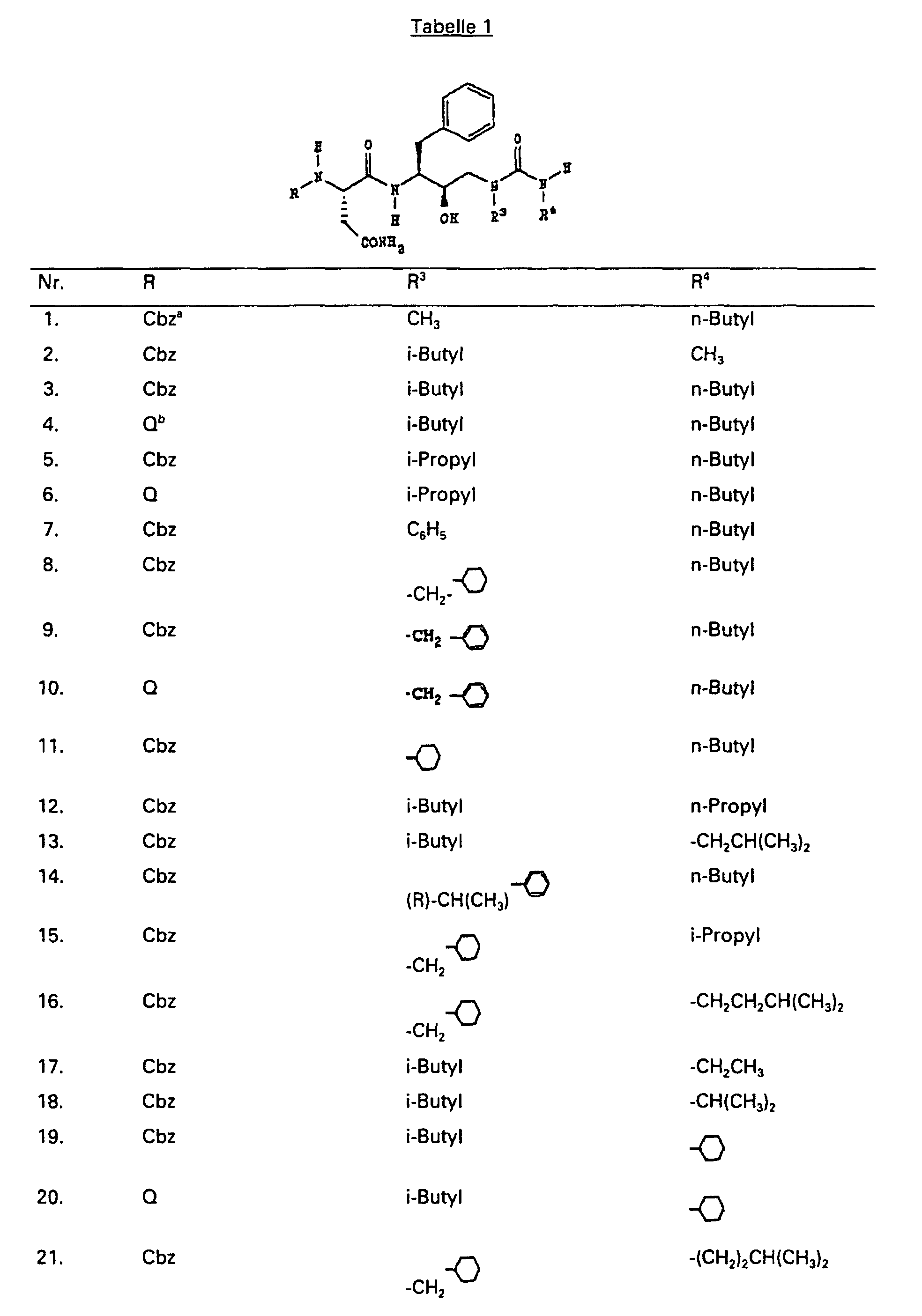

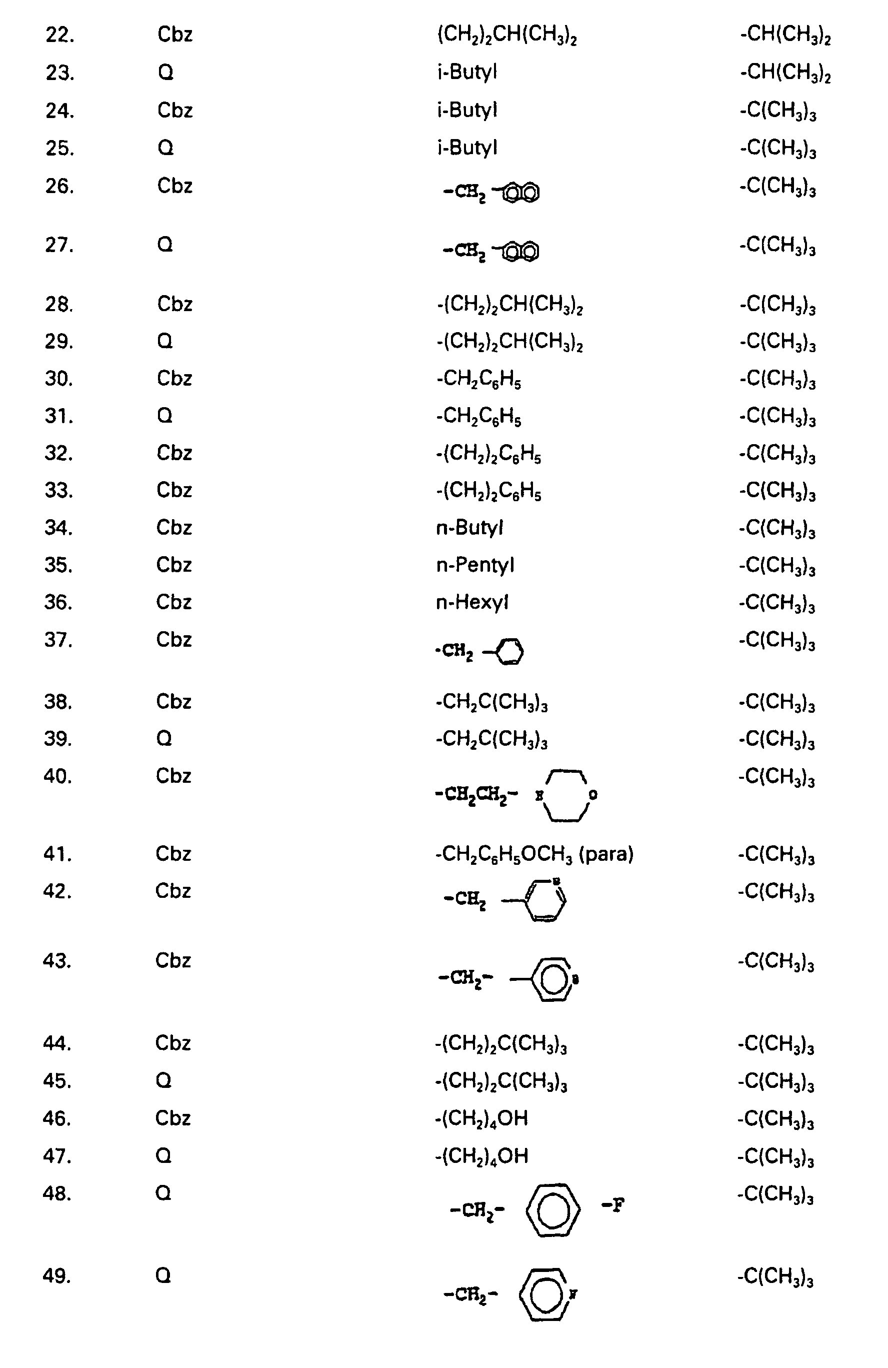

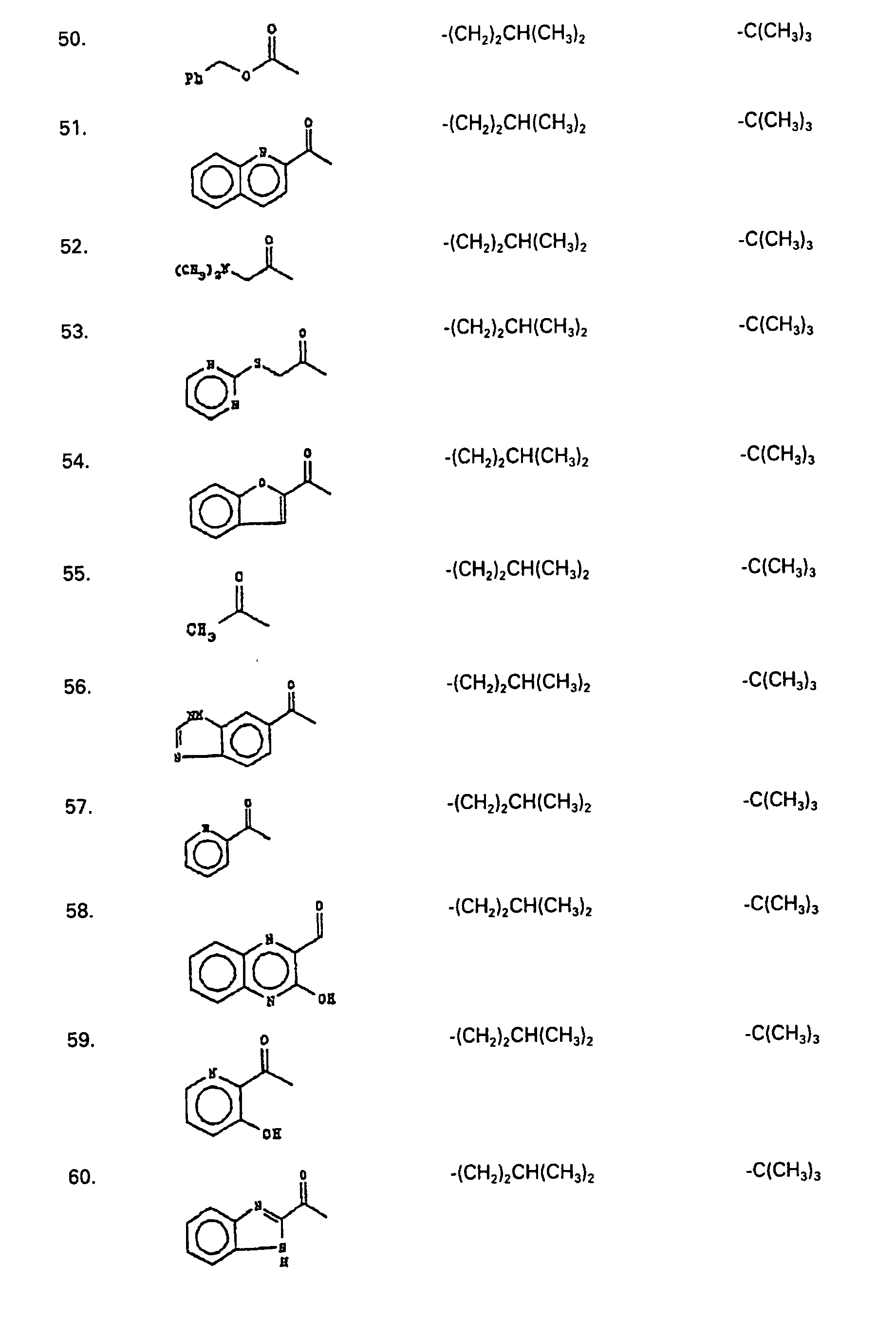

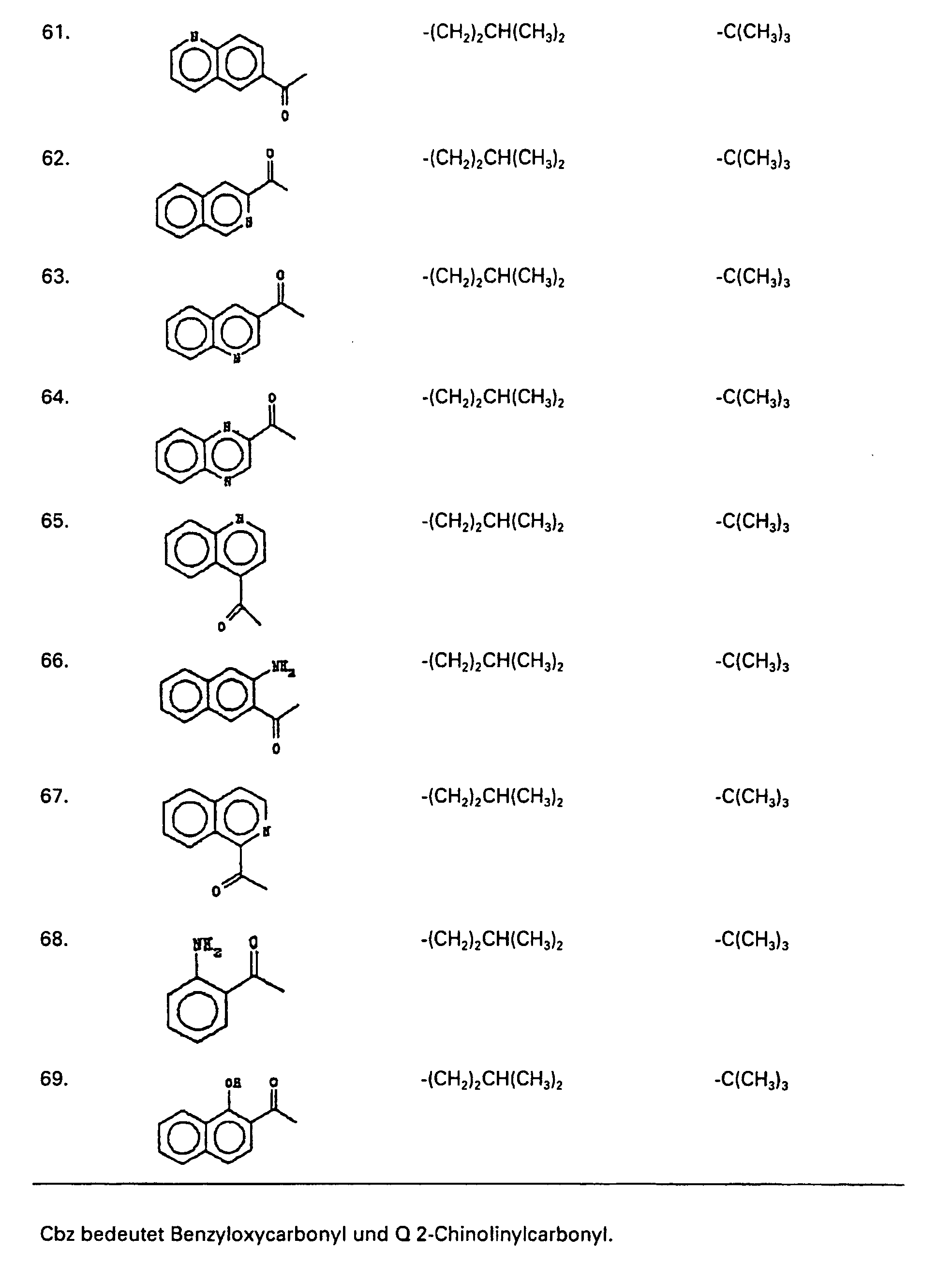

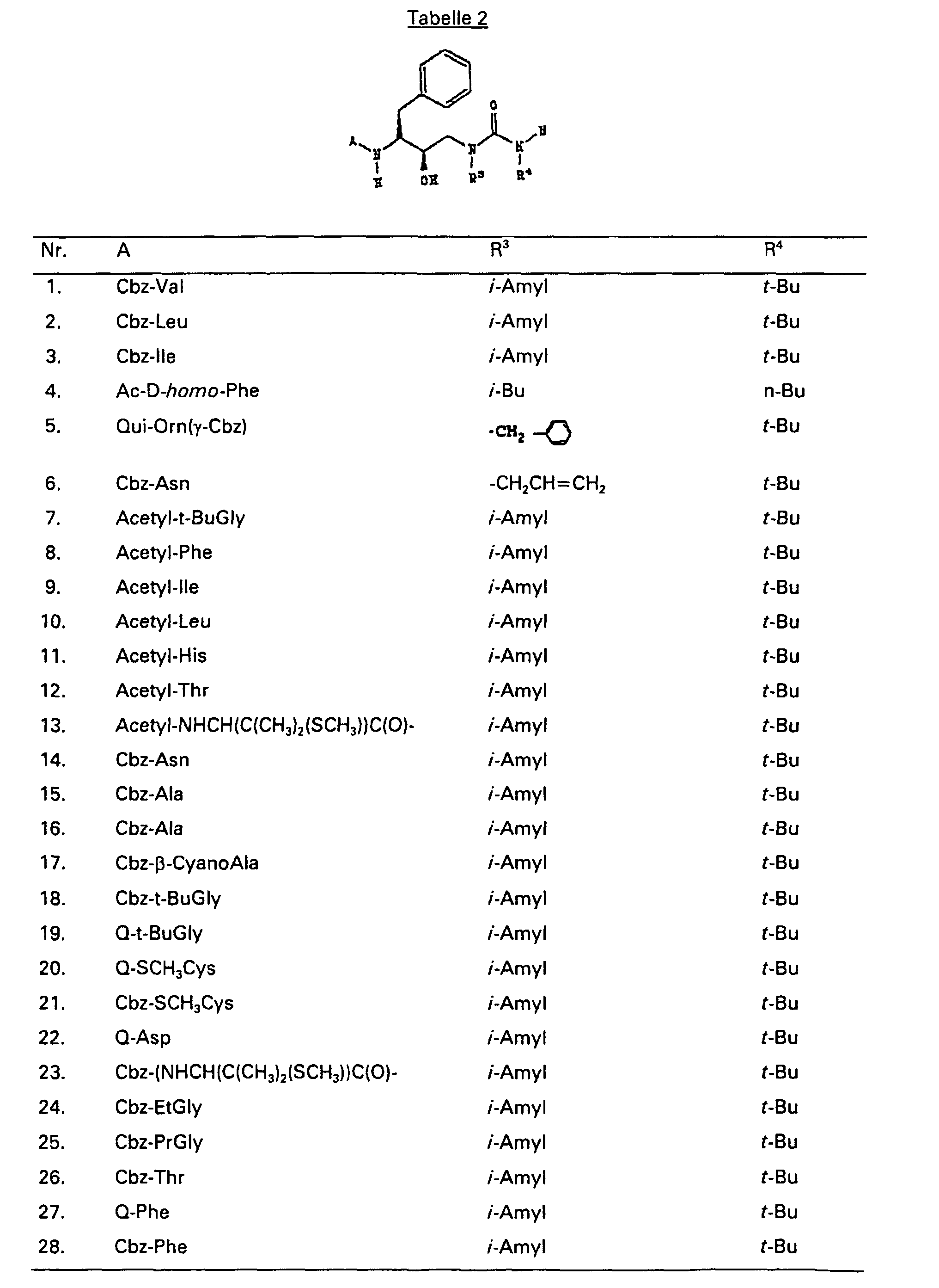

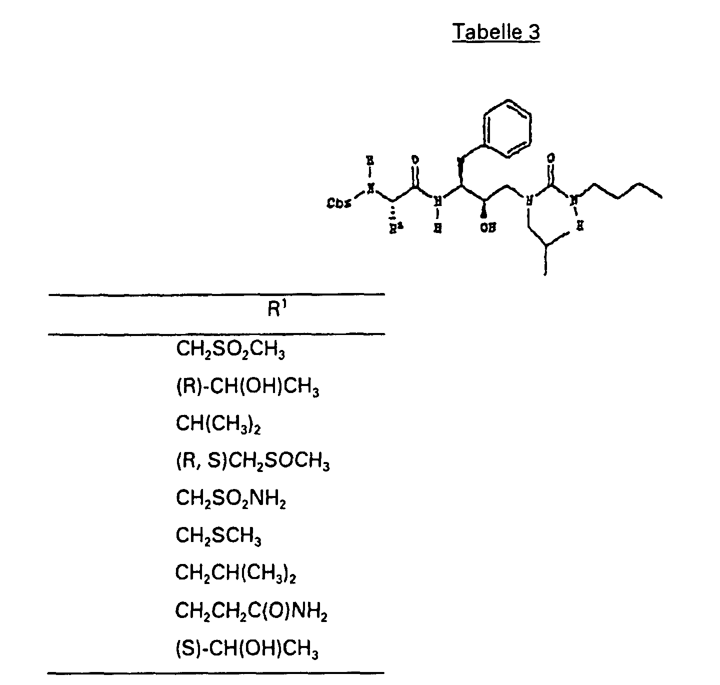

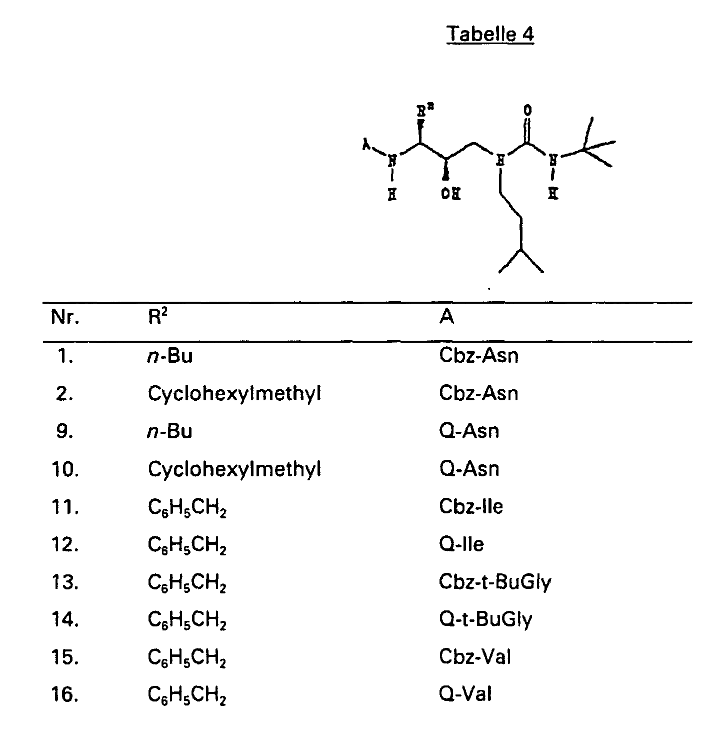

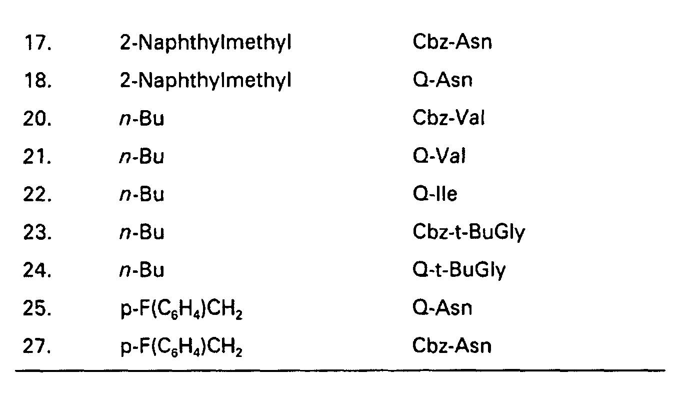

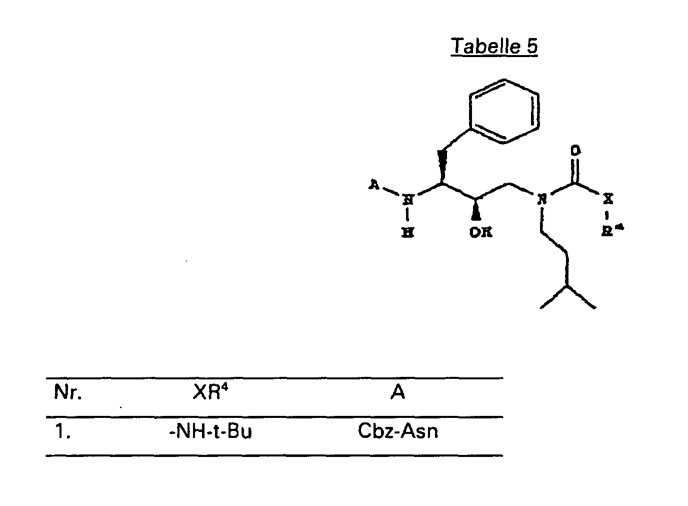

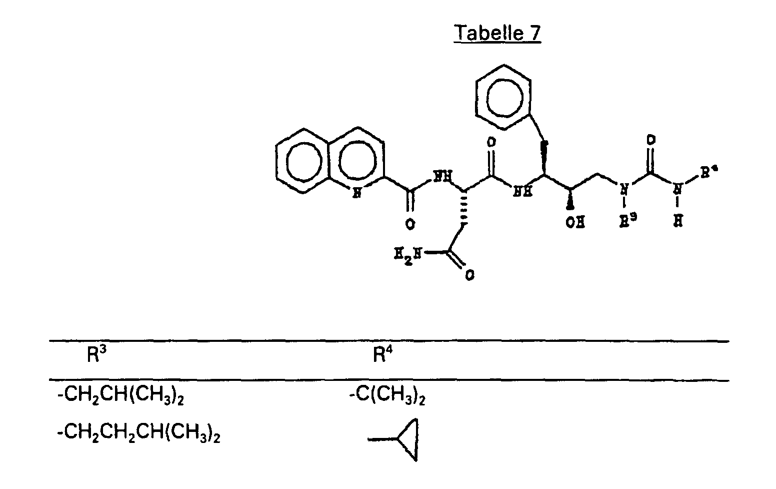

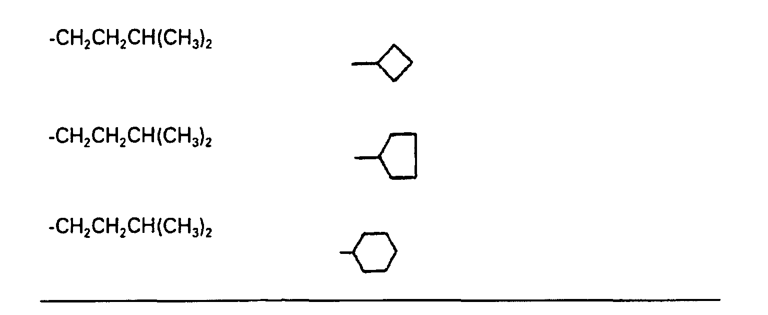

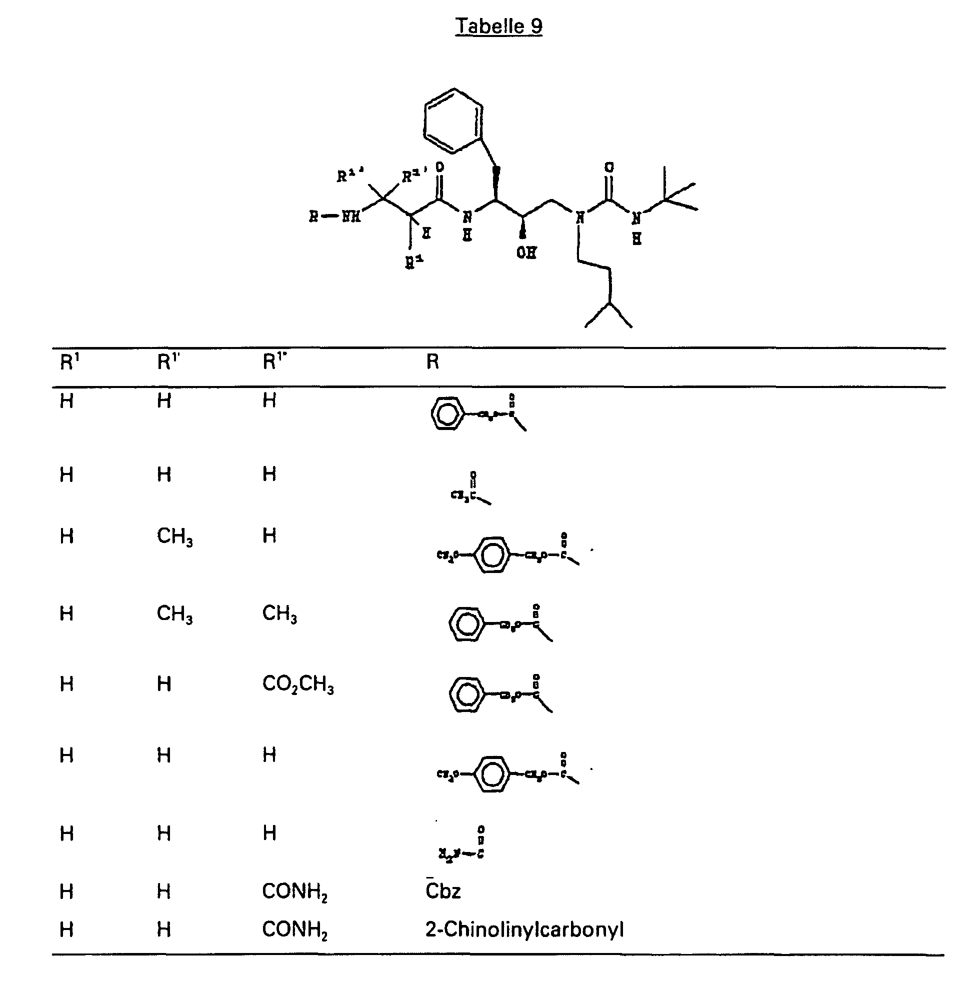

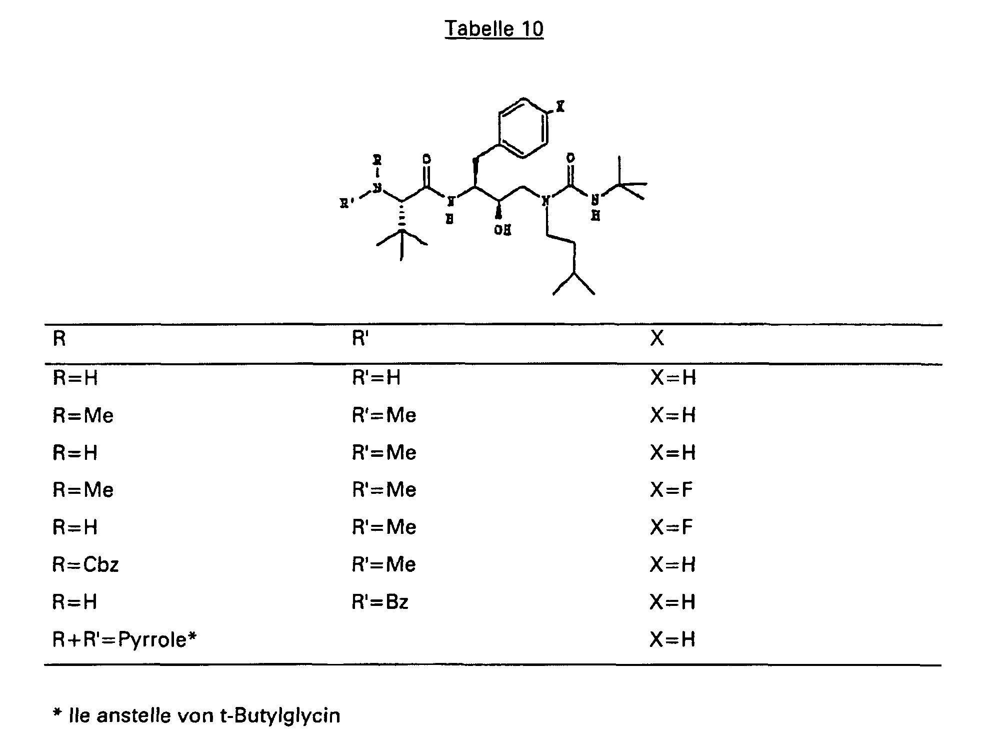

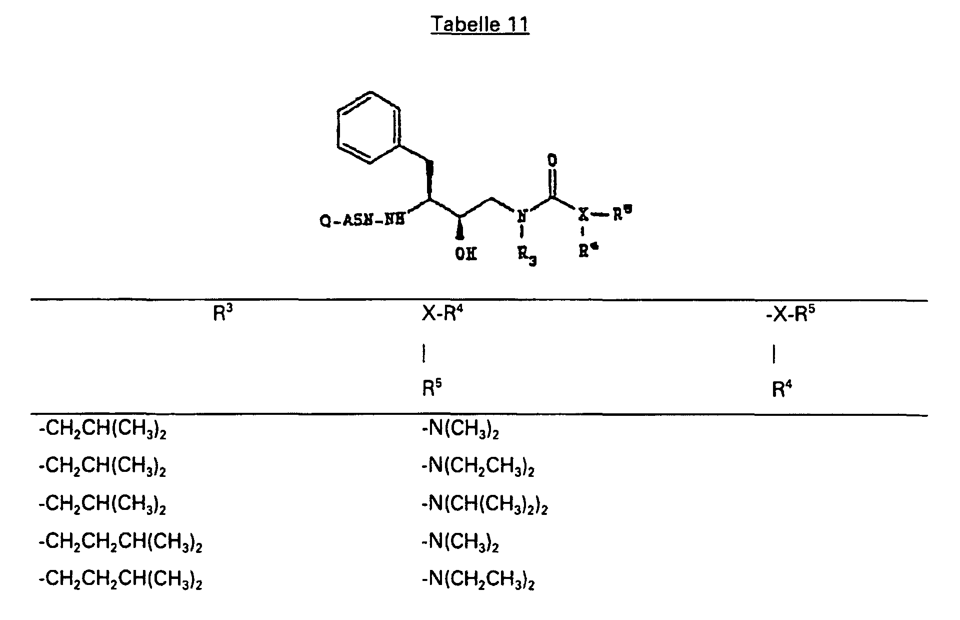

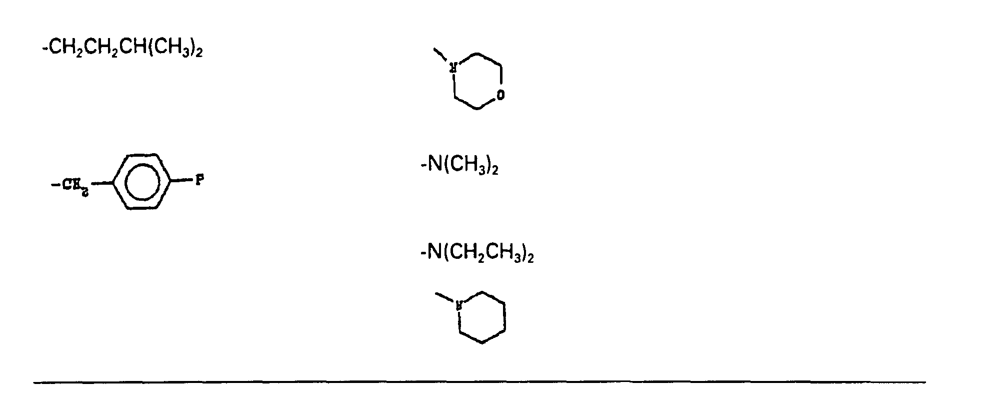

28. Verwendung nach einem der Ansprüche 1 bis 26, worin die Verbindung aus der Gruppe ausgewählt ist, die besteht aus:
[1S-[1R*(R*), 2S*]]-N¹-[3-[[[(1,1-Dimethylethyl)amino]carbonyl](2-methylpropyl)amino]-2-hydroxy-1-(phenylmethyl)propyl]-2-[(N-benzyloxycarbonyl)amino]-butandiamid
[1S-[1R*(R*), 2S*]]-N¹-[3-[[[(1,1-Dimethylethyl)amino]carbonyl](2-methylpropyl)amino]-2-hydroxy-1-(phenylmethyl)propyl]-2-[(2-chinolinylcarbonyl)amino]-butandiamid
[1S-[1R*(R*), 2S*]]-N¹-[3-[[[(1,1-Dimethylethyl)amino]carbonyl](3-methylbutyl)amino]-2-hydroxy-1-(phenylmethyl)propyl]-2-[(Nbenzyloxycarbonyl)amino]-butandiamid
[1S-[1R*(R*), 2S*]]-N¹-[3-[[[(1,1-Dimethylethyl)amino]carbonyl](3-methylbutyl)amino]-2-hydroxy-1-(phenylmethyl)propyl]-2-[(2-chinolinylcarbonyl)amino]-butandiamid
[1S-[1R*(R*), 2S*]]-N¹-[3-[[[(1,1-Dimethylethyl)amino]carbonyl](2-naphthylmethyl)amino]-2-hydroxy-1-(phenylmethyl)propyl]-2-[(Nbenzyloxycarbonyl)amino]-butandiamid
[1S-[1R*(R*), 2S*]]-N¹-[3-[[[(1,1-Dimethylethyl)amino]carbonyl](2-naphthylmethyl)amino]-2-hydroxy-1-(phenylmethyl)propyl]-2-[(2-chinolinylcarbonyl)amino]-butandiamid
[1S-[1R*(R*), 2S*]]-N¹-[3-[[[(1,1-Dimethylethyl)amino]carbonyl](2-phenylethyl)amino]-2-hydroxy-1-(phenylmethyl)propyl]-2-[(Nbenzyloxycarbonyl)amino]-butandiamid
[1S-(1R*(R*), 2S*]]-N¹-[3-[[[(1,1-Dimethylethyl)amino]carbonyl](2-phenylethyl)amino]-2-hydroxy-1-(phenylmethyl)propyl]-2-[(2-chinolinylcarbonyl)amino]-butandiamid
[1S-[1R*(R*), 2S*]]-N¹-[3-[[[(1,1-Dimethylethyl)amino]carbonyl](3,3-dimethylpropyl)amino]-2-hydroxy-1-(phenylmethyl)propyl]-2-[(Nbenryloxycarbonyl)amino]-butandiamid
[1S-[1R*(R*), 2S*]]-N¹-[3-[[[(1,1-Dimethylethyl)amino]carbonyl](3,3-dimethylpropyl)amino]-2-hydroxy-1-(phenylmethyl)propyl]-2-[(2-chinolinylcarbonyl)amino]-butandiamid
[1S-[1R*(S*), 2S*]]-3-(Acetylamino)-N-[3-[[[(1,1-dimethylethyl)amino]carbonyl](3-methylbutyl)amino]-2-hydroxy-1-(phenylmethyl)propyl]-2-methyl-propanamid
[1S-[1R*(S*), 2S*]]-3-(4-Methoxybenzyloxycarbonyl)amino)-N-[3-[[[(1,1-dimethylethyl)amino]carbonyl](3-methylbutyl)amino]-2-hydroxy-1-(phenylmethyl)propyl]-2-methyl-propanamid
[1S-[1R*(R*), 2S*]]-N¹-[2-Hydroxy-3-[(4-fluorphenylmethyl)(1-pyrrolidinylcarbonyl)amino]-1-(phenylmethyl)propyl]-2-[(2-chinolinylcarbonyl)amino]-butandiamid
[1S-[1R*(R*), 2S*]]-2-(Acetylamino)-N-[3-[[[(1,1-dimethylethyl)amino]carbonyl](3-methylbutyl)amino]-2-hydroxy-1-(phenylmethyl)propyl]-3,3-dimethyl-butanamid
[1S-[1R*(R*), 2S*]]-N¹-[3-[[[n-Butylamino]carbonyl](2-methylpropyl)amino]-2-hydroxy-1-(phenylmethyl)propyl]-2-[(phenylmethylcarbamoyl)amino]-butandiamid
[1S-[1R*(R*), 2S*]]-N¹-[3-[[[n-Butylamino]carbonyl](2-methylpropyl)amino]-2-hydroxy-1-(phenylmethyl)propyl]-2-[(2-chinolinylcarbonyl)amino]-butandiamid
[1S-[1R*(R*),2S*]]-N¹-[3-[[[1,1-Dimethylethyl)amino]-carbonyl](2-phenylethyl)amino]-2-hydroxy-1-(phenylmethyl)propyl]-2-[(2-chinolinylcarbonyl)amino]-butandiamid
[1S-[1R*(R*),2S*]]-N¹-[3-[[[1,1-Dimethylethyl)amino]-carbonyl](2-methylpropyl)amino]-2-hydroxy-1-(phenylmethyl)propyl]-2-[(2-chinolinylcarbonyl)amino]-butandiamid
[1S-[1R*(R*),2S*]]-N¹-[3-[[[1,1-Dimethylethyl)amino]carbonyl]pentylamino]-2-hydroxy-1-(phenylmethyl)propyl]-2-[(2-chinolinylcarbonyl)amino]-butandiamid
[1S-[1R*(R*),2S*]]-N¹-[3-[[[1,1-Dimethylethyl)amino]carbonyl]hexylamino]-2-hydroxy-1-(phenylmethyl)propyl]-2-[(2-chinolinylcarbonyl)amino]-butandiamid
[1S-[1R*(R*),2S*]]-N¹-[2-Hydroxy-3-[(3-methylbutyl)(1-pyrrolidinylcarbonyl)amino]-1-(phenylmethyl)propyl]-2-[(2-chinolinylcarbonyl)amino]-butandiamid
[1S-[1R*(R*),2S*]]-N¹-[2-Hydroxy-3-[(3-methylbutyl)(1-pyrrolidinylcarbonyl)amino]-1-(4-fluorphenylmethyl)propyl]-2-[(2-chinolinylcarbonyl)amino]-butandiamid
[1S-[1R*(R*),2S*]]-N¹-[2-Hydroxy-3-[(3-methylbutyl)(1-pyrrolidinylcarbonyl)amino]-1-(cyclohexylmethyl)propyl]-2-[(2-chinolinylcarbonyl)amino]-butandiamid
[1S-(1R*(R*),2S*]]-N¹-[2-Hydroxy-3-[(phenylmethyl)(4-morpholinylcarbonyl)amino]-1-(phenylmethyl)propyl]-2-[(2-chinolinylcarbonyl)amino]-butandiamid
[1S-[1R*(R*),2S*]]-N¹-[2-Hydroxy-3-[(3-methylbutyl)(1-piperidinylcarbonyl)amino]-1-(phenylmethyl)propyl]-2-[(2-chinolinylcarbonyl)amino]-butandiamid
[1S-[1R*(R*),2S*]]-N¹-[2-Hydroxy-3-[(phenylmethyl)(1-piperidinylcarbonyl)amino]-1-(phenylmethyl)propyl]-2-[(2-chinolinylcarbonyl)amino]-butandiamid
[1S-[1R*(R*),2S*]]-2-(Acetylamino)-N-[3-[[[(1,1-dimethylethyl)amino]carbonyl](3-methylbutyl)amino]-2-hydroxy-1-(cyclohexylmethyl)propyl]-3,3-dimethyl-butanamid
[1S-[1R*(R*), 2S*]]-2-(Acetylamino)-N-[3-[[[(1,1-dimethylethyl)amino]carbonyl]butylamino]-2-hydroxy-1-(phenylmethyl)propyl]-3,3-dimethyl-butanamid
[1S-[1R*(R*), 2S*]]-2-(Acetylamino)-N-[3-[[[(1,1-dimethylethyl)amino]carbonyl](phenylmethyl)amino]-2-hydroxy-1-(phenylmethyl)propyl]-3,3-dimethyl-butanamid
[1S-[1R*(R*), 2S*]]-2-(Acetylamino)-N-[3-[[[(1,1-dimethylethyl)amino]carbonyl](cyclohexylmethyl)amino]-2-hydroxy-1-(phenylmethyl)propyl]-3,3-dimethyl-butanamid
[1S-[1R*(R*), 2S*]]-2-[(2,2-Dimethylaminoacetyl)amino]-N-[3-[[[(1,1-dimethylethyl)amino]carbonyl](3-methylbutyl)amino]-2-hydroxy-1-(4-fluorphenylmethyl)propyl]-3,3-dimethyl-butanamid
[1S-[1R*(R*), 2S*]]-2-[(2,2-Dimethylaminoacetyl)amino]-N-[3-[[[(1,1-dimethylethyl)amino]carbonyl](3-methylbutyl)amino]-2-hydroxy-1-(cyclohexylmethyl)propyl]-3,3-dimethyl-butanamid
[1S-[1R*(R*), 2S*]]-2-[(2,2-Dimethylaminoacetyl)amino]-N-[3-[[[(1,1-dimethylethyl)amino]carbonyl]butylamino]-2-hydroxy-1-(phenylmethyl)propyl]-3,3-dimethyl-butanamid
[1S-[1R*(R*), 2S*]]-2-[(2,2-Dimethylaminoacetyl)amino]-N-[3-[[[(1,1-dimethylethyl)amino]carbonyl](phenylmethyl)amino]-2-hydroxy-1-(phenylmethyl)propyl]-3,3-dimethyl-butanamid
[1S-[1R*(R*), 2S*]]-2-[(2,2-Dimethylaminoacetyl)amino]-N-[3-[[[(1,1-dimethylethyl)amino]carbonyl](4-fluorphenylmethyl)amino]-2-hydroxy-1-(phenylmethyl)propyl]-3,3-dimethyl-butanamid
[1S-[1R*(R*), 2S*]]-2-[(2,2-Dimethylaminoacetyl)amino]-N-[3-[[[(1,1-dimethylethyl)amino]carbonyl](cyclohexylmethyl)amino]-2-hydroxy-1-(phenylmethyl)propyl]-3,3-dimethyl-butanamid

## Revendications

1. Utilisation d'un composé représenté par la formule : 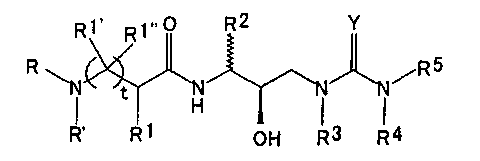 pour préparer un médicament pour inhiber une protéase rétrovirale, dans laquelle :
R représente hydrogène, des radicaux alcoxycarbonyle, aralcoxycarbonyle, alkylcarbonyle, cycloalkylcarbonyle, cycloalkylalcoxycarbonyle, cyclo-alkylalcanoyle, alcanoyle, aralkanoyle, aroyle, aryloxycarbonyle, aryloxyalcanoyle, hétérocyclo-carbonyle, hétérocyclyloxycarbonyle, hétérocyclyl-alcanoyle, hétérocyclylalcoxycarbanoyle, hétéroar-alcoxycarbonyle, hétéroaryloxycarbonyle, hétéroaroyle, alkyle, aryle, aralkyle, aryloxyalkyle, hétéroaryloxyalkyle, hydroxyalkyle, alkylaminocarbonyle, arylaminocarbonyle, aralkylaminoalkylcarbonyle et aminoalcanoyle ; des radicaux alkylaminoalkylcarbonyle et aminoalcanoyle mono- et disubstitués, où les substituants sont choisis parmi alkyle, aryle, aralkyle, cycloalkyle, cycloalkylalkyle, hétéroaryle, hétéroaralkyle, hétérocycloalkyle, hétérocycloalkyl-alkyle ;
R' représente hydrogène et des radicaux tels que définis pour R³, ou R et R' avec l'azote auquel ils sont attachés, forment un radical hétérocycloalkyle ou hétéroaryle ;
R¹ représente -CH₂SO₂NH₂, hydrogène, -CO₂CH₃, -CONH₂, des radicaux alkyle et cycloalkyle et des chaînes latérales d'acide aminé, choisi parmi l'asparagine, la S-méthylcystéine et ses dérivés sulfoxyde (SO) et sulfone (SO₂) correspondants, l'histidine, la norleucine, la glutamine, la glycine, l'alloisoleucine, l'alanine, la thréonine, l'isoleucine, la leucine, la t-leucine, la phénylalanine, l'ornithine, l'allothréonine, la sérine, l'acide aspartique, la bêta-cyanoalanine et la valine;
R^{1'} et R^{1''} représentent indépendamment, hydrogène et les radicaux définis pour R¹ ;
R² représente des radicaux alkyle, aryle, cycloalkyle, cycloalkylalkyle et aralkyle, le cas échéant substitués par un radical choisi parmi des radicaux halogène et -NO₂, -OR⁹, -SR⁹, et, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, aralkyle, hétéroaryle et hétéroaralkyle ;
R⁴ et R⁵ représentent indépendamment, hydrogène et les radicaux tels que définis pour R³, ou avec l'atome d'azote auquel ils sont liés, ils représentent un radical hétérocycloalkyle ou hétéroaryle ;
t représente 0 ou 1, et
Y représente O ou S,
et dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

2. Utilisation selon la revendication 1, où R représente des radicaux aralcoxycarbonyle et hétéroaryle ; R¹ représente des radicaux alkyle et des chaînes latérales d'acide aminé, choisi parmi le groupe consistant en l'asparagine, la valine, la thréonine, l'allothréonine, l'isoleucine, la S-méthylcystéine et ses dérivés sulfoxyde et sulfone, l'alanine et l'alloisoleucine, et R³, R⁴ et R⁵ représentent indépendamment des radicaux alkyle, alcényle, hydroxyalkyle, cycloalkyle, cycloalkylalkyle, hétérocycloalkyle, hétérocycloalkylalkyle, aryle, aralkyle, et hétéroaralkyle.

3. Utilisation selon la revendication 1, où R représente carbobenzoxy, 2-benzofurannylcarbonyle et 2-quinoléinylcarbonyle, R¹ représente les radicaux méthyle, t-butyle, isopropyle et s-butyle, et des chaînes latérales d'acide aminé, choisi parmi le groupe consistant en l'asparagine, la valine, la S-méthylcystéine, l'alloisoleucine, l'isoleucine, la thréonine, la sérine, l'acide aspartique, la bêta-cyanoalanine et l'allothréonine ; R² représente les radicaux CH₃SCH₂CH₂-, isobutyle, n-butyle, benzyle, 4-fluorobenzyle, 2-naphtylméthyle et cyclohexylméthyle ; et R³, R⁴ et R⁵ représentent indépendamment, des radicaux alkyle ayant d'environ 2 à environ 5 atomes de carbone, des radicaux cycloalkylalkyle, aralkyle, hétérocycloalkylalkyle et hétéroaralkyle.

4. Utilisation selon la revendication 1, où t est 0.

5. Utilisation selon la revendication 1, où t est 1.

6. Utilisation selon la revendication 3, où R³ représente benzyle, para-fluorobenzyle, para-méthoxybenzyle, para-méthylbenzyle et 2-naphtyl-méthyle et R⁴ représente t-butyle.

7. Utilisation selon la revendication 1, où R⁴ et R⁵ représentent avec l'azote auquel ils sont liés, un hétérocycle à 5 ou 6 membres, le cas échéant substitué avec un radical alkyle ayant de 1 à environ 3 atomes de carbone.

8. Utilisation selon la revendication 7, où R⁴ et R⁵ représentent avec l'azote auquel ils sont liés, les radicaux pyrrolidinyle, pipéridinyle, morpholinyle et pipérazinyle.

9. Utilisation selon la revendication 1, où la protéase rétrovirale est la protéase de HIV.

10. Utilisation d'un composé représenté par la formule : 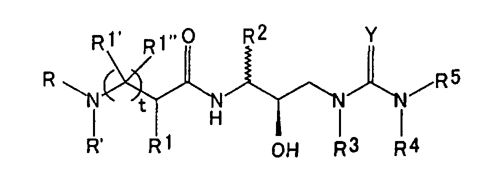 pour préparer un médicament pour traiter une infection rétrovirale, dans laquelle :
R représente hydrogène, des radicaux alcoxycarbonyle, aralcoxycarbonyle, alkylcarbonyle, cycloalkylcarbonyle, cycloalkylalcoxycarbonyle, cycloalkylalcanoyle, alcanoyle, aralkanoyle, aroyle, aryloxycarbonyle, aryloxyalcanoyle, hétérocyclocarbonyle, hétérocyclyloxycarbonyle, hétérocyclylalcanoyle, hétérocyclylalcoxycarbanoyle, hétéroaralcoxycarbonyle, hétéroaryloxycarbonyle, hétéroaroyle, alkyle, aryle, aralkyle, aryloxyalkyle, hétéroaryloxyalkyle, hydroxyalkyle, alkylaminocarbonyle, arylaminocarbonyle, aralkylaminoalkylcarbonyle et aminoalcanoyle ; des radicaux alkylaminoalkylcarbonyle et aminoalcanoyle mono- et disubstitués, où les substituants sont choisis parmi alkyle, aryle, aralkyle, cycloalkyle, cycloalkylalkyle, hétéroaryle, hétéroaralkyle, hétérocycloalkyle, hétérocycloalkylalkyle ;
R' représente hydrogène et des radicaux tels que définis pour R³, ou R et R' avec l'azote auquel ils sont attachés, forment un radical hétérocycloalkyle ou hétéroaryle ;
R¹ représente -CH₂SO₂NH₂, hydrogène, -CO₂CH₃, -CONH₂, des radicaux alkyle et cycloalkyle et des chaînes latérales d'acide aminé, choisi parmi l'asparagine, la S-méthylcystéine et ses dérivés sulfoxyde (SO) et sulfone (SO₂) correspondants, l'histidine, la norleucine, la glutamine, la glycine, l'alloisoleucine, l'alanine, la thréonine, l'isoleucine, la leucine, la t-leucine, la phénylalanine, l'ornithine, l'allothréonine, la sérine, l'acide aspartique, la bêta-cyanoalanine et la valine;
R^{1'} et R^{1"} représentent indépendamment, hydrogène et les radicaux définis pour R¹ ;
R² représente des radicaux alkyle, aryle, cycloalkyle, cycloalkylalkyle et aralkyle, le cas échéant substitués par un radical choisi parmi des radicaux halogène et -NO₂, -OR⁹, -SR⁹, et, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, aralkyle, hétéroaryle et hétéroaralkyle ;
R⁴ et R⁵ représentent indépendamment, hydrogène et les radicaux tels que définis pour R³, ou avec l'atome d'azote auquel ils sont liés, ils représentent un radical hétérocycloalkyle ou hétéroaryle ;
t représente 0 ou 1, et
Y représente O ou S,
et dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

11. Utilisation selon la revendication 10, où R représente des radicaux aralcoxycarbonyle et hétéroaryle ; R¹ représente des radicaux alkyle et des chaînes latérales d'acide aminé, choisi parmi le groupe consistant en l'asparagine, la valine, la thréonine, l'allothréonine, l'isoleucine, la S-méthylcystéine et ses dérivés sulfoxyde et sulfone, l'alanine et l'alloisoleucine, et R³, R⁴ et R⁵ représentent indépendamment des radicaux alkyle, alcényle, hydroxyalkyle, cycloalkyle, cycloalkylalkyle, hétérocycloalkyle, hétérocycloalkylalkyle, aryle, aralkyle, et hétéroaralkyle.

12. Utilisation selon la revendication 10, où R représente carbobenzoxy, 2-benzofurannylcarbonyle et 2-quinoléinylcarbonyle, R¹ représente les radicaux méthyle, t-butyle, isopropyle et s-butyle, et des chaînes latérales d'acide aminé, choisi parmi le groupe consistant en l'asparagine, la valine, la S-méthylcystéine, l'alloisoleucine, l'isoleucine, la thréonine, la sérine, l'acide aspartique, la bêta-cyanoalanine et l'allothréonine ; R² représente les radicaux CH₃SCH₂CH₂-, isobutyle, n-butyle, benzyle, 4-fluorobenzyle, 2-naphtylméthyle et cyclohexylméthyle ; et R³, R⁴ et R⁵ représentent indépendamment, des radicaux alkyle ayant d'environ 2 à environ 5 atomes de carbone, des radicaux cycloalkylalkyle, aralkyle, hétérocycloalkylalkyle et hétéroaralkyle.

13. Utilisation selon la revendication 10, où t est 0.

14. Utilisation selon la revendication 10, où t est 1.

15. Utilisation selon la revendication 12, où R³ représente benzyle, para-fluorobenzyle, para-méthoxybenzyle, para-méthylbenzyle et 2-naphtylméthyle et R⁴ représente t-butyle.

16. Utilisation selon la revendication 10, où R⁴ et R⁵ représentent avec l'atome d'azote auquel ils sont liés, un hétérocycle à 5 ou 6 membres, le cas échéant substitué avec un radical alkyle ayant de 1 à environ 3 atomes de carbone.

17. Utilisation selon la revendication 16, où R⁴ et R⁵ représentent avec l'azote auquel ils sont liés, les radicaux pyrrolidinyle, pipéridinyle, morpholinyle et pipérazinyle.

18. Utilisation selon la revendication 10, où l'infection rétrovirale est une infection de HIV.

19. Utilisation d'un composé représenté par la formule : 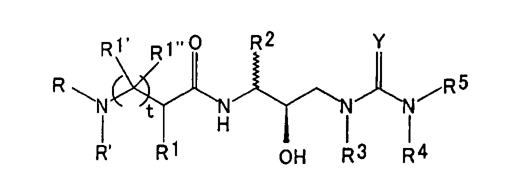 pour préparer un médicament pour traiter le sida, dans laquelle :
R représente hydrogène, des radicaux alcoxycarbonyle, aralcoxycarbonyle, alkylcarbonyle, cycloalkylcarbonyle, cycloalkylalcoxycarbonyle, cycloalkylalcanoyle, alcanoyle, aralkanoyle, aroyle, aryloxycarbonyle, aryloxyalcanoyle, hétérocyclocarbonyle, hétérocyclyloxycarbonyle, hétérocyclylalcanoyle, hétérocyclylalcoxycarbanoyle, hétéroaralcoxycarbonyle, hétéroaryloxycarbonyle, hétéroaroyle, alkyle, aryle, aralkyle, aryloxyalkyle, hétéroaryloxyalkyle, hydroxyalkyle, alkylaminocarbonyle, arylaminocarbonyle, aralkylaminoalkylcarbonyle et aminoalcanoyle ; des radicaux alkylaminoalkylcarbonyle et aminoalcanoyle mono- et disubstitués, où les substituants sont choisis parmi alkyle, aryle, aralkyle, cycloalkyle, cycloalkylalkyle, hétéroaryle, hétéroaralkyle, hétérocycloalkyle, hétérocycloalkylalkyle ;
R' représente hydrogène et des radicaux tels que définis pour R³, ou R et R' avec l'azote auquel ils sont attachés, forment un radical hétérocycloalkyle ou hétéroaryle ;
R¹ représente -CH₂SO₂NH₂, hydrogène, -CO₂CH₃, -CONH₂, des radicaux alkyle et cycloalkyle et des chaînes latérales d'acide aminé, choisi parmi l'asparagine, la S-méthylcystéine et ses dérivés sulfoxyde (SO) et sulfone (SO₂) correspondants, l'histidine, la norleucine, la glutamine, la glycine, l'alloisoleucine, l'alanine, la thréonine, l'isoleucine, la leucine, la t-leucine, la phénylalanine, l'ornithine, l'allothréonine, la sérine, l'acide aspartique, la bêta-cyanoalanine et la valine;
R^{1'} et R^{1''} représentent indépendamment, hydrogène et les radicaux définis pour R¹ ;
R² représente des radicaux alkyle, aryle, cycloalkyle, cycloalkylalkyle et aralkyle, le cas échéant substitués par un radical choisi parmi des radicaux halogène et -NO₂, -OR⁹, -SR⁹, et, où R⁹ représente hydrogène et des radicaux alkyle ;
R³ représente des radicaux alkyle, alcényle, hydroxyalkyle, cycloalkyle, cycloalkylalkyle, hétérocycloalkyle, hétérocycloalkylalkyle, aryle, aralkyle, hétéroaryle et hétéroaralkyle ;
R⁴ et R⁵ représentent indépendamment, hydrogène et les radicaux tels que définis pour R³, ou avec l'atome d'azote auquel ils sont liés, ils représentent un radical hétérocycloalkyle ou hétéroaryle ;
t représente 0 ou 1, et
Y représente O ou S,
et dans laquelle :
- alkyle signifie un alkyle ayant 1 à 10 atomes de carbone,
- cycloalkyle signifie un cycloalkyle ayant 3 à 8 atomes de carbone,
- aralkyle signifie un alkyle ayant 1 à 10 atomes de carbone, dans lequel un hydrogène est remplacé par un aryle,
- aryle est phényle ou naphtyle, qui porte le cas échéant, un ou plusieurs substituants choisis parmi alkyle, alcoxy, halogène, hydroxy, amino et nitro,
- hétérocycloalkyle est un hétérocycle saturé ou partiellement insaturé, monocyclique, bicyclique ou tricyclique, qui contient un ou plusieurs hétéroatomes choisis parmi azote, oxygène et soufre, qui est le cas échéant substitué sur un ou plusieurs atomes de carbone, par halogène, alkyle, alcoxy ou oxo, et/ou sur un atome d'azote secondaire, par alkyle, aralcoxycarbonyle, alcanoyle, phényle ou phénylalkyle ou sur un atome d'azote tertiaire, par oxydo et qui est attaché via un atome de carbone,
- hétéroaryle est un hétérocycle aromatique monocyclique, bicyclique ou tricyclique, qui contient les hétéroatomes et qui est le cas échéant, substitué tel que défini ci-dessus pour la définition de hétérocycloalkyle,
- hétéroaralkyle est un radical alkyle ayant 1 à 10 atomes de carbone, dans lequel un hydrogène est remplacé par un hétéroaryle.

20. Utilisation selon la revendication 190, où R représente des radicaux aralcoxycarbonyle et hétéroaryle ; R¹ représente des radicaux alkyle et des chaînes latérales d'acide aminé, choisi parmi le groupe consistant en l'asparagine, la valine, la thréonine, l'allothréonine, l'isoleucine, la S-méthylcystéine et ses dérivés sulfoxyde et sulfone, l'alanine et l'alloisoleucine, et R³, R⁴ et R⁵ représentent indépendamment des radicaux alkyle, alcényle, hydroxyalkyle, cycloalkyle, cycloalkylalkyle, hétérocycloalkyle, hétérocycloalkylalkyle, aryle, aralkyle, et hétéroaralkyle.

21. Utilisation selon la revendication 19, où R représente carbobenzoxy, 2-benzofurannylcarbonyle et 2-quinoléinylcarbonyle, R¹ représente les radicaux méthyle, t-butyle, isopropyle et s-butyle, et des chaînes latérales d'acide aminé, choisi parmi le groupe consistant en l'asparagine, la valine, la S-méthylcystéine, l'alloisoleucine, l'isoleucine, la thréonine, la sérine, l'acide aspartique, la bêta-cyanoalanine et l'allothréonine ; R² représente les radicaux CH₃SCH₂CH₂-, isobutyle, n-butyle, benzyle, 4-fluorobenzyle, 2-naphtylméthyle et cyclohexylméthyle ; et R³, R⁴ et R⁵ représentent indépendamment, des radicaux alkyle ayant d'environ 2 à environ 5 atomes de carbone, des radicaux cycloalkylalkyle, aralkyle, hétérocycloalkylalkyle et hétéroaralkyle.

22. Utilisation selon la revendication 19, où t est 0.

23. Utilisation selon la revendication 19, où t est 1.

24. Utilisation selon la revendication 21, où R³ représente benzyle, para-fluorobenzyle, para-méthoxybenzyle, para-méthylbenzyle et 2-naphtylméthyle et R⁴ représente t-butyle.

25. Utilisation selon la revendication 19, où R⁴ et R⁵ représentent avec l'atome d'azote auquel ils sont liés, un hétérocycle à 5 ou 6 membres, le cas échéant substitué avec un radical alkyle ayant de 1 à environ 3 atomes de carbone.

26. Utilisation selon la revendication 25, où R⁴ et R⁵ représentent avec l'azote auquel ils sont liés, les radicaux pyrrolidinyle, pipéridinyle, morpholinyle et pipérazinyle.

27. Utilisation selon l'une quelconque des revendications 1 à 26, où le composé est choisi parmi le groupe consistant en les composés des tableaux suivants :

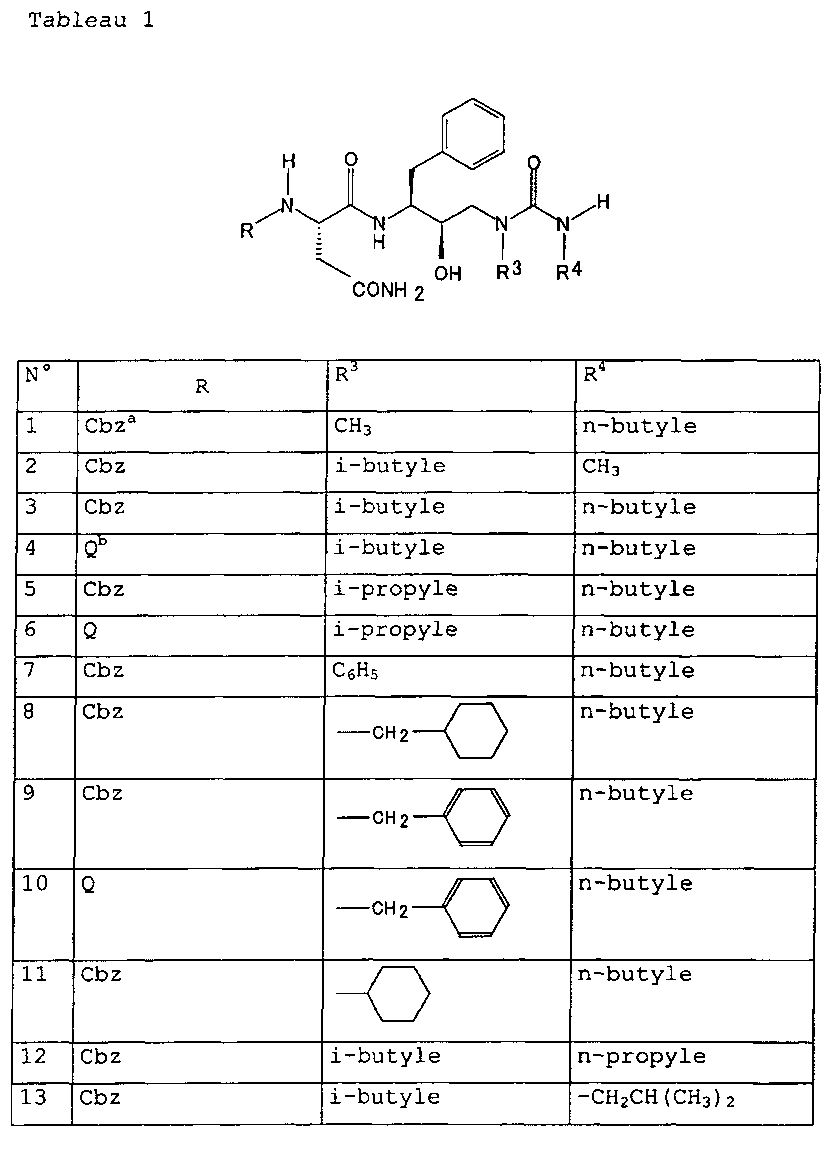

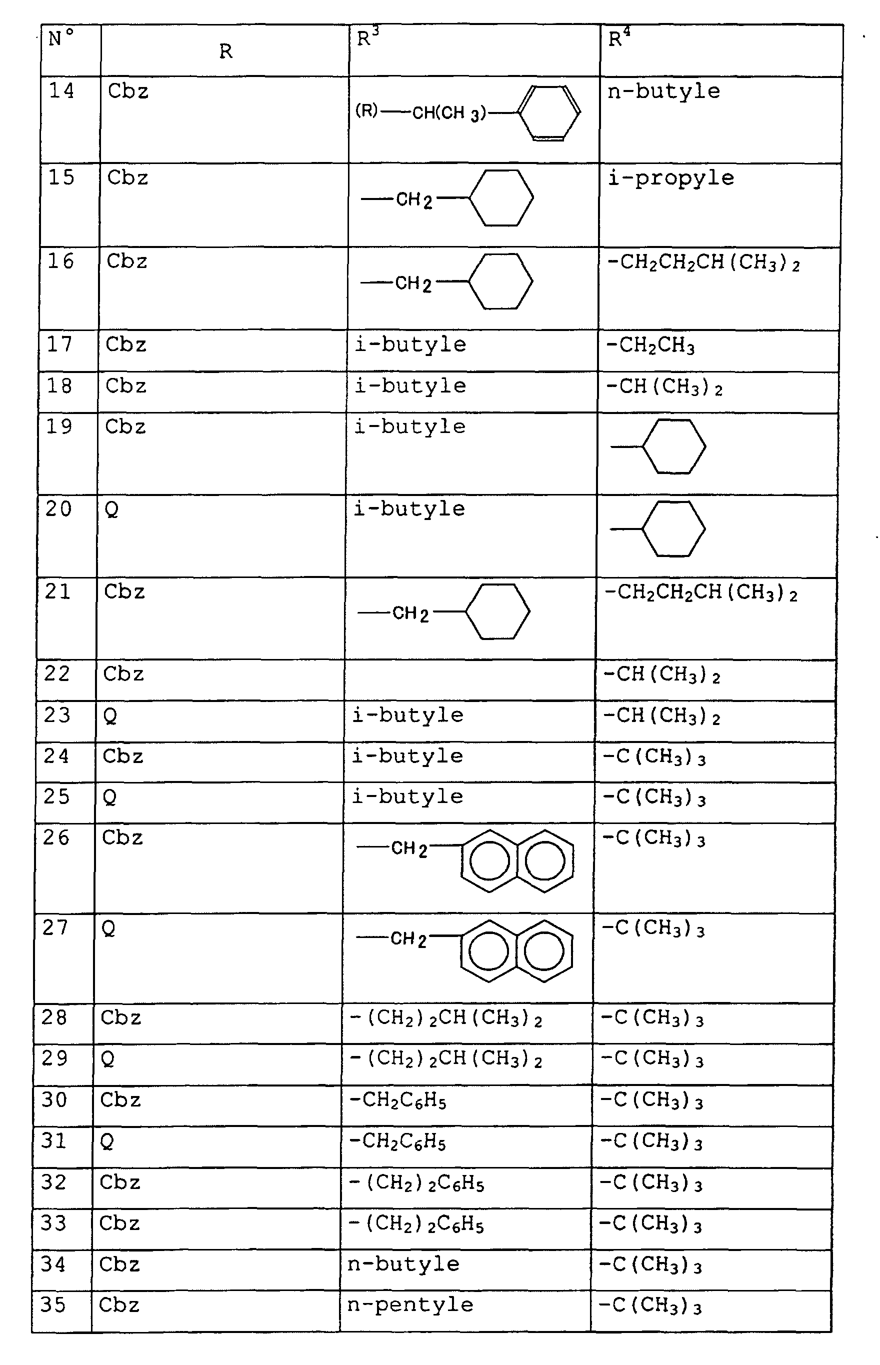

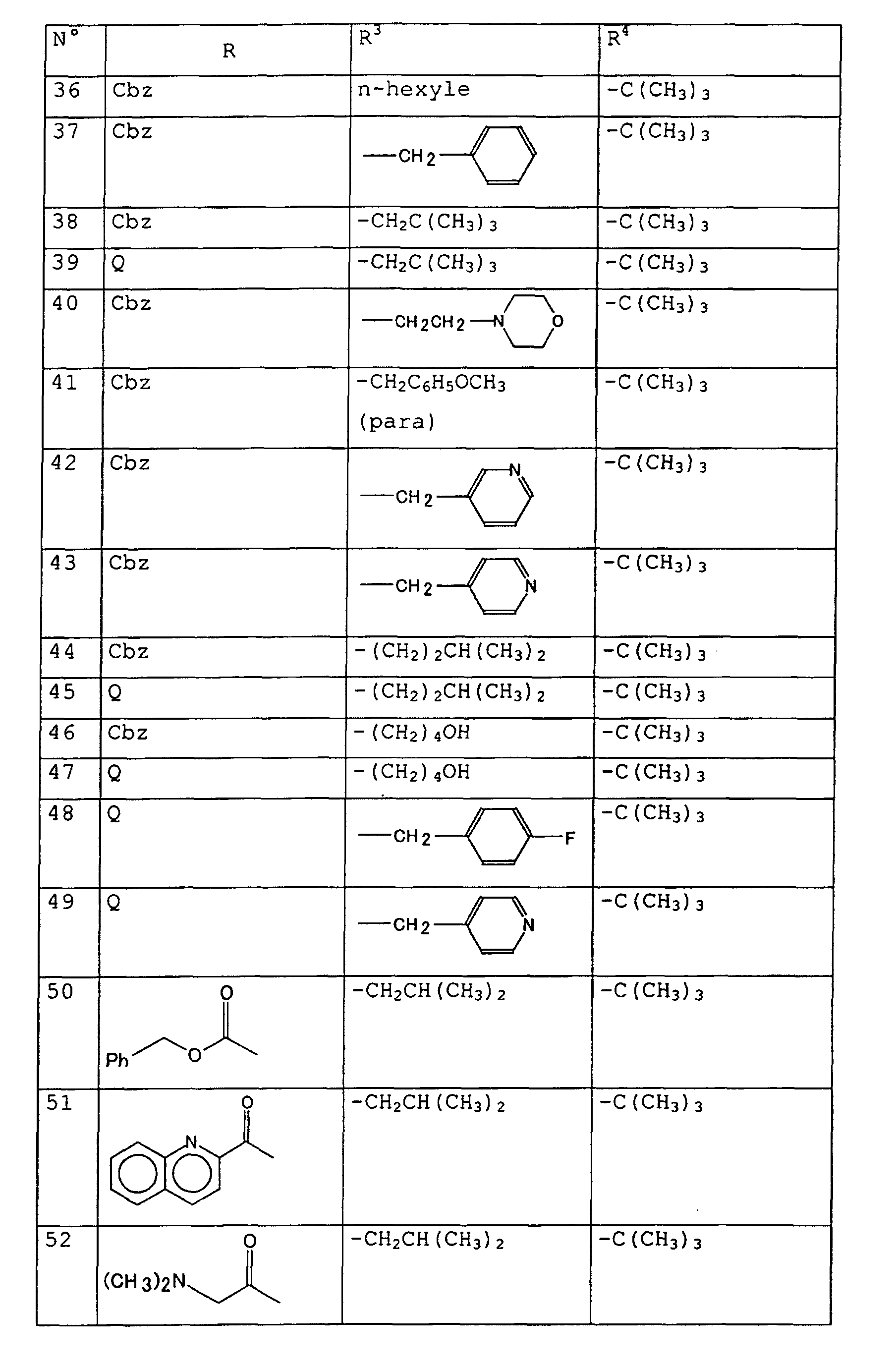

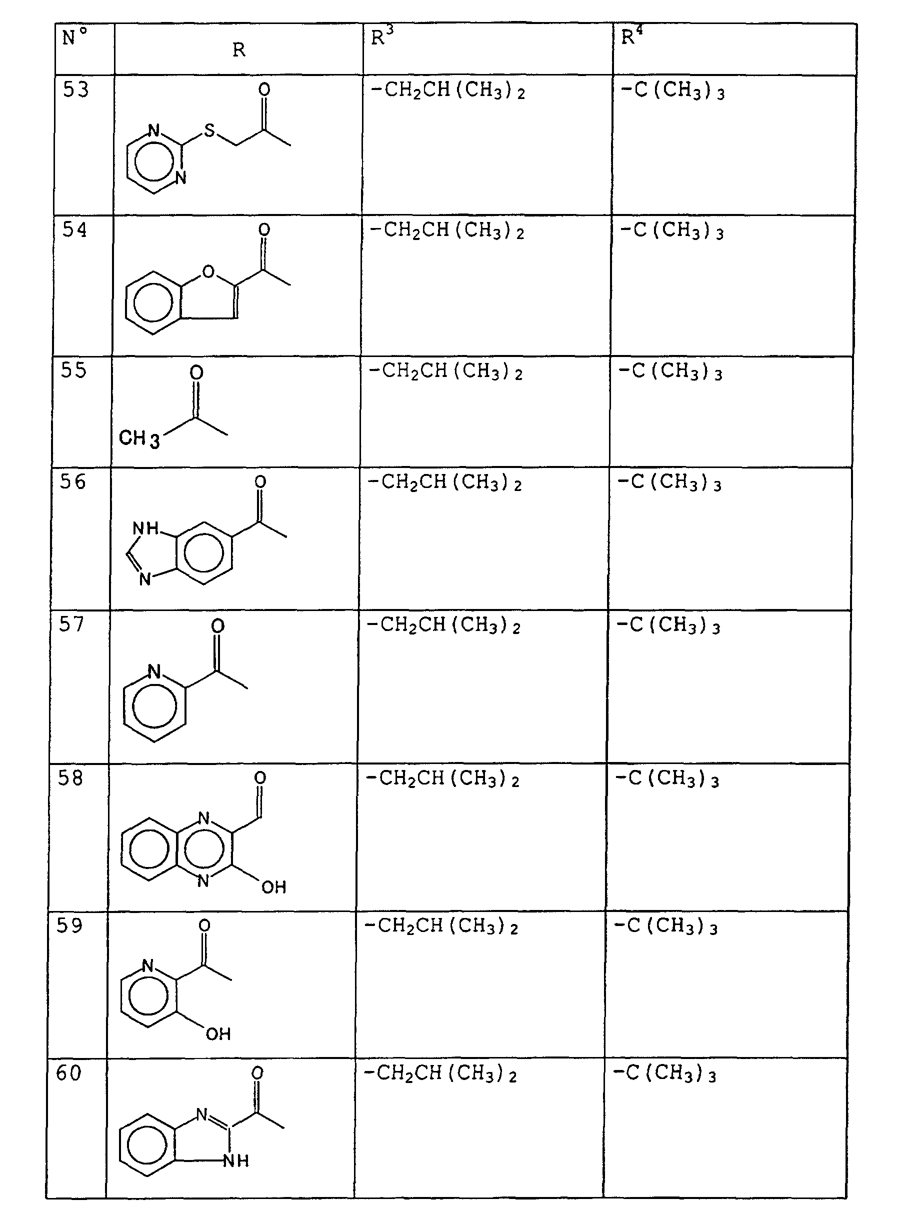

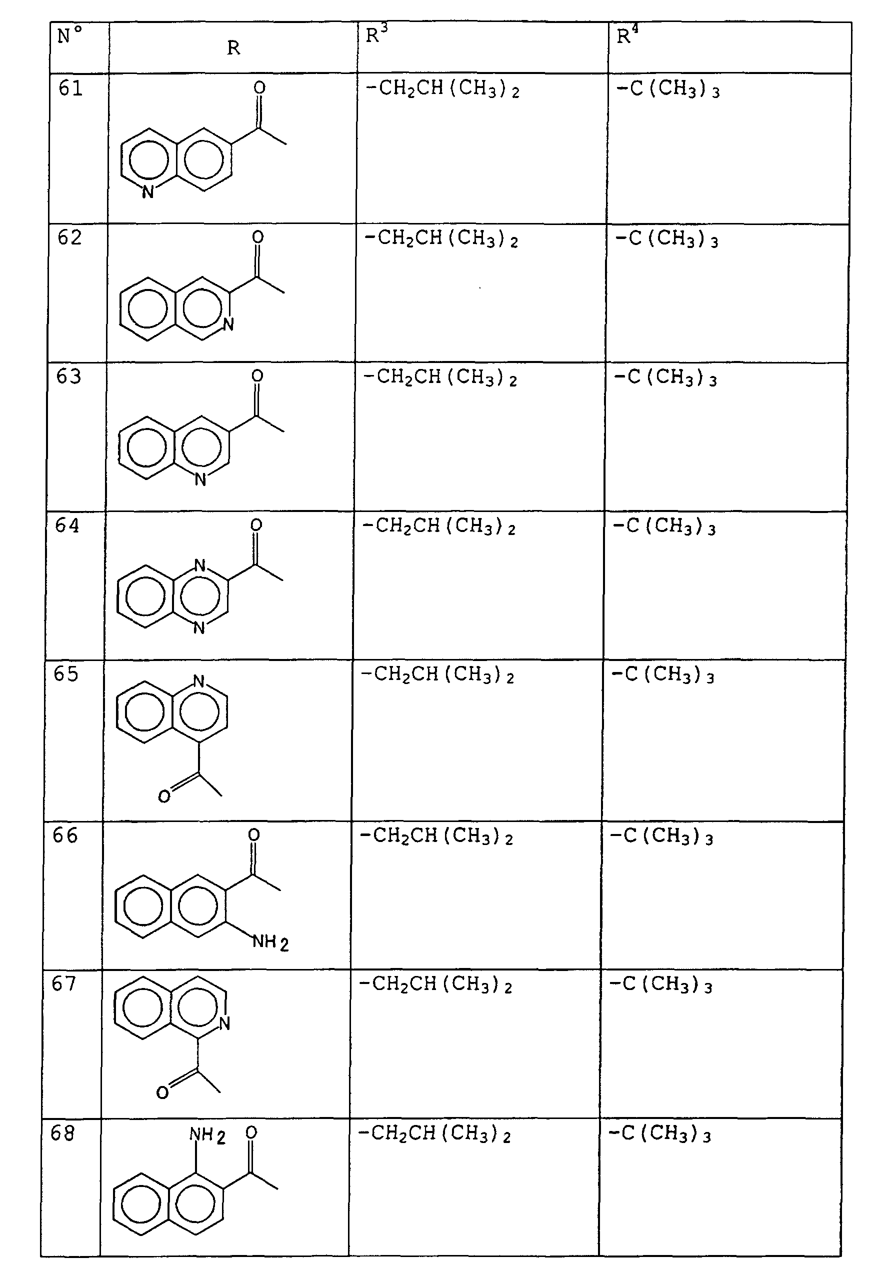

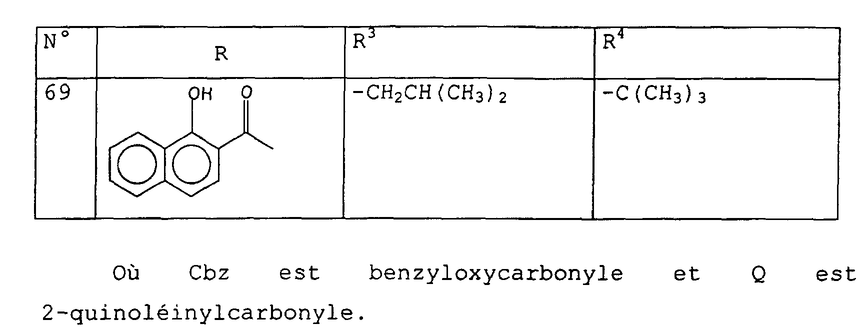

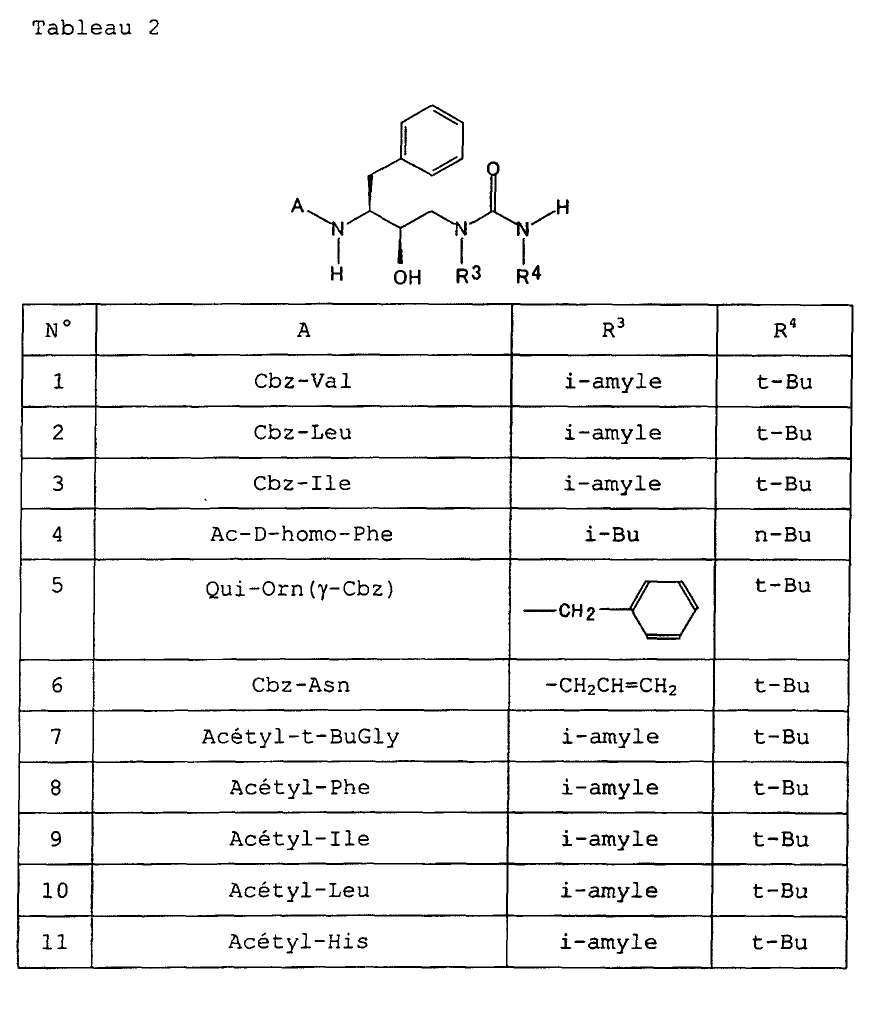

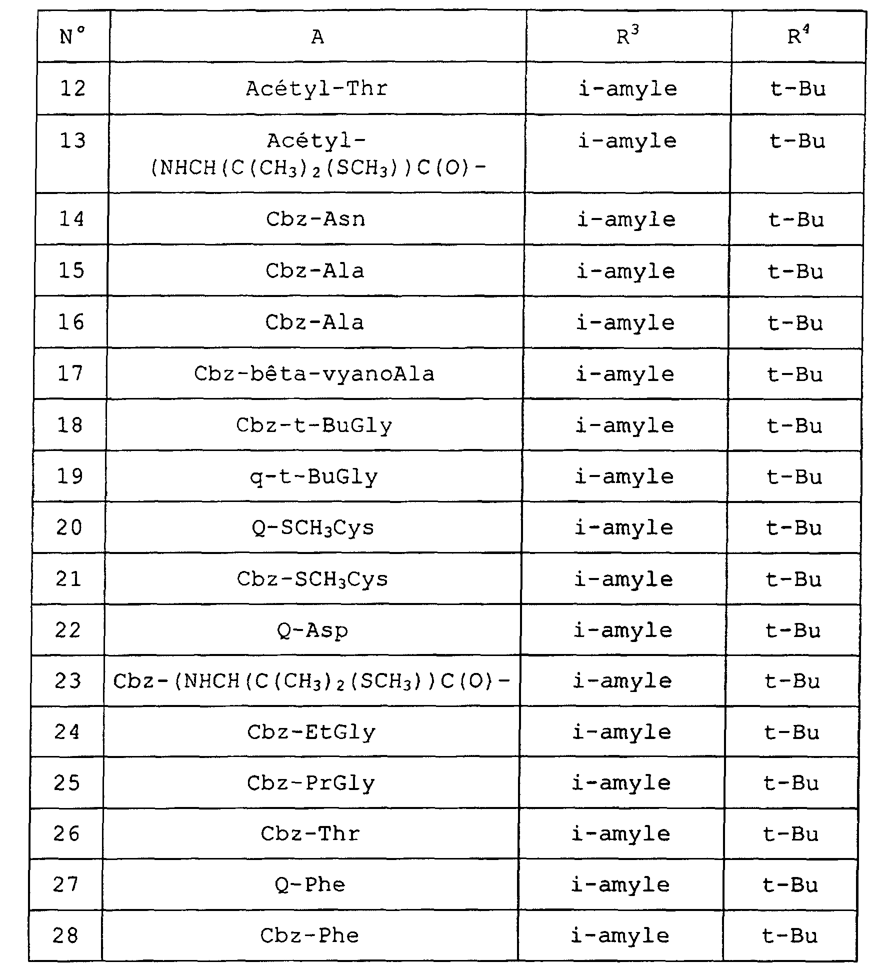

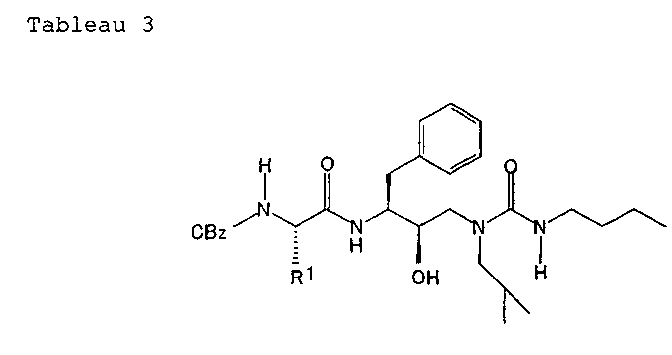

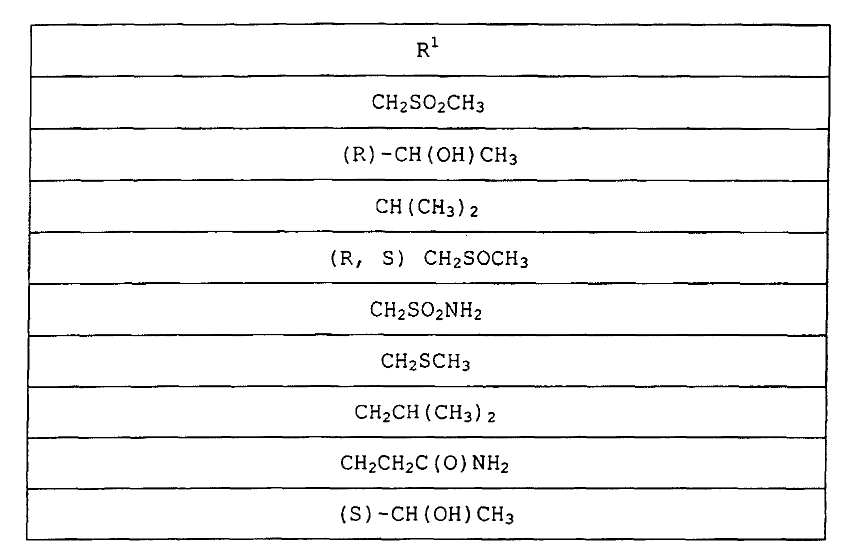

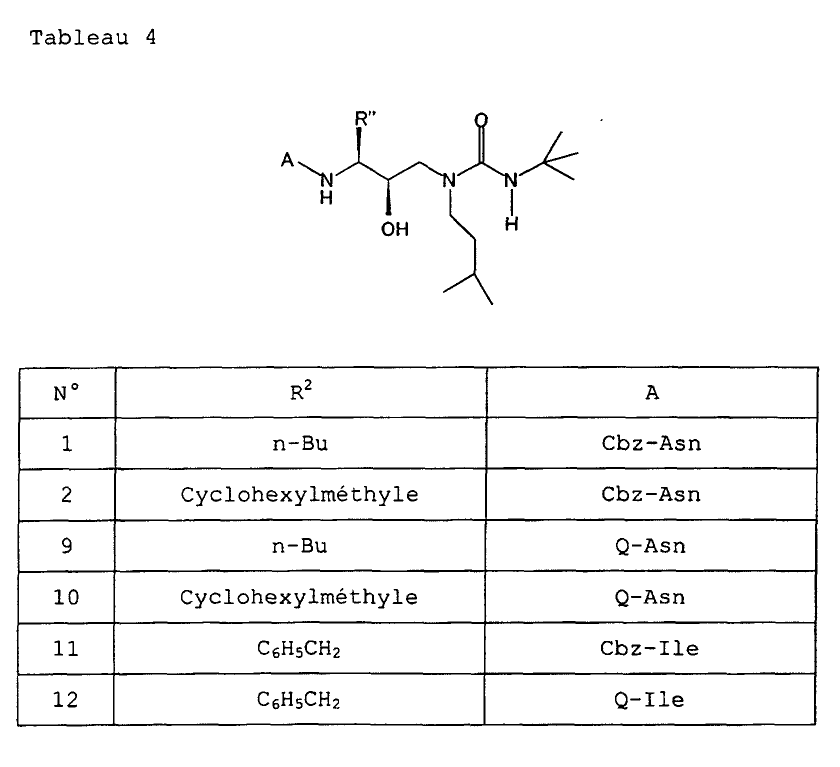

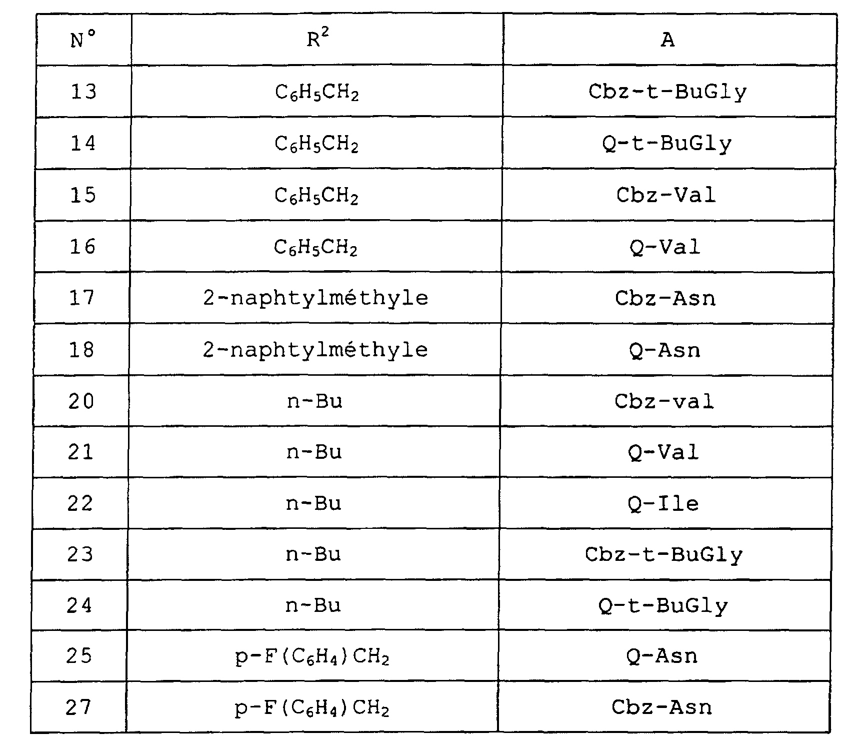

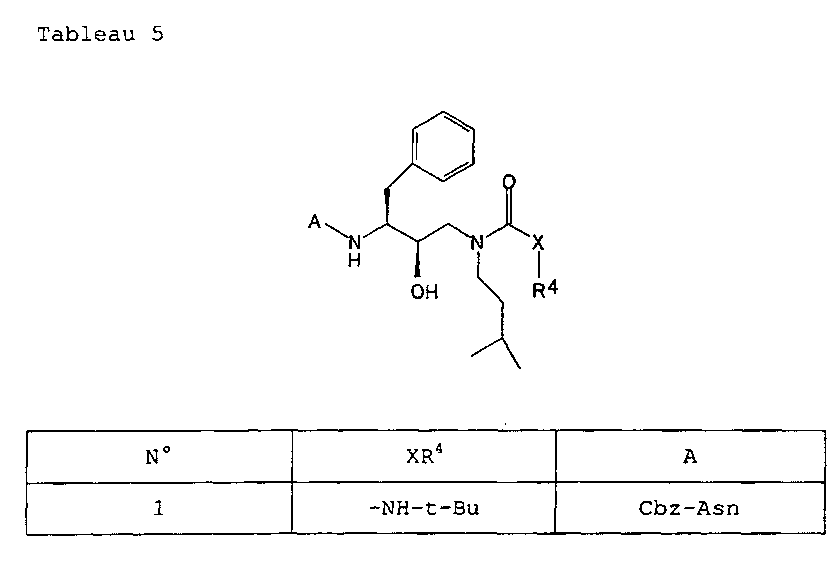

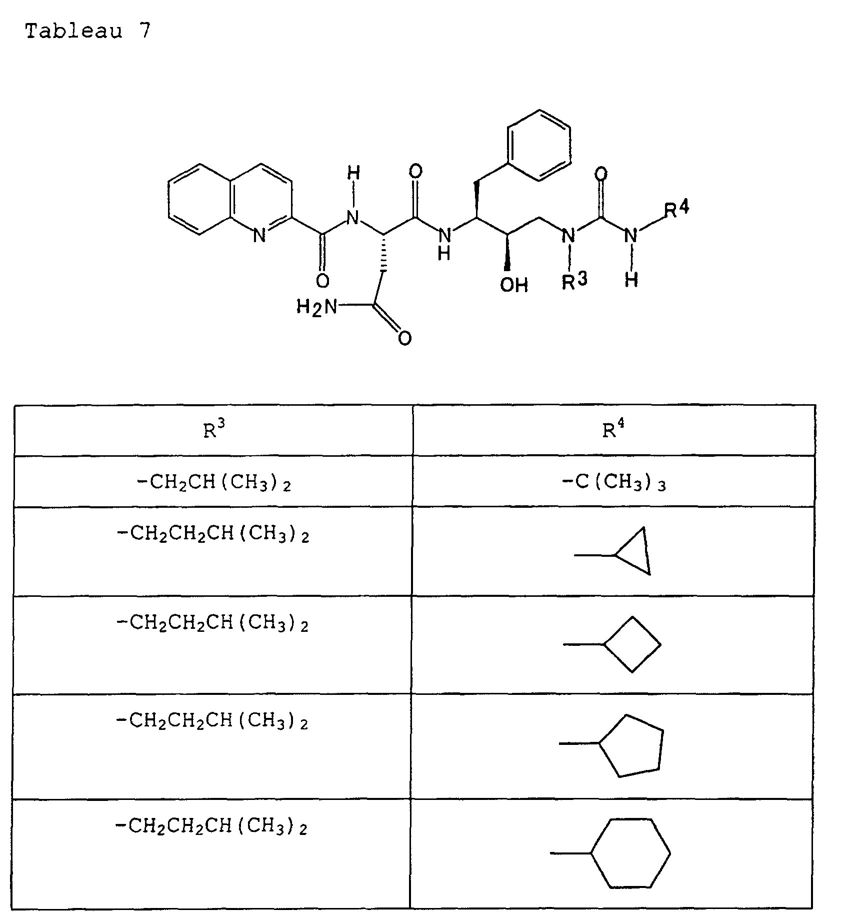

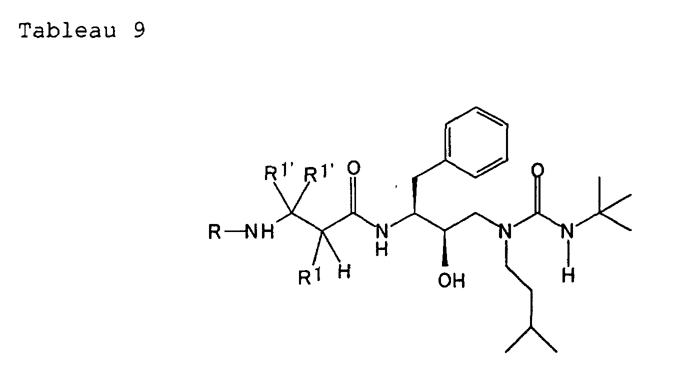

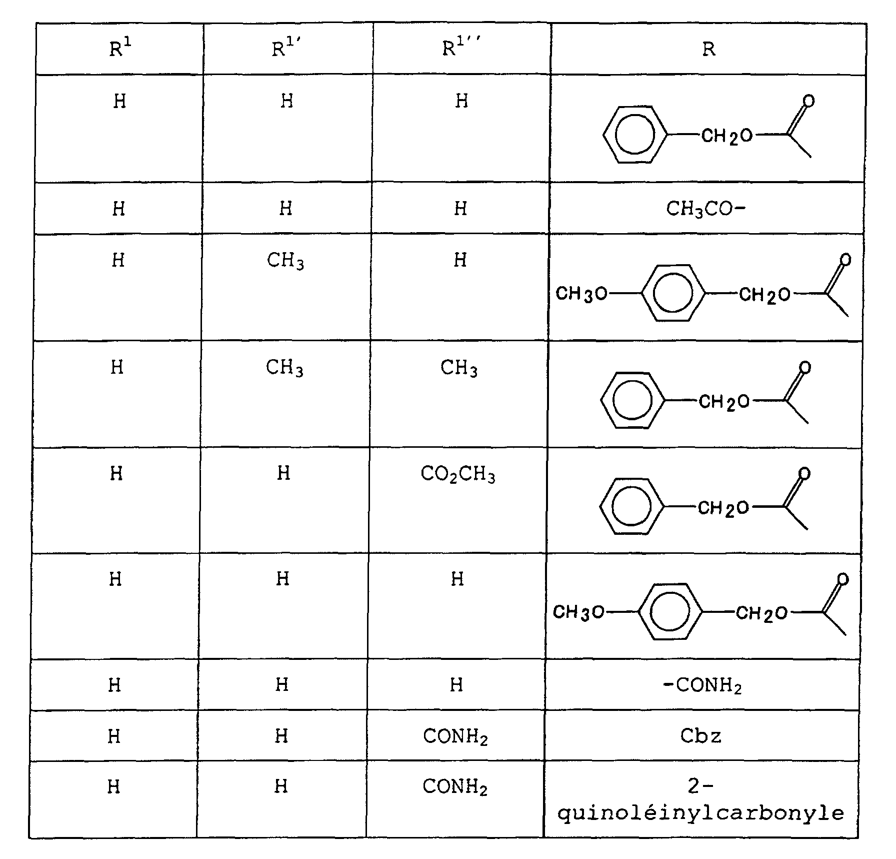

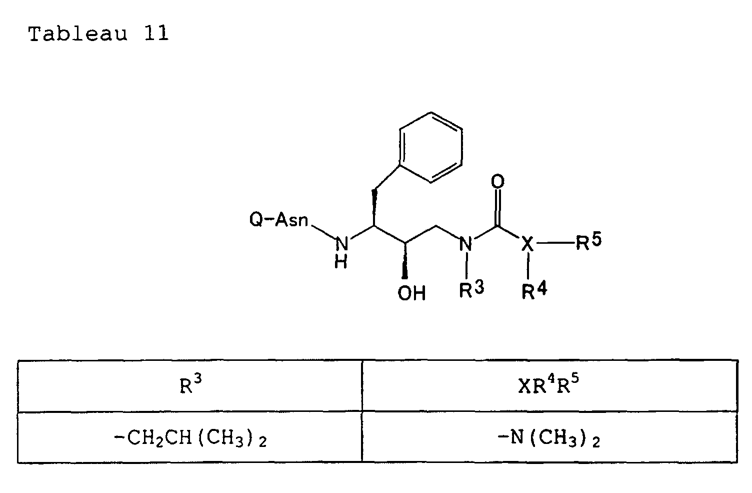

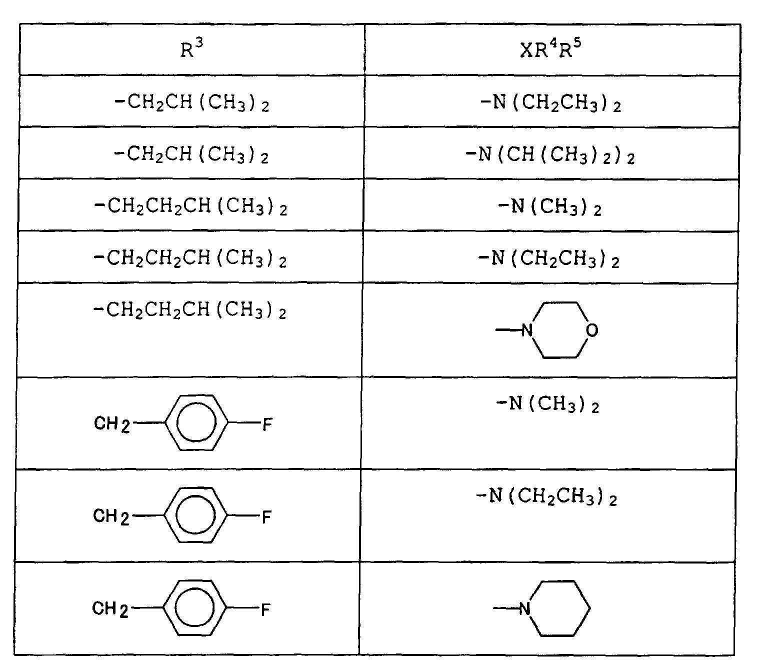

28. Utilisation selon l'une quelconque des revendications 1 à 26, où le composé est choisi parmi le groupe consistant en
le butanediamide, N¹-[3-[[[(1,1-diméthyléthyl)amino]carbonyl](2-méthylpropyl)amino]-2-hydroxy-1-(phénylméthyl)propyl]-2-[(N-benzyloxycarbonyl)amino]-, [1S-[1R*(R*),2S*]]-
le butanediamide, N¹-[3-[[[(1,1-diméthyléthyl)amino]carbonyl](2-méthylpropyl)amino]-2-hydroxy-1-(phénylméthyl)propyl]-2-[(2-quinoléinylcarbonyl)amino]-, [1S-[1R*(R*),2S*]]-
le butanediamide, N¹-[3-[[[(1,1-diméthyléthyl)amino]carbonyl](3-méthylbutyl)amino]-2-hydroxy-1-(phénylméthyl)propyl]-2-[(N-benzyloxycarbonyl)amino]-, [1S-[1R*(R*),2S*]]-
le butanediamide, N¹-[3-[[[(1,1-diméthyléthyl)amino]carbonyl](3-méthylbutyl)amino]-2-hydroxy-1-(phénylméthyl)propyl]-2-[(2-quinoléinylcarbonyl)amino]-, [1S-[1R*(R*),2S*]]-
le butanediamide, N¹-[3-[[[(1,1-diméthyléthyl)amino]carbonyl](2-naphtylméthyl)amino]-2-hydroxy-1-(phénylméthyl)propyl]-2-[(N-benzyloxycarbonyl)amino]-, [1S-[1R*(R*),2S*]]-
le butanediamide, N¹-[3-[[[(1,1-diméthyléthyl)amino]carbonyl](2-naphtylméthyl)amino]-2-hydroxy-1-(phénylméthyl)propyl]-2-[(2-quinoléinylcarbonyl)amino]-, [1S-[1R*(R*),2S*]]-
le butanediamide, N¹-[3-[[[(1,1-diméthyléthyl)amino]carbonyl](2-phényléthyl)amino]-2-hydroxy-1-(phénylméthyl)propyl]-2-[(N-benzyloxycarbonyl)amino]-, [1S-[1R*(R*),2S*]]-
le butanediamide, N¹-[3-[[[(1,1-diméthyléthyl)amino]carbonyl](2-phényléthyl)amino]-2-hydroxy-1-(phénylméthyl)propyl]-2-[(2-quinoléinylcarbonyl)amino]-, [1S-[1R*(R*),2S*]]-
le butanediamide, N¹-[3-[[[(1,1-diméthyléthyl)amino]carbonyl](3,3-diéthylpropyl)amino]-2-hydroxy-1-(phénylméthyl)propyl]-2-[(N-benzyloxycarbonyl)amino]-, [1S-[1R*(R*),2S*]]-
le butanediamide, N¹-[3-[[[(1,1-diméthyléthyl)amino]carbonyl](3,3-diméthylpropyl)amino]-2-hydroxy-1-(phénylméthyl)propyl]-2-[(2-quinoléinylcarbonyl)amino]-, [1S-[1R*(R*),2S*]]-
le propaneamide, 3-acétylamino-N-[3-[[[(1,1-diméthyléthyl)amino]carbonyl](3-méthylbutyl)amino]-2-hydroxy-1-(phénylméthyl)propyl]-2-méthyl-, [1S-[1R*(S*),2S*]]-
le propaneamide, 3-(4-méthoxybenzyloxycarbonyl)amino-N-[3-[[[(1,1-diméthyléthyl)amino]carbonyl](3-méthylbutyl)amino]-2-hydroxy-1-(phénylméthyl)propyl]-2-méthyl-, [1S-[1R*(S*),2S*]]-
le butaneamide, N¹-[2-hydroxy-3-[(4-fluorophénylméthyl)(1-pyrrolidinylcarbonyl)amino]-1-(phénylméthyl)propyl]-2-[(2-quinoléinylcarbonyl)amino]-, [1S-[1R*(R*),2S*]]-
le butanediamide, 2-acétylamino-N-[3-[[[(1,1-diméthyléthyl)amino]carbonyl](3-méthylbutyl)amino]-2-hydroxy-1-(phénylméthyl)propyl]-3,3-diméthyl-, [1S-[1R*(R*),2S*]]-
le butanediamide, N¹-[3-[[[n-butylamino]carbonyl](2-méthylpropyl)amino]-2-hydroxy-1-(phénylméthyl)propyl]-2-[(phénylméthylcarbamoyl)amino]-, [1S-[1R*(R*),2S*]]-
le butanediamide, N¹-[3-[[[n-butylamino]carbonyl]-(2-méthylpropyl)amino]-2-hydroxy-1-(phénylméthyl)propyl]-2-[(2-quinoléinylcarbonyl)amino]-, [1S-[1R*(R*),2S*]]-
le butanediamide, N¹-[3-[[[(1,1-diméthyléthyl)-amino]carbonyl](2-phényléthyl)amino]-2-hydroxy-1-(phénylméthyl)propyl]-2-[(2-quinoléinylcarbonyl)amino]-, [1S-[1R*(R*),2S*]]-
le butanediamide, N¹-[3-[[[(1,1-diméthyléthyl)amino]carbonyl](2-méthylpropyl)amino]-2-hydroxy-1-(phénylméthyl)propyl]-2-[(2-quinoléinylcarbonyl)amino]-, [1S-[1R*(R*),2S*]]-
le butanediamide, N¹-[3-[[[(1,1-diméthyléthyl)-amino]carbonyl]pentylamino]-2-hydroxy-1-(phénylméthyl)propyl]-2-[(2-quinoléinylcarbonyl)amino]-, [1S-[1R*(R*),2S*]]-
le butanediamide, N¹-[3-[[[(1,1-diméthyléthyl)amino]carbonyl]hexylamino]-2-hydroxy-1-(phénylméthyl)propyl]-2-[(2-quinoléinylcarbonyl)amino]-, [1S-[1R*(R*),2S*]]-
le butanediamide, N¹-[2-hydroxy-3-[(3-méthylbutyl)(1-pyrrolidinylcarbonyl)amino]-1-(phénylméthyl)propyl]-2-[(2-quinoléinylcarbonyl)amino]-, [1S-[1R*(R*),2S*]]-
le butanediamide, N¹-[2-hydroxy-3-[(3-méthylbutyl)(1-pyrrolidinylcarbonyl)amino]-1-(4-fluorophénylméthyl)propyl]-2-[(2-quinoléinylcarbonyl)amino]-, [1S-[1R*(R*),2S*]]-
le butanediamide, N¹-[2-hydroxy-3-[(3-méthylbutyl)(1-pyrrolidinylcarbonyl)amino]-1-(cyclohexylméthyl)propyl]-2-[(2-quinoléinylcarbonyl)amino]-, [1S-[1R*(R*),2S*]]-
le butanediamide, N¹-[2-hydroxy-3-[(phénylméthyl)(1-morpholinylcarbonyl)amino]-1-(phénylméthyl)propyl]-2-[(2-quinoléinylcarbonyl)amino]-, [1S-[1R*(R*),2S*]]-
le butanediamide, N¹-[2-hydroxy-3-[(3-méthylbutyl)(1-pipéridinylcarbonyl)amino]-1-(phénylméthyl)propyl]-2-[(2-quinoléinylcarbonyl)amino]-, [1S-[1R*(R*),2S*]]-
le butanediamide, N¹-[2-hydroxy-3-[(phénylméthyl)(1-pipéridinylcarbonyl)amino]-1-(phénylméthyl)propyl]-2-[(2-quinoléinylcarbonyl)amino]-, [1S-[1R*(R*),2S*]]-
le butaneamide, 2-acétylamino-N-[3-[[[(1,1-diméthyléthyl)amino]carbonyl](3-méthylbutyl)amino]-2-hydroxy-1-(cyclohexylméthyl)propyl]-3,3-diméthyl-, [1S-[1R*(R*),2S*]]-
le butaneamide, 2-acétylamino-N-[3-[[[(1,1-diméthyléthyl)amino]carbonyl]butylamino]-2-hydroxy-1-(phénylméthyl)propyl]-3,3-diméthyl-, [1S-[1R*(R*),2S*]]-
le butaneamide, 2-acétylamino-N-[3-[[[(1,1-diméthyléthyl)amino]carbonyl](phénylméthyl)amino]-2-hydroxy-1-(phénylméthyl)propyl]-3,3-diméthyl-, [1S-[1R*(R*),2S*]]-
le butaneamide, 2-acétylamino-N-[3-[[[(1,1-diméthyléthyl)amino]carbonyl](cyclohexylméthyl)amino]-2-hydroxy-1-(phénylméthyl)propyl]-3,3-diméthyl-, [1S-[1R*(R*),2S*]]-
le butaneamide, 2-(2,2-diméthylaminoacétyl)amino-N-[3-[[[(1,1-diméthyléthyl)amino]carbonyl](3-méthylbutyl)amino]-2-hydroxy-1-(4-fluorophénylméthyl)propyl]-3,3-diméthyl-, [1S-[1R*(R*),2S*]]-
le butaneamide, 2-(2,2-diméthylaminoacétyl)amino-N-[3-[[[(1,1-diméthyléthyl)amino]carbonyl](3-méthylbutyl)amino]-2-hydroxy-1-(cyclohexylméthyl)propyl]-3,3-diméthyl-, [1S-[1R*(R*),2S*]]-
le butaneamide, 2-(2,2-diméthylaminoacétyl)amino-N-[3-[[[(1,1-diméthyléthyl)amino]carbonyl]butylamino]-2-hydroxy-1-(phénylméthyl)propyl]-3,3-diméthyl-, [1S-[1R*(R*),2S*]]-
le butaneamide, 2-(2,2-diméthylaminoacétyl)amino-N-[3-[[[(1,1-diméthyléthyl)amino]carbonyl](phénylméthyl)amino]-2-hydroxy-1-(phénylméthyl)propyl]-3,3-diméthyl-, [1S-[1R*(R*),2S*]]-
le butaneamide, 2-(2,2-diméthylaminoacétyl)amino-N-[3-[[[(1,1-diméthyléthyl)amino]carbonyl](4-fluorophénylméthyl)amino]-2-hydroxy-1-(phénylméthyl)propyl]-3,3-diméthyl-, [1S-[1R*(R*),2S*]]-
le butaneamide, 2-(2,2-diméthylaminoacétyl)amino-N-[3-[[[(1,1-diméthyléthyl)amino]carbonyl](cyclohexylméthyl)amino]-2-hydroxy-1-(phénylméthyl)propyl]-3,3-diméthyl-, [1S-[1R*(R*),2S*]]-
